# EUROPEAN PATENT SPECIFICATION

(11) **EP 2 550 257 B1**
(45) Date of publication and mention of the grant of the patent: **21.12.2016**
(21) Application number: 11711039.5
(22) Date of filing: 20.03.2011
(51) Int. Cl.: C07D 251/22, C07D 401/04, C07D 403/12, C07D 407/04, A61K 31/53, A61P 35/00, A61P 37/00, C07D 401/12

(54) **PHARMACEUTICALLY ACTIVE DISUBSTITUTED TRIAZINE DERIVATIVES**
PHARMAZEUTISCH AKTIVE DISUBSTITUIERTE TRIAZINDERIVATE
DÉRIVÉS DE TRIAZINE DISUBSTITUÉ UTILISÉS COMME PHARMACEUTIQUES

(30) Priority: 29.03.2010 US 282766 P; 22.03.2010 EP 10075131
(43) Date of publication of application: 30.01.2013
(73) Proprietor: Lead Discovery Center GmbH, 44227 Dortmund (DE); Bayer Intellectual Property GmbH, 40789 Monheim (DE)
(72) Inventor: EICKHOFF, Jan, 58131 Herdecke (DE); NUSSBAUMER, Peter, 44227 Dortmund (DE); RÜHTER, Gerd, 21129 Hamburg (DE); SCHULTZ-FADEMRECHT, Carsten, 44309 Dortmund (DE); LÜCKING, Ulrich, 10317 Berlin (DE); CHOIDAS, Axel, 58313 Herdecke (DE); KLEBL, Bert, 44229 Dortmund (DE)
(74) Representative: Arth, Hans-Lothar
(86) International application number: PCT/EP2011/001445
(87) International publication number: WO 2011/116951

(56) References cited:
- WO-A1-01/25220
- WO-A1-03/037346
- WO-A1-2008/129080
- WO-A2-2008/079933
- US-A1- 2004 116 388
- RANJITKAR PRATISTHA ET AL: "Affinity Reagents that Target a Specific Inactive Form of Protein Kinases", CHEMISTRY & BIOLOGY (CAMBRIDGE), vol. 17, no. 2, February 2010 (2010-02), pages 195-206, XP002640101, ISSN: 1074-5521
- CHEN C ET AL: "A convenient synthetic method for trisubstituted s-triazines", JOURNAL OF ORGANIC CHEMISTRY 1995 US, vol. 60, no. 26, 1995, pages 8428-8430, XP002640102, ISSN: 0022-3263

## Description

The present invention relates to disubstituted triazine derivatives and/or pharmaceutically acceptable salts thereof, the use of these derivatives as pharmaceutically active agents, especially for the prophylaxis and/or treatment of cell proliferative diseases, inflammatory and immunological diseases, cardiovascular diseases and infectious diseases. Furthermore, the present invention is directed towards a pharmaceutical composition containing at least one of the disubstituted triazine derivatives and/or pharmaceutically acceptable salts thereof.

Cyclin-dependent kinase (CDK) family members that trigger passage through the cell cycle are being considered as attractive therapeutic targets, especially for cancer. CDK family members that control other processes such as transcription and RNA processing have caught less attention so far, although experimental evidence for their involvement in different pathological processes is emerging. As a general regulator of transcription, CDK9 is a therapeutic target for treatment of diseases like inflammation, virus replication such as HIV, EBV, and HCV, cancer and cardiac hypertrophy.

CDK9 regulates transcription by phosphorylation of RNA polymerase II as well as additional regulatory factors, thereby enabling productive elongation of transcription. Certain subgroups of genes, especially genes encoding RNAs or proteins with fast turnover like immediate early genes of the inflammatory response, NF-kappaB activated genes (Brasier 2008, Cell Cycle 7:17, 2661-2666, Hargreaves et al. (2009) Cell 138,129-145); and antiapoptotic genes such as MCL-1 and Bcl-2 family members appear to be especially sensitive to CDK9 inhibition.

In addition, it has been reported that hypertrophic growth of cardiomyocytes is related to CDK9 activation. Furthermore, viruses like the human immune deficiency virus recruit CDK9 actively to nascent RNA transcripts, facilitating their replicating. The dependency of the expression of antiapoptotic genes on CDK9 activity makes it an attractive therapeutic target for various forms of leukaemia such as chronic lymphocytic leukaemia (CLL), acute myelogenous leukaemia (AML) and acute lymphoblastic leukaemia, and solid tumours like prostate, lung, colon, breast and pancreas cancer. In addition, CDK9 inhibitors have been active in models of stroke (Osuga 2000, PNAS 97 (18): 10254-10259).

For reviews, see Wang, 2009 (Trends in Pharmacological Sciences 29:6, 302-313), and Kohoutek, 2009 (Cell Division 2009, 4:19).

It is object of the present invention to provide compounds and/or pharmaceutically acceptable salts thereof which can be used as pharmaceutically active agents, especially for prophylaxis and/ or treatment of cell proliferative diseases, inflammatory diseases, immunological diseases, cardiovascular diseases and infectious diseases, as well as compositions comprising at least one of those compounds and/or pharmaceutically acceptable salts thereof as pharmaceutically active ingredients.

This object is solved by the compounds and/or their pharmaceutically acceptable salts according to independent claim 1, the compounds of the present invention for use as pharmaceutically active agents, and the compounds of the present invention for use in the prophylaxis and/or treatment of infectious diseases, including opportunistic diseases, immunological diseases, autoimmune diseases, cardiovascular diseases, cell proliferative diseases, inflammation, erectile dysfunction and stroke .

Further advantageous features, aspects and details of the invention are evident from the dependent claims, the description, the examples and the drawings.

The novel disubstituted triazine compounds according to the present invention are defined by the general formula (I) wherein 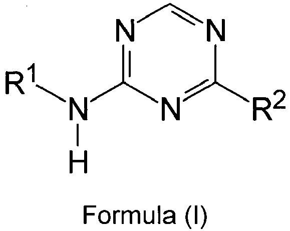 **R¹** represents 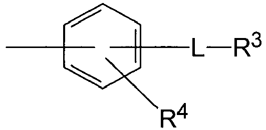 in which
L is -CH₂-, -CH₂CH₂-, or -CF₂-;
**R³** is -SO₂NH₂, -SO₂NH(CH₃), -SO₂N(CH₃)₂, -SO₂NH(CH₂CH₂OCH₃), -NHSO₂CH₃, -NHSO₂CH₂CH₃, -NHSO₂CH₂CH₂CH₃, -NHSO₂CF₃, -SO₂CH₃, -NHSO₂NH₂, -SO(NH)CH₃;
**R⁴** is -H, -CH₃, -F, -Cl, or -CF₃;
**R**² represents 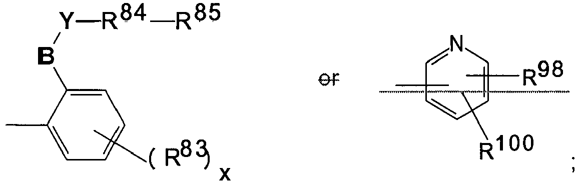 in which the group **-B-Y-R⁸⁴-R⁸⁵** is -OCH₃, -OCH₂CH₃, -OCH₂CH₂CH₃, -OCH₂CH₂CH₂CH₃, -OCH(CH₃)₂, -OPh, -OCH₂Ph, -OCH₂(4-pyridyl) ;
**R**⁸³ is -H, -F, or -Cl;
x is 0, 1, or 2;
or enantiomers stereoisomeric forms mixtures of enantiomers, diastereomers, mixtures of diastereomers, hydrates, solvates, acid salt forms, tautomers, and racemates and pharmaceutically acceptable salts thereof.

The expression tautomer is defined as an organic compound that is interconvertible by a chemical reaction called tautomerization. Tautomerization can be catalyzed preferably by bases or acids or other suitable compounds.

Preferred is compound of Formula (I), wherein
the substituent **-L-R³** is -CH₂SO₂NH₂, -CH₂CH₂SO₂NH₂, -CF₂SO₂NH₂, -CH₂NHSO₂NH₂, -SO(NH)CH₃, -CH₂SO(NH)CH₃;
**R⁴** is -H; and
R² is 2-methoxyphenyl, 4-fluoro-2-methoxyphenyl, or 6-fluoro-2-methoxyphenyl.

The following residues **R**² are preferred:

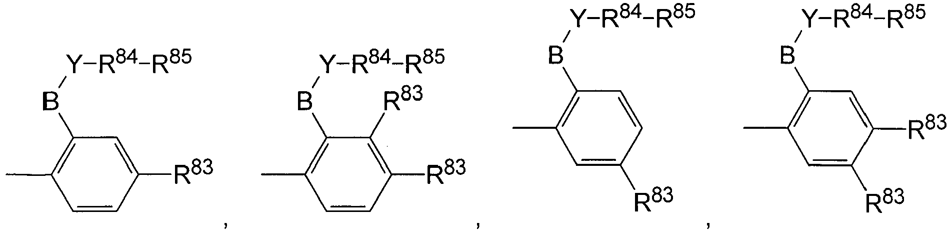

In a particularly preferred embodiment the present invention concerns compounds of formula (I), wherein
**R¹** represents 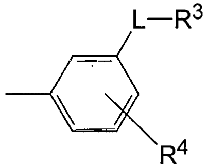 in which
the substituent -L-R³ is -CH₂SO₂NH₂;
R⁴ is -H;
R² represents 2-methoxyphenyl, 4-fluoro-2-methoxyphenyl or 2-benzyloxyphenyl,
or their salts, solvates or salts of solvates and especially the hydrochloride salt or the trifluoroacetate salt.

In another particularly preferred embodiment the present invention concerns compounds of formula (I) selected from
3-[(4-(2-Methoxyphenyl)-1,3,5-triazin-2-yl)amino]benzenemethanesulfonamide (B1),
3-[(4-(2-Methoxyphenyl)-1,3,5-triazin-2-yl)amino]benzenesulfonamide (C1),
3-[(4-(4-Fluoro-2-methoxyphenyl)-1,3,5-triazin-2-yl)amino]benzenemethanesulfonamide (B2),
3-[(4-(2-Benzyloxyphenyl)-1,3,5-triazin-2-yl)amino]benzenemethanesulfonamide (B13),
or their salts, solvates or salts of solvates and especially the hydrochloride salt or the trifluoroacetate salt.

In another particularly preferred embodiment the present invention concerns 3-[(4-(4-Fluoro-2-methoxyphenyl)-1,3,5-triazin-2-yl)amino]benzenemethanesulfonamide, or its salts, solvates or salts of solvates and especially the hydrochloride salt or the trifluoroacetate salt.

In another particularly preferred embodiment the present invention concerns 1-(3-{[4-(4-fluoro-2-methoxyphenyl)-1,3,5-triazin-2-yl]amino}phenyl)methanesulfonamide hydrochloride.

In a further aspect of the present invention, the novel compounds according to the general formula (I) represent chiral compounds. The novel compounds according to the general formula (I) represent a racemate, or a S or a R enantiomer or a mixture of isomers.

In yet another preferred embodiment of the present invention, the compound according to the general formula (I) is selected from the group of compounds depicted in the following Table 1.

**Table 1**

| **Compound No.** | **Structure** | **Nomenclature** |
|---|---|---|
| **B1** | 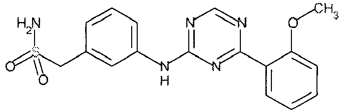 | 3-[(4-(2-Methoxyphenyl)-1,3,5-triazin-2-yl)amino]benzenemethanesulfonamide |
| **B2** | 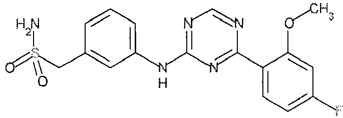 | 3-[(4-(4-Fluoro-2-methoxyphenyl)-1,3,5-triazin-2-yl)amino] benzenemethanesulfonamide |
| **B3** | 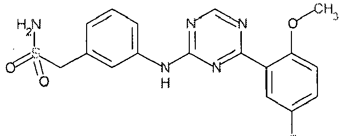 | 3-[(4-(5-Fluoro-2-methoxyphenyl)-1,3,5-triazin-2-yl)amino] benzenemethanesulfonamide |
| **B4** | 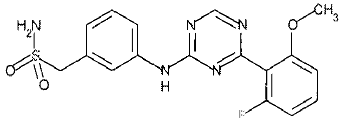 | 3-[(4-(6-Fluoro-2-methoxyphenyl)-1,3,5-triazin-2-yl)amino] benzenemethanesulfonamide |
| **B5** | 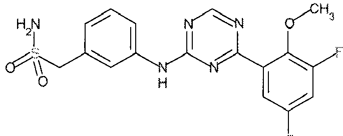 | 3-[(4-(3,5-Difluoro-2-methoxyphenyl)-1,3,5-triazin-2-yl)amino] benzenemethanesulfonamide |
| **B6** | 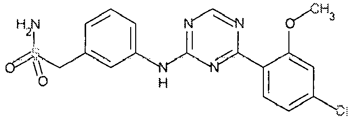 | 3-[(4-(4-Chloro-2-methoxyphenyl)-1,3,5-triazin-2-yl)amino] benzenemethanesulfonamide |
| **B7** | 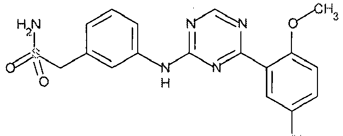 | 3-[(4-(5-Chloro-2-methoxyphenyl)-1,3,5-triazin-2-yl)amino] benzenemethanesulfonamide |
| **B8** | 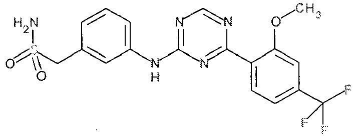 | 3-[(4-(2-Methoxy-4-trifluoromethyl-phenyl)-1,3,5-triazin-2-yl)amino] benzenemethanesulfonamide |
| **B9** | 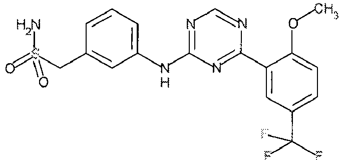 | 3-[(4-(2-Methoxy-5-trifluoromethyl-phenyl)-1,3,5-triazin-2-yl)amino] benzenemethanesulfonamide |
| **B10** | 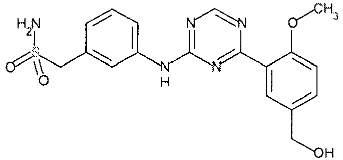 | 3-[(4-(5-Hydroxymethyl-2-methoxyphenyl)-1,3,5-triazin-2-yl)amino] benzenemethanesulfonamide |
| **B11** | 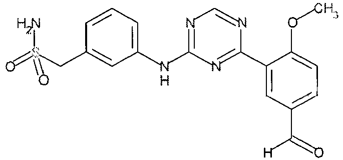 | 3-[(4-(5-Formyl-2-methoxyphenyl)-1,3,5-triazin-2-yl)amino] benzenemethanesulfonamide |
| **B12** | 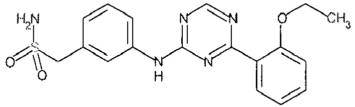 | 3-[(4-(2-Ethoxyphenyl)-1,3,5-triazin-2-yl)amino]benzenemethanesulfonamide |
| **B13** | 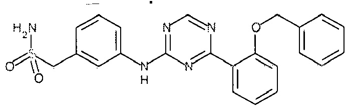 | 3-[(4-(2-Benzyloxyphenyl)-1,3,5-triazin-2-yl)amino] benzenemethanesulfonamide |
| **B14** | 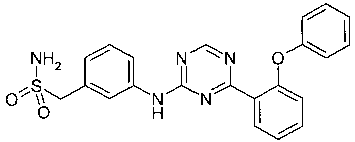 | 1-(3-{[4-(2-phenoxyphenyl)-1,3,5-triazin-2-yl]amino}phenyl)methanesulfonamide |
| **B15** | 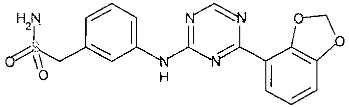 | 3-[(4-(1,3-Benzodioxol-4-yl)-1,3,5-triazin-2-yl)amino]benzenemethanesulfonamide |
| **B16** | 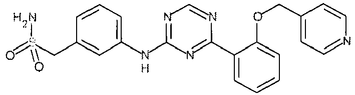 | 3-[(4-(2-((4-Pyridinyl)methoxy)phenyl)-1,3,5-triazin-2-yl)amino]benzenemethanesulfonamide |
| **B17** | 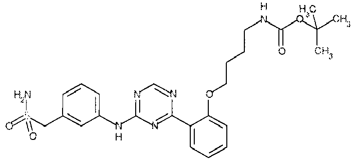 | 3-[(4-(2-(4-(tert-Butoxycarbonylamino)butoxy)phenyl)-1,3,5-triazin-2-yl)amino]benzenemethanesulfonamide |
| **B18** | 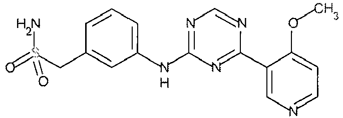 | 3-[(4-(4-Methoxypyridin-3-yl)-1,3,5-triazin-2-yl)amino]benzenemethanesulfonamide |
| **B19** | 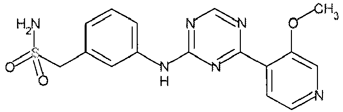 | 3-[(4-(3-Methoxypyridin-4-yl)-1,3,5-triazin-2-yl)amino]benzenemethanesulfonamide |
| **B20** | 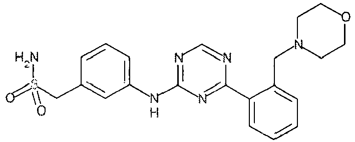 | 3-[(4-(2-((Morpholin-4-yl)methyl)phenyl)-1,3,5-triazin-2-yl)amino]benzenemethanesulfonamide |
| **B21** | 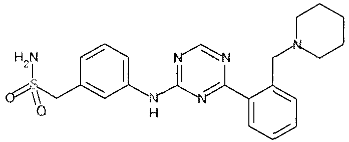 | 3-[(4-(2-((Piperidin-1-yl)methyl)phenyl)-1,3,5-triazin-2-yl)amino]benzenemethanesulfonamide |
| **B22** | 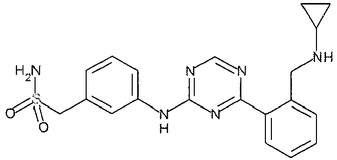 | 3-[(4-(2-(Cyclopropylamino-methyl)phenyl)-1,3,5-triazin-2-yl)amino]benzenemethanesulfonamide |
| **B23** | 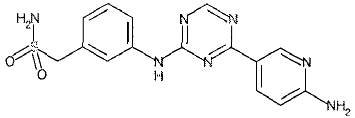 | 3-[(4-(6-Aminopyridin-3-yl)-1,3,5-triazin-2-yl)amino]benzenemethanesulfonamide |
| **B24** | 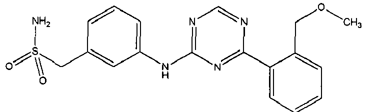 | 3-[(4-(2-(Methoxymethyl)phenyl)-1,3,5-triazin-2-yl)amino]benzenemethanesulfonamide |
| **C1** | 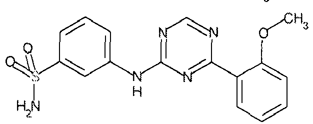 | 3-[(4-(2-Methoxyphenyl)-1,3,5-triazin-2-yl)amino]benzenesulfonamide |
| **D1** | 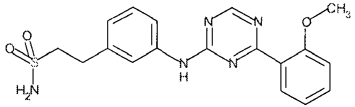 | 2-[3-((4-(2-Methoxyphenyl)-1,3,5-triazin-2-yl)amino)phenyl]ethanesulfonamide |
| **D2** | 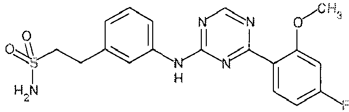 | 2-[3-((4-(4-Fluoro-2-methoxyphenyl)-1,3,5-triazin-2-yl)amino)phenyl]ethanesulfonamide |
| **G1** | 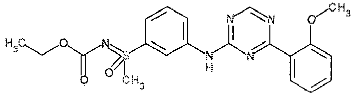 | rac-S-[3-((4-(2-Methoxyphenyl)-1,3,5-triazin-2-yl)amino)phenyl]-N-ethoxycarbonyl-S-methyl-sulfoximide |
| **I1** | 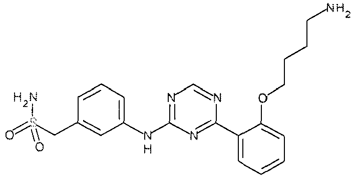 | 3-[(4-(2-(4-Aminobutoxy)phenyl)-1,3,5-triazin-2-yl)amino]benzenemethanesulfonamide |
| **J1** | 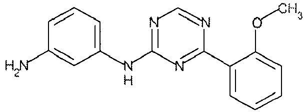 | N-(3-Aminophenyl)-4-(2-methoxyphenyl)-1,3,5-triazine-2-amine |
| **K1** | 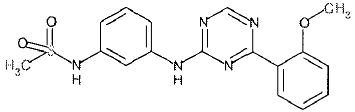 | N-[3-((4-(2-Methoxyphenyl)-1,3,5-triazin-2-yl)amino)phenyl]-methanesulfonamide |
| **L1** | 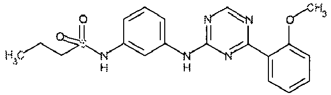 | N-[3-((4-(2-Methoxyphenyl)-1,3,5-triazin-2-yl)amino)phenyl]-propanesulfonamide |
| **N1** | 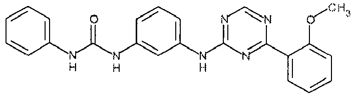 | N-[3-((4-(2-Methoxyphenyl)-1,3,5-triazin-2-yl)amino)phenyl]-N'-phenyl-urea |
| **O1** | 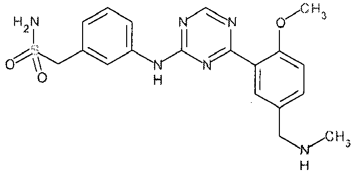 | 3-[(4-(2-Methoxy-5-(methylamino-methyl)phenyl)-1,3,5-triazin-2-yl)amino]benzenemethanesulfonamide |
| **P1** | 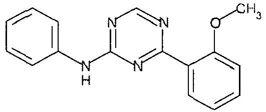 | 4-(2-Methoxyphenyl)-N-phenyl-1,3,5-triazine-2-amine |
| **Q1** | 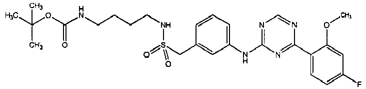 | tert-Butyl [4-((3-((4-(4-Fluoro-2-methoxyphenyl)-1,3,5-triazin-2-yl)amino)phenyl)methylsulfonamido) butyl]carbamate |
| **R1** | 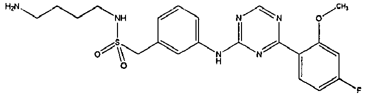 | N-(4-Aminobutyl)-1-[3-((4-(4-fluoro-2-methoxyphenyl)-1,3,5-triazin-2-yl)amino)phenyl]methanesulfonamide |
| **S1** | 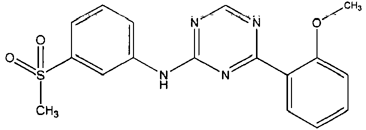 | 4-(2-Methoxyphenyl)-N-(3-(methylsulfonyl)phenyl)-1,3,5-triazin-2-amine |
| **T1** | 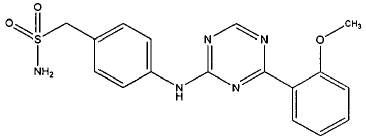 | 4-[(4-(2-Methoxyphenyl)-1,3,5-triazin-2-yl)amino]benzenemethane-sulfonamide |
| **U1** | 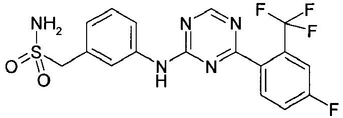 | 1-[3-({4-[4-fluoro-2-(trifluoromethyl)phenyl]-1,3,5-triazin-2-yl}amino)phenyl]methanesulfonamide |
| **U2** | 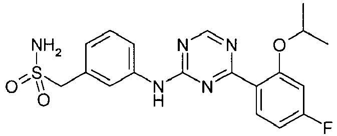 | 1-[3-({4-[4-fluoro-2-(propan-2-yloxy)phenyl]-1,3,5-triazin-2-yl}amino)phenyl]methanesulfonamide |
| **U3** | 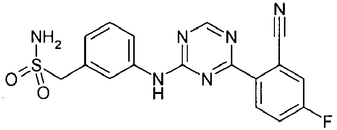 | 1-(3-{[4-(2-cyano-4-fluorophenyl)-1,3,5-triazin-2-yl]amino}phenyl)methanesulfonamide |
| **U4** | 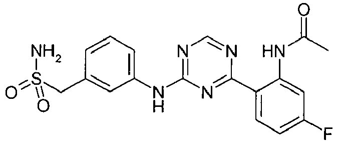 | N-[5-fluoro-2-(4-{[3-(sulfamoylmethyl)phenyl]amino}-1,3,5-triazin-2-yl)phenyl]acetamide |
| **U5** | 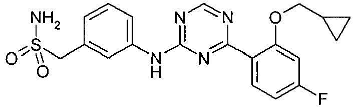 | 1-[3-({4-[2-(cyclopropylmethoxy)-4-fluorophenyl]-1,3,5-triazin-2-yl}amino)phenyl]methanesulfonamide |
| **U6** | 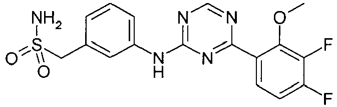 | 1-(3-{[4-(3,4-difluoro-2-methoxyphenyl)-1,3,5-triazin-2-yl]amino}phenyl)methanesulfonamide |
| **U7** | 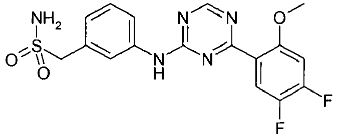 | 1-(3-{[4-(4,5-difluoro-2-methoxyphenyl)-1,3,5-triazin-2-yl]amino}phenyl)methanesulfonamide |
| **U8** | 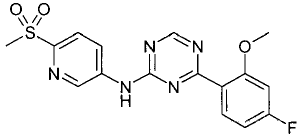 | 4-(4-fluoro-2-methoxyphenyl)-N-[6-(methylsulfonyl)pyridin-3-yl]-1,3,5-triazin-2-amine |
| **B1**' | 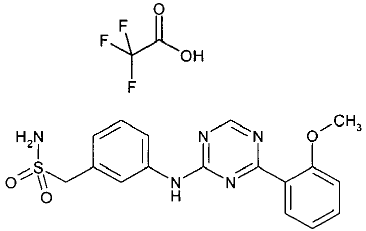 | 3-[(4-(2-Methoxyphenyl)-1,3,5-triazin-2-yl)amino]benzenemethanesulfonamide trifluoroacetic acid salt |
| **B2**' | 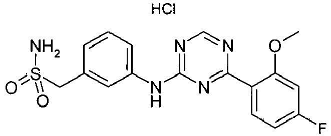 | 1-(3-{[4-(4-fluoro-2-methoxyphenyl)-1,3,5-triazin-2-yl]amino}phenyl)methanesulfonamide hydrochloride |
| **B13**' | 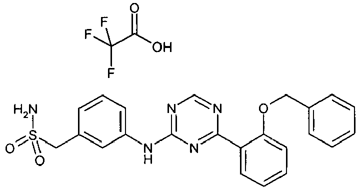 | 3-[(4-(2-Benzyloxyphenyl)-1,3,5-triazin-2-yl)amino]benzenemethanesulfon-amide trifluoroacetic acid salt |
| **C1**' | 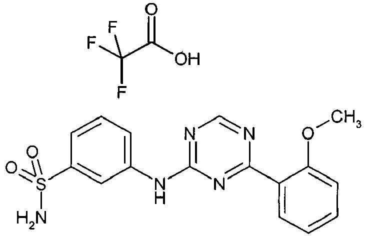 | 3-[(4-(2-Methoxyphenyl)-1,3,5-triazin-2-yl)amino]benzenesulfonamide trifluoroacetic acid salt |
| **B2"** | 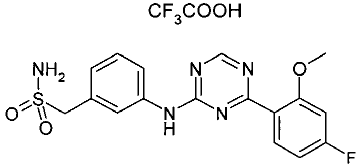 | 1-(3-{[4-(4-fluoro-2-methoxyphenyl)-1,3,5-triazin-2-yl]amino}phenyl)methanesulfonamide trifluoroacetic acid salt |

The compounds of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, (o, m, p)-toluic acid, naphthylamine sulfonic acid, trifluoroacetic acid, and other mineral or carboxylic acids well known to those skilled in the art. The salts are prepared by contacting the free base form with a sufficient amount of the desired acid to produce a salt in the conventional manner. Preferred is the mesylate salt, hydrochloride salt and the trifluoroacetate salt and especially preferred is the trifluoroacetate salt and the hydrochloride salt.

In the case the inventive compounds bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

### Syntheses of compounds

The synthesis of the inventive disubstituted triazines according to the present invention is preferably carried out according to the general synthetic sequences, shown in **Schemes 1** to **3.**

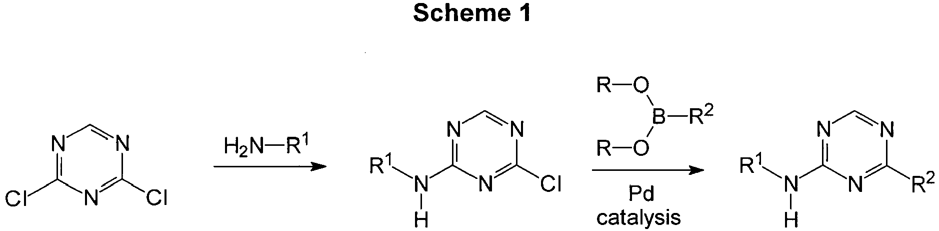

In a first step 2,4-Dichloro-1,3,5-triazine is reacted with anilines R¹NH₂ to give 2-arylamino-4-chloro-1,3,5-triazines. The reaction is carried out with one equivalent of the aniline in an inert solvent like DMF, THF, DME, dioxane or an alcohol like isopropanol, or mixtures of such solvents. Preferably the reaction is carried out at a temperature below room temperature in such a way that the reaction mixture is kept homogenous. Preferred conditions use an additional base like triethylamine or N,N-diisopropylethylamine.

In a second step the intermediate 2-arylamino-4-chloro-1,3,5-triazine is reacted with a boronic acid derivative R²-B(OR)₂ to give compounds of Formula (I). The boronic acid derivative may be a boronic acid (R = -H) or an ester of the boronic acid, e.g. its isopropyl ester (R = -CH(CH₃)₂), preferably an ester derived from pinacol in which the boronic acid intermediate forms a 2-aryl-4,4,5,5-tetramethyl-1,3,2-dioxaborolane (R-R = -C(CH₃)₂-C(CH₃)₂-). Both R represent independently of each other preferably hydrogen or an alkyl chain with 1-10 carbon atoms or a cycloalkyl chain with 3 to 12 carbon atoms or both residues R represent together a residue derived from pinacol. The coupling reaction is catalyzed by Pd catalysts, e.g. by Pd(0) catalysts like tetrakis(triphenylphosphine)palladium(0) [Pd(PPh₃)₄], tris(dibenzylideneacetone)di-palladium(0) [Pd₂(dba)₃], or by Pd(II) catalysts like dichlorobis(triphenylphosphine)-palladium(II) [Pd(PPh₃)₂Cl₂], palladium(II) acetate and triphenylphosphine or more preferred by [1,1'-bis(diphenylphosphino)ferrocene]palladium dichloride. The reaction is preferably carried out in a mixture of a solvent like dioxane, DMF, DME, THF, or isopropanol with water and in the presence of a base like aqueous sodium bicarbonate or K₃PO₄.

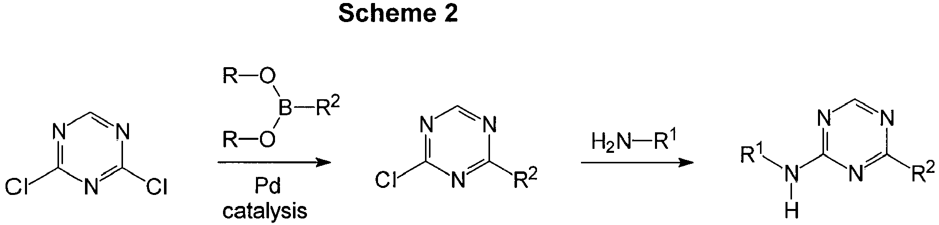

The synthesis of 1,3,5-triazines of Formula (I) starting from 2,4-dichloro-1,3,5-triazine may be carried out in the inverse order of the reaction steps compared to Scheme 1, in such a manner that in a first step the reaction of a triazine with a boronic acid derivative is followed in a second step by the reaction of the intermediate triazine with an aniline. Preferred conditions for the coupling reaction of the first step are heating the reacting agents in toluene with dichlorobis(triphenylphosphine)palladium(II) [Pd(PPh₃)₂Cl₂] as a catalyst in the presence of sodium or potassium carbonate as a base.

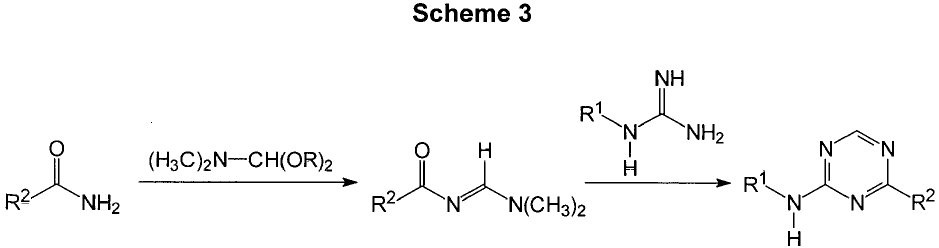

Compounds of Formula (I) may be prepared by the methodology described in J. Org. Chem. 60 (1995), 8428-8430. Primary amides R²-CONH₂ are heated with acetals and preferably dialkylacetals of N,N-dimethylformamide, preferably with its dimethyl or diethyl acetal, in particular with the dimethyl acetal (R = -CH₃). The intermediate N-acylformamidine is not isolated and subsequently converted to 1,3,5-triazines of Formula (I) by heating with a guanidine R¹-NH-C(NH)NH₂. Preferably the reaction is carried out by heating the reacting agents in dioxane in the presence of a base like potassium tert-butoxide.

Several compounds of Formula (I) may be prepared by converting substituents which are attached to the aromatic rings R¹ and/or R² to other substituents using standard reactions which are known to the person skilled in the art. For example, a nitro group can be reduced to an amino group, such an amino group can be converted to a sulfonamide by reaction with a sulfonyl chloride, to a carboxamide by reaction with a carbonyl chloride or another activated derivative of a carboxylic acid, to an urea by reaction with an isocyanate. Carbamate substituents may be cleaved to amino groups, in particular tert-butyl carbamates by reaction with acids like trifluoroacetic acid or hydrochloric acid. Formyl groups may be converted to aminomethyl groups by reaction with primary amines under conditions of a reductive amination.

### Methods of Use

In a further aspect of the present invention, the novel compounds according to the general formula (I) are used as pharmaceutically active agent.

In one embodiment, the present invention is directed to the compounds of general formula (I) for use in the prophylaxis and/or treatment of infectious diseases including opportunistic diseases, immunological diseases, autoimmune diseases, cardiovascular diseases, cell proliferative diseases, inflammation, erectile dysfunction and stroke.

The compounds of the present invention may be used to inhibit the activity or expression of CDK9. Therefore, the compounds of formula (I) are expected to be valuable as therapeutic agents. Accordingly, in another embodiment, the present invention provides a method of treating disorders relating to or mediated by CDK9 activity in a patient in need of such treatment, comprising administering to the patient an effective amount of a compound of formula (I) as defined above. In certain embodiments, the disorders relating to CDK9 activity are cell proliferative disorders, particularly cancer.

The term "treating" or "treatment" as stated throughout this document is used conventionally, e.g., the management or care of a subject for the purpose of combating, alleviating, reducing, relieving, improving the condition of a disease or disorder, such as a carcinoma.

The term "subject" or "patient" includes organisms which are capable of suffering from a cell proliferative disorder or a disorder associated with reduced or insufficient programmed cell death (apoptosis) or who could otherwise benefit from the administration of a compound of the invention, such as human and non-human animals. Preferred humans include human patients suffering from or prone to suffering from a cell proliferative disorder or associated state, as described herein. The term "non-human animals" includes vertebrates, e.g., mammals, such as non-human primates, sheep, cow, dog, cat and rodents, e.g., mice, and non-mammals, such as chickens, amphibians, reptiles, etc.

The term "disorders relating to or mediated by CDK9" shall include diseases associated with or implicating CDK9 activity, for example the hyperactivity of CDK9, and conditions that accompany with these diseases. Examples of "disorders relating to or mediated by CDK9" include disorders resulting from increased CDK9 activity due to mutations in genes regulating CDK9 activity auch as LARP7, HEXIM1/2 or 7sk snRNA, or disorders resulting from increased CDK9 activity due to activation of the CDK9/cyclinT/RNApolymerase II complex by viral proteins such as HIV-TAT or HTLV-TAX or disorders resulting from increased CDK9 activity due to activation of mitogenic signaling pathways.

The term "hyperactivity of CDK9" refers to increased enzymatic activity of CDK9 as compared to normal non-diseased cells, or it refers to increased CDK9 activity leading to unwanted cell proliferation, or to reduced or insufficient programmed cell death (apoptosis), or mutations leading to constitutive activation of CDK9.

The term "cell proliferative disorder" includes disorders involving the undesired or uncontrolled proliferation of a cell and it includes disorders involving reduced or insufficient programmed cell death (apoptosis). The compounds of the present invention can be utilized to prevent, inhibit, block, reduce, decrease, control, etc., cell proliferation and/or cell division, and/or produce apoptosis. This method comprises administering to a subject in need thereof, including a mammal, including a human, an amount of a compound of this invention, or a pharmaceutically acceptable salt, isomer, polymorph, metabolite, hydrate or solvate thereof which is effective to treat or prevent the disorder.

### Infectious diseases including opportunistic infections

The term "infectious diseases" comprises infections caused by viruses, bacteria, prions, fungi, and/or parasites.

Especially, virally induced infectious diseases, including opportunistic diseases are addressed. In a preferred embodiment of this aspect, the virally induced infectious diseases, including opportunistic diseases, are caused by retroviruses, human endogenous retroviruses (HERVs), hepadnaviruses, herpesviruses, flaviviridae, and/or adenoviruses. Preferably, the retroviruses are selected from lentiviruses or oncoretroviruses, wherein the lentivirus is preferably selected from the group comprising: HIV-1, HIV-2, feline immunodeficiency virus (FIV), bovine immunodeficiency virus (BIV), sivian immunodeficiency viruses (SIVs), chimeras of HIV and SIV (SHIV), caprine arthritis encephalitis virus (CAEV), visna/maedi virus (VMV) or equine infectious anemia virus (EIAV), preferably HIV-1 and HIV-2, and the oncoretrovirus is preferably selected from HTLV-I, HTLV-II or bovine leukemia virus (BLV), preferably HTLV-I and HTLV-II.

The hepadnavirus is preferably selected from HBV, ground squirrel hepatitis virus (GSHV) or woodchuck hepatitis virus (WHV), preferably HBV, the herpesvirus is selected from the group comprising: Herpes simplex virus I (HSV I), herpes simplex virus II (HSV II), Epstein-Barr virus (EBV), varicella zoster virus (VZV), human cytomegalovirus (HCMV) or human herpesvirus 8 (HHV-8), preferably HCMV, and the flaviviridae is selected from HCV, West nile virus or Yellow Fever virus.

It is to be understood, that all the viruses mentioned above, also comprise drug resistant virus strains.

Examples of infective diseases are AIDS, Alveolar Hydatid Disease (AHD, Echinococcosis), Amebiasis (Entamoeba histolytica Infection), Angiostrongylus Infection, Anisakiasis, Anthrax, Babesiosis (Babesia Infection), Balantidium Infection (Balantidiasis), Baylisascaris Infection (Raccoon Roundworm), Bilharzia (Schistosomiasis), Blastocystis hominis Infection (Blastomycosis), Borreliosis, Botulism, Brainerd Diarrhea, Brucellosis, BSE (Bovine Spongiform Encephalopathy), Candidiasis, Capillariasis (Capillaria Infection), CFS (Chronic Fatigue Syndrome), Chagas Disease (American Trypanosomiasis), Chickenpox (Varicella-Zoster virus), Chlamydia pneumoniae Infection, Cholera, CJD (Creutzfeldt-Jakob Disease), Clonorchiasis (Clonorchis Infection), CLM (Cutaneous Larva Migrans, Hookworm Infection), Coccidioidomycosis, Conjunctivitis, Coxsackievirus A16 (Hand, Foot and Mouth Disease), Cryptococcosis, Cryptosporidium Infection (Cryptosporidiosis), Culex mosquito (Vector of West Nile Virus), Cyclosporiasis (Cyclospora Infection), Cysticercosis (Neurocysticercosis), Cytomegalovirus Infection, Dengue / Dengue Fever, Dipylidium Infection (Dog and Cat Flea Tapeworm), Ebola Virus Hemorrhagic Fever, Echinococcosis (Alveolar Hydatid Disease), Encephalitis, Entamoeba coli Infection, Entamoeba dispar Infection, Entamoeba hartmanni Infection, Entamoeba histolytica Infection (Amebiasis), Entamoeba polecki Infection, Enterobiasis (Pinworm Infection), Enterovirus Infection (non-polio), Epstein-Barr Virus Infection, Escherichia coli Infection, Foodborne Infection, Foot and mouth Disease, Fungal Dermatitis, Gastroenteritis, Group A streptococcal Disease, Group B streptococcal Disease, Hansen's Disease (Leprosy), Hantavirus Pulmonary Syndrome, Head Lice Infestation (Pediculosis), Helicobacter pylori Infection, Hematologic Disease, Hendra Virus Infection, Hepatitis (HCV, HBV), Herpes Zoster (Shingles), HIV Infection, Human Ehrlichiosis, Human Parainfluenza Virus Infection, Influenza, Isosporiasis (Isospora Infection), Lassa Fever, Leishmaniasis, Kala-azar (Kala-azar, Leishmania Infection), Leprosy, Lice (Body lice, Head lice, Pubic lice), Lyme Disease, Malaria, Marburg Hemorrhagic Fever, Measles, Meningitis, Mosquito-borne Diseases, Mycobacterium avium Complex (MAC) Infection, Naegleria Infection, Nosocomial Infections, Nonpathogenic Intestinal Amebae Infection, Onchocerciasis (River Blindness), Opisthorciasis (Opisthorcis Infection), Parvovirus Infection, Plague, PCP (Pneumocystis carinii Pneumonia), Polio, Q Fever, Rabies, Respiratory Syncytial Virus (RSV) Infection, Rheumatic Fever, Rift Valley Fever, Rotavirus Infection, Roundworms Infection, Salmonellosis, Salmonella Enteritidis, Scabies, Shigellosis, Shingles, Sleeping Sickness, Smallpox, Streptococcal Infection, Tapeworm Infection (Taenia Infection), Tetanus, Toxic Shock Syndrome, Tuberculosis, Ulcers (Peptic Ulcer Disease), Valley Fever, Vibrio parahaemolyticus Infection, Vibrio vulnificus Infection, Viral Hemorrhagic Fever, Warts, Waterborne infectious Diseases, West Nile Virus Infection (West Nile Encephalitis), Whooping Cough, Yellow Fever.

### Immunological diseases

Immunological diseases are, for instance, asthma and diabetes, rheumatic and autoimmune diseases, AIDS, rejection of transplanted organs and tissues (cf. below), rhinitis, chronic obstructive pulmonary diseases, osteoporisis, ulcerative colitis, sinusitis, lupus erythematosus, recurrent infections, atopic dermatitis / eczema and occupational allergies, food allergies, drug allergies, severe anaphylactic reactions, anaphylaxis, and other manifestations of allergic disease, as well as uncommon problems such as primary immunodeficiencies, including antibody deficiency states, cell mediated immunodeficiencies (e.g., severe combined immunodeficiency, DiGeorge syndrome, Hyper-IgE syndrome, Wiskott-Aldrich syndrome, ataxia-telangiectasia), immune mediated cancers, and white cell defects.

In autoimmune diseases, such as systemic lupus erythematosus, rheumatoid arthritis (RA), multiple sclerosis (MS), immune-mediated or type 1 diabetes mellitus, immune mediated glomerulonephritis, scleroderma, pernicious anemia, alopecia, pemphigus, pemphigus vulgaris, myasthenia gravis, inflammatory bowel diseases, Crohn's disease, psoriasis, autoimmune thyroid diseases, and Hashimoto's disease, dermatomyositis, Goodpasture syndrome, myasthenia gravis pseudoparalytica, ophtalmia sympatica, phakogene uveitis, chronical agressive hepatitis, primary billiary cirrhosis, autoimunehemolytic anemy, Werlhof disease, specific cells uncontrollably attack the body's own tissues and organs (autoimmunity), producing inflammatory reactions and other serious symptoms and diseases.

Hashimoto's thyroiditis is one of the most common autoimmune diseases. "Autoimmune disease" refers to a category of more than 80 chronic illnesses, each very different in nature, that can affect everything from the endocrine glands (like the thyroid) to organs like the kidneys, as well as to the digestive system.

There are many different autoimmune diseases, and they can each affect the body in different ways. For example, the autoimmune reaction is directed against the brain in multiple sclerosis and the gut in Crohn's disease. In other autoimmune diseases such as systemic lupus erythematosus (lupus), affected tissues and organs may vary among individuals with the same disease. One person with lupus may have affected skin and joints whereas another may have affected skin, kidney, and lungs. Ultimately, damage to certain tissues by the immune system may be permanent, as with destruction of insulin-producing cells of the pancreas in Type 1 diabetes mellitus.

### Cardiovascular diseases

Cardiovascular diseases are, for example, cardiac hypertrophy, adult congenital heart disease, aneurysm, stable angina, unstable angina, angina pectoris, angioneurotic edema, aortic valve stenosis, aortic aneurysm, arrhythmia, arrhythmogenic right ventricular dysplasia, arteriosclerosis, arteriovenous malformations, atrial fibrillation, Behçet syndrome, bradycardia, cardiac tamponade, cardiomegaly, congestive cardiomyopathy, hypertrophic cardiomyopathy, restrictive cardiomyopathy, cardiovascular disease prevention, carotid stenosis, cerebral hemorrhage, Churg-Strauss syndrome, diabetes, Ebstein's Anomaly, Eisenmenger complex, cholesterol embolism, bacterial endocarditis, fibromuscular dysplasia, congenital heart defects, heart diseases, congestive heart failure, heart valve diseases, heart attack, epidural hematoma, hematoma, subdural, Hippel-Lindau disease, hyperemia, hypertension, pulmonary hypertension, hypertrophic growth, left ventricular hypertrophy, right ventricular hypertrophy, hypoplastic left heart syndrome, hypotension, intermittent claudication, ischemic heart disease, Klippel-Trenaunay-Weber syndrome, lateral medullary syndrome, long QT syndrome mitral valve prolapse, Moya Moya disease, mucocutaneous lymph node syndrome, myocardial infarction, myocardial ischemia, myocarditis, pericarditis, peripheral vascular diseases, phlebitis, polyarteritis nodosa, pulmonary atresia, Raynaud disease, restenosis, Sneddon syndrome, stenosis, superior vena cava syndrome, syndrome X, tachycardia, Takayasu's arteritis, hereditary hemorrhagic telangiectasia, telangiectasis, temporal arteritis, tetralogy of fallot, thromboangiitis obliterans, thrombosis, thromboembolism, tricuspid atresia, varicose veins, vascular diseases, vasculitis, vasospasm, ventricular fibrillation, Williams syndrome, peripheral vascular disease, varicose veins and leg ulcers, deep vein thrombosis, Wolff-Parkinson-White syndrome.

Preferred are cardiac hypertrophy, adult congenital heart disease, aneurysms, angina, angina pectoris, arrhythmias, cardiovascular disease prevention, cardiomyopathies, congestive heart failure, myocardial infarction, pulmonary hypertension, hypertrophic growth, restenosis, stenosis, thrombosis and arteriosclerosis.

### Proliferative disease

In yet another preferred embodiment, the proliferative disease is selected from the group comprising:
adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, cervix, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's lymphomas), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberg disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma, tongue cancer, invasive ductal carcinoma, invasive lobular carcinoma, ductal carcinoma *in situ,* lobular carcinoma *in situ,* small-cell lung carcinoma, non-small-cell lung carcinoma, bronchial adenoma, pleuropulmonary blastoma, mesothelioma, brain stem glioma, hypophtalmic glioma, cerebellar astrocytoma, cerebral astrocytoma, neuro-ectodermal tumours, pineal tumors, sarcoma of the uterus, salivary gland cancers, anal gland adenocarcinomas, mast cell tumors, pelvis tumours, ureter tumours, hereditary papillary renal cancers, sporadic papillary renal cancers, intraocular melanoma, hepatocellular carcinoma (liver cell carcinomas with or without fibrolamellar variant), cholangiocarcinoma (intrahepatic bile duct carcinoma), mixed hepatocellular cholangiocarcinoma, squamous cell carcinoma, malignant melanoma, Merkel cell skin cancer, non-melanoma skin cancer, hypopharyngeal cancer, nasopharyngeal cancer, oropharyngeal cancer, oral cavity cancer, squamous cell cancer, oral melanoma, AIDS-related lymphoma, cutaneous T-cell lymphoma, lymphoma of the central nervous system, malignant fibrous histiocytoma, lymphosarcoma, rhabdomyosarcoma, malignant histiocytosis, fibrosarcoma, hemangiosarcoma, hemangiopericytoma, leiomyosarcoma, canine mammary carcinoma, and feline mammary carcinoma.

Preferred are the following cancer types: Leukemias including but not limited to chronic lymphocytic leukemia, chronic myelogenous leukemia, acute lymphoblastic leukemia, acute myeloid leukemia, mixed lineage leukemia, bladder cancer, breast cancer, breast carcinoma, cancer of the central nervous system, colon carcinoma, gastric cancer, lung cancer, kidney cancer, melanoma, head and neck tumors (tumors of the ear, nose and throat area), ovarian cancer, ovarial carcinoma, cervical cancer, cervical carcinoma, glioblastomas, pancreatic cancer, pancreatic carcinoma, prostate cancer, stomach cancer, skin cancer, skin testis cancer, Hodgkin's lymphoma, liver cancer, liver metastases and renal cell carcinomas.

### Inflammation

The inflammation is mediated preferably by the cytokines TNF-α, IL-1ß, GM-CSF, IL-6 and/or IL-8.

As described above, the compounds according to general formula (I) are pharmaceutically active agents for prophylaxis and/or treatment of inflammatory diseases. Thus, these compounds are used for the manufacture of a pharmaceutical formulation for prophylaxis and/or treatment of inflammations and inflammatory diseases in mammals, including humans.

Inflammatory diseases can emanate from infectious and non-infectious inflammatory conditions which may result from infection by an invading organism or from irritative, traumatic, metabolic, allergic, autoimmune, or idiopathic causes as shown in the following list.
I. Acute infections

| | |
|---|---|
| A. Viral | B. Bacterial |

II. Noninfectious causes
III. Chronic (granulomatous) diseases

| | |
|---|---|
| A. Bacterial | B. Spirochetal |
| C. Mycotic (Fungal) | D. Idiopathic |

IV. Allergic, immune, and idiopathic disorders
A. Hypersensitivity reactions
B. Immune and idiopathic disorders

V. Miscellaneous inflammatory conditions
A. Parasitic infections
B. Inhalation causes:
   - Acute (thermal) injury
   - Pollution and inhalant allergy
   - Carcinogens
C. Radiation injury:
   - Radionecrosis

Inflammations or inflammatory diseases can be caused by invading organisms such as viruses, bacteria, prions, and parasites and by irritative, traumatic, metabolic, allergic, autoimmune, or idiopathic reasons.

The following bacteria are known to cause inflammatory diseases: mycoplasma pulmonis (causes e.g. chronic lung diseases (CLD), murine chronic respiratory disease), ureaplasma urealyticum (causes pneumonia in newborns), mycoplasma pneumoniae and chlamydia pneumoniae (cause chronic asthma), C. pneumoniae (causes atherosclerosis, pharyngitis to pneumonia with empyema, human coronary heart disease), Helicobacter pylori (human coronary heart disease, stomach ulcers).

The following viruses are known to cause inflammatory diseases: herpesviruses especially cytomegalovirus (causes human coronary heart disease).
inflammatory diseases also includes the central nervous system (CNS), inflammatory rheumatic diseases, inflammatory diseases of blood vessels, inflammatory diseases of the middle ear, inflammatory bowel diseases, inflammatory diseases of the skin, inflammatory disease uveitis, inflammatory diseases of the larynx.

Examples for inflammatory diseases of the central nervous system (CNS) are algal disorders, protothecosis, bacterial disorders, abscessation, bacterial meningitis, idiopathic inflammatory disorders, eosinophilic meningoencephalitis, feline polioencephalomyelitis, granulomatous meningoencephalomyelitis, meningitis, steroid responsive meningitis-arteritis, miscellaneous meningitis / meningoencephalitis, meningoencephalitis in greyhounds, necrotizing encephalitis, pyogranulomatous meningoencephalomyelitis, shaker dog disease, mycotic diseases of the CNS, parasitic encephalomyelitis, prion protein induced diseases, feline spongiform encephalopathy, protozoal encephalitis-encephalomyelitis, toxoplasmosis, neosporosis, sarcocystosis, encephalitozoonosis, trypanosomiasis, acanthamebiasis, babesiosis, leishmaniasis, rickettsial disorders, rocky mountain spotted fever, canine ehrlichiosis, salmon poisoning, viral disorders, Aujeszky's disease, Borna disease, canine herpes virus encephalomyelitis, canine distemper encephalomyelitis, canine distemper encephalomyelitis in immature animals, chronic relapsing encephalomyelitis, post-vaccinal canine distemper encephalitis, feline immunodeficiency virus, feline infectious peritonitis, feline leukemia virus, infectious canine hepatitis, La Crosse virus encephalitis, parvovirus encephalitis, rabies, post-vaccinal rabies.

Examples for inflammatory rheumatic diseases are rheumatoid arthritis, scleroderma, lupus, polymyositis, dermatomyositis, psoriatic arthritis, ankylosing spondylitis, Reiters's syndrome, juvenile rheumatoid arthritis, bursitis, tendinitis (tendonitis), and fibromyositis.

Examples for inflammatory diseases of blood vessels are vasculitis, autoantibodies in vasculitis, microscopic polyangiitis, giant cell arteritis, Takayasu's arteritis, vasculitis of the central nervous system, thromboangiitis obliterans (Buerger's Disease), vasculitis secondary to bacterial, fungal, and parasitic infection, vasculitis and rheumatoid arthritis, vasculitis in systemic lupus erythematosus, vasculitis in the idiopathic inflammatory myopathies, relapsing polychondritis, systemic vasculitis in sarcoidosis, vasculitis and malignancy, and drug-induced vasculitis.

Examples for inflammatory diseases of the middle ear are acute suppurative otitis media, bullous myringitis, granular myringitis, and chronic suppurative otitis media, which can manifest as mucosal disease, cholesteatoma, or both.

Examples for inflammatory bowel diseases are ulcerative colitis, Crohn's disease.

Examples for inflammatory diseases of the skin are acute inflammatory dermatoses, urticaria (hives), spongiotic dermatitis, allergic contact dermatitis, irritant contact dermatitis, atopic dermatitis, erythemal multiforme (EM minor), Stevens-Johnson syndrome (SJS, EM major), toxic epidermal necrolysis (TEN), chronic inflammatory dermatoses, psoriasis, lichen planus, discoid lupus erythematosus, and acne vulgaris.

Uveitis are inflammations located in and/or on the eye and may be associated with inflammation elsewhere in the body. In most circumstances, patients who have uveitis as part of a disease elsewhere in the body are aware of that illness. The majority of patients with uveitis do not have an apparent associated systemic illness. Causes of uveitis can be infectious causes, masquerade syndromes, suspected immune-mediated diseases, and/or syndromes confined primarily to the eye.

The following viruses are associated with inflammations: human immunodeficiency virus-I, herpes simplex virus, herpes zoster virus, and cytomegalovirus.

Bacterial or spirochetal caused, induced, initiated and/or enhanced inflammations are tuberculosis, leprosy, proprionobacterium, syphilis, Whipple's disease, leptospirosis, brucellosis, and Lyme disease.

Parasitic (protozoan or helminthic) caused, induced, initiated and/or enhanced inflammations are toxoplasmosis, acanthameba, toxocariasis, cysticercosis, onchocerciasis.

Examples of inflammatory diseases caused, induced, initiated and/or enhanced by fungi are histoplasmosis, coccidioidomycosis, candidiasis, aspergillosis, sporotrichosis, blastomycosis, and cryptococcosis.

Masquerade syndromes are, for instance, leukemia, lymphoma, retinitis pigmentosa, and retinoblastoma.

Suspected immune-mediated diseases can be selected from the group comprising ankylosing spondylitis, Behçet's disease, Crohn's disease, drug or hypersensitivity reaction, interstitial nephritis, juvenile rheumatoid arthritis, Kawasaki's disease, multiple sclerosis, psoriatic arthritis, Reiter's syndrome, relapsing polychondritis, sarcoidosis, Sjogren's syndrome, systemic lupus erythematosus, ulcerative colitis, vasculitis, vitiligo, Vogt Koyanagi Harada syndrome.

Syndromes confined primarily to the eye are, for instance, acute multifocal placoid pigmentary epitheliopathy, acute retinal necrosis, birdshot choroidopathy, Fuchs' heterochromic cyclitis, glaucomatocyclitic crisis, lens-induced uveitis, multifocal choroiditis, pars planitis, serpiginous choroiditis, sympathetic ophthalmia, and trauma.

Examples for inflammatory diseases of the larynx are gastroesophageal (laryngopharyngeal) reflux disease, pediatric laryngitis, acute laryngeal infections of adults, chronic (granulomatous) diseases, allergic, immune, and idiopathic disorders and miscellaneous inflammatory conditions.

Pediatric laryngitis is known as acute (viral or bacterial) infection such as laryngotracheitis (croup), supraglottitis (epiglottitis), diphtheria, and noninfectious causes are for example spasmodic croup and traumatic laryngitis.

Acute laryngeal infections of adults are, for instance, viral laryngitis, common upper respiratory infection, laryngotracheitis, herpes simplex, bacterial laryngitis, supraglottitis, laryngeal abscess, and gonorrhea.

Chronic (granulomatous) diseases can be selected from the group comprising bacterial diseases, tuberculosis, leprosy, scleroma, actinomycosis, tularemia, glanders, spirochetal (syphilis) diseases, mycotic (fungal) diseases, candidiasis, blastomycosis, histoplasmosis, coccidiomycosis, aspergillosis, idiopathic diseases, sarcoidosis, and Wegener's granulomatosis.

Allergic, immune, and idiopathic disorders are, for example, hypersensitivity reactions, angioedema, Stevens-Johnson syndrome, immune and idiopathic disorders, infections of the immunocompromised host, rheuatoid arthritis, systeic lupus erythematosus, cicatricial pemphigoid, relapsing polychondritis, Sjogren's syndrome, and amyloidosis.

Miscellaneous inflammatory conditions are, for instance, parasitic infections, trichinosis, leishmaniasis, schistosomiasis, syngamus laryngeus, inhalation laryngitis, acute (thermal) injury, pollution and inhalant allergy, carcinogens, radiation injury, radiation laryngitis, radionecrosis, vocal abuse, vocal-cord hemorrhage, muscle tension dysphonias, and contact ulcer and granuloma.

### Stroke

The inventive compounds according to the general formula (I) as well as pharmaceutically acceptable salts thereof are also useful for treatment of stroke.

The compounds according to the general formula (I) as well as pharmaceutically acceptable salts thereof are used as an inhibitor for a protein kinase, preferably as an inhibitor for a cellular protein kinase.

Preferred, said cellular protein kinase consists of Cyclin-dependent protein kinases (CDKs).

The cyclin-dependent protein kinase can be selected from the group comprising: CDK1, CDK2, CDK3, CDK4, CDK5, CDK6, CDK7, CDK8, CDK9, CDK10, CDK11, CrkRS (Crk7, CDC2-related protein kinase 7), CDKL1 (cyclin-dependent kinase-like 1); KKIALRE, CDKL2 (cyclin-dependent kinase-like 2), KKIAMRE, CDKL3 (cyclin-dependent kinase-like 3), NKIAMRE, CDKL4, similar to cyclin-dependent kinase-like 1, CDC2L1 (cell division cycle 2-like 1), PITSLRE B, CDC2L1 (cell division cycle 2-like 1), PITSLRE A, CDC2L5 (cell division cycle 2-like 5), PCTK1 (PCTAIRE protein kinase 1), PCTK2 (PCTAIRE protein kinase 2), PCTK3 (PCTAIRE protein kinase 3) or PFTK1 (PFTAIRE protein kinase 1).

Particularly preferred, said cyclin-dependent protein kinase is CDK9. Thus, the compounds according to the general formula (I) as well as pharmaceutically acceptable salts thereof are used as an inhibitor for CDK9.

Furthermore, the compounds according to the invention show a high potency (demonstrated by a low IC₅₀ value) for inhibiting CDK9 activity. In context of the present invention, the IC₅₀ value with respect to CDK9 can be determined by the methods described in the method section below. Preferably, it is determined according to the method described in section 3.6.

Surprisingly it turned out that the compounds according to the general formula (I) as well as pharmaceutically acceptable salts thereof selectively inhibit CDK9 in comparison to other protein kinases and in comparison to other cyclin-dependent protein kinases. Thus, the compounds according to the general formula (I) as well as pharmaceutically acceptable salts thereof are used as selective inhibitors for CDK9.

Particularly preferred compounds of the present invention according to formula (I) show a stronger CDK9 than CDK2 inhibition. In context of the present invention, the IC₅₀ value with respect to CDK2 can be determined by the methods described in the method section below. Preferably, it is determined according to the method described in section 3.5.

Further, the compounds of the present invention according to formula (I) mediate an anti-proliferative activity in tumor cell lines such as HeLa, MaTu/ADR, H460, DU145, CACO-2 or B16F10. In context of the present invention, the IC₅₀ values of the compounds with respect to these cell lines is preferably determined according to the methods described below..

Preferred compounds of the present invention mediate a particularly strong anti-proliferative activity in tumor cell line HeLa.

As used herein, a kinase "inhibitor" refers to any compound capable of downregulating, decreasing, suppressing or otherwise regulating the amount and/or activity of a kinase. Inhibition of these kinases can be achieved by any of a variety of mechanisms known in the art, including, but not limited to binding directly to the kinase polypeptide, denaturing or otherwise inactivating the kinase, or inhibiting the expression of the gene (e.g., transcription to mRNA, translation to a nascent polypeptide, and/or final polypeptide modifications to a mature protein), which encodes the kinase. Generally, kinase inhibitors may be proteins, polypeptides, nucleic acids, small molecules, or other chemical moieties.

As used herein the term "inhibiting" or "inhibition" refers to the ability of a compound to downregulate, decrease, reduce, suppress, inactivate, or inhibit at least partially the activity of an enzyme, or the expression of an enzyme or protein and/or the virus replication.

The compounds shown explicitly in Table 1 are preferred to be used within the methods or indications disclosed herein. Another aspect of the present invention is that at least one compound according to the general formula (I) used as a pharmaceutically active agent may be administered in combination with further therapeutic compounds.

For the indication HIV compounds according to the general formula (I), preferably those for CDK9 as shown in Table 4, may be administered in combination with anti-retroviral drugs, selected from the following five classes:
1) Nucleoside reverse transcriptase inhibitors (NRTIs),
2) Non-nucleoside reverse transcriptase inhibitors (NNRTIs),
3) Protease inhibitors (PIs),
4) Fusion inhibitors or
5) Immune stimuli.

### Pharmaceutical Compositions and Drug Combinations

Another aspect of the present invention relates to drug combinations and pharmaceutical compositions comprising at least one compound of general formula (I) as active ingredient together with at least one pharmaceutically acceptable carrier, excipient and/or diluent and optionally together with one or more other anti-tumor agents or with one or more anti-retroviral drugs. As used herein the term "drug combination" refers to a combination of at least to pharmaceutically active agents or therapeutic agents with or without further ingredients, carrier, diluents and/or solvents. As used herein the term "pharmaceutical composition" refers to a galenic formulation of at least one pharmaceutically active agent together with at least one further ingredient, carrier, diluent and/or solvent.

Compounds of formula (I) may be administered as the sole pharmaceutical agent or in combination with one or more additional therapeutic agents, wherein the drug combination causes no unacceptable adverse effects. This combination therapy includes administration of a single pharmaceutical dosage formulation, which contains a compound of formula (I) and one or more additional therapeutic agents in form of a single pharmaceutical composition, as well as administration of the compound of formula (I) and each additional therapeutic agent in its own separate pharmaceutical dosage formulation, i.e. in its own separate pharmaceutical composition. For example, a compound of formula (I) and a therapeutic agent may be administered to the patient together in a single oral dosage composition such as a tablet or capsule, or each agent may be administered in separate pharmaceutical compositions.

Where separate pharmaceutical compositions are used, the compound of formula (I) and one or more additional therapeutic agents may be administered at essentially the same time (e.g., concurrently) or at separately staggered times (e.g., sequentially).

In particular, the compounds of the present invention may be used in fixed or separate pharmaceutical compositions with other anti-tumor agents such as alkylating agents, anti-metabolites, plant-derived anti-tumor agents, hormonal therapy agents, topoisomerase inhibitors, camptothecin derivatives, kinase inhibitors, targeted drugs, antibodies, interferons and/or biological response modifiers, anti-angiogenic compounds, and other anti-tumor drugs. In this regard, the following is a non-limiting list of examples of secondary agents that may be used in combination with the compounds of the present invention:
- Alkylating agents include, but are not limited to, nitrogen mustard *N*-oxide, cyclophosphamide, ifosfamide, thiotepa, ranimustine, nimustine, temozolomide, altretamine, apaziquone, brostallicin, bendamustine, carmustine, estramustine, fotemustine, glufosfamide, mafosfamide, and mitolactol; platinum-coordinated alkylating compounds include, but are not limited to, cisplatin, carboplatin, eptaplatin, lobaplatin, nedaplatin, oxaliplatin, and satraplatin;
- Anti-metabolites include, but are not limited to, methotrexate, 6-mercaptopurine riboside, mercaptopurine, 5-fluorouracil alone or in combination with leucovorin, tegafur, doxifluridine, carmofur, cytarabine, cytarabine ocfosfate, enocitabine, gemcitabine, fludarabin, 5-azacitidine, capecitabine, cladribine, clofarabine, decitabine, eflornithine, ethynylcytidine, cytosine arabinoside, hydroxyurea, melphalan, nelarabine, nolatrexed, ocfosfite, disodium premetrexed, pentostatin, pelitrexol, raltitrexed, triapine, trimetrexate, vidarabine, vincristine, and vinorelbine;
- Hormonal therapy agents include, but are not limited to, exemestane, Lupron, anastrozole, doxercalciferol, fadrozole, formestane, 11-beta hydroxysteroid dehydrogenase 1 inhibitors, 17-alpha hydroxylase/17,20 lyase inhibitors such as abiraterone acetate, 5-alpha reductase inhibitors such as finasteride and epristeride, anti-estrogens such as tamoxifen citrate and fulvestrant, Trelstar, toremifene, raloxifene, lasofoxifene, letrozole, anti-androgens such as bicalutamide, flutamide, mifepristone, nilutamide, Casodex, and anti-progesterones and combinations thereof;
- Plant-derived anti-tumor substances include, e.g., those selected from mitotic inhibitors, for example epothilones such as sagopilone, ixabepilone and epothilone B, vinblastine, vinflunine, docetaxel, and paclitaxel;
- Cytotoxic topoisomerase inhibiting agents include, but are not limited to, aclarubicin, doxorubicin, amonafide, belotecan, camptothecin, 10-hydroxycamptothecin, 9-aminocamptothecin, diflomotecan, irinotecan, topotecan, edotecarin, epimbicin, etoposide, exatecan, gimatecan, lurtotecan, mitoxantrone, pirambicin, pixantrone, rubitecan, sobuzoxane, tafluposide, and combinations thereof;
- Immunologicals include interferons such as interferon alpha, interferon alpha-2a, interferon alpha-2b, interferon beta, interferon gamma-1a and interferon gamma-n1, and other immune enhancing agents such as L19-IL2 and other IL2 derivatives, filgrastim, lentinan, sizofilan, TheraCys, ubenimex, aldesleukin, alemtuzumab, BAM-002, dacarbazine, daclizumab, denileukin, gemtuzumab, ozogamicin, ibritumomab, imiquimod, lenograstim, lentinan, melanoma vaccine (Corixa), molgramostim, sargramostim, tasonermin, tecleukin, thymalasin, tositumomab, Vimlizin, epratuzumab, mitumomab, oregovomab, pemtumomab, and Provenge; Merial melanoma vaccine;
- Biological response modifiers are agents that modify defense mechanisms of living organisms or biological responses such as survival, growth or differentiation of tissue cells to direct them to have anti-tumor activity; such agents include, *e.g.,* krestin, lentinan, sizofiran, picibanil, ProMune, and ubenimex;
- Anti-angiogenic compounds include, but are not limited to, acitretin, aflibercept, angiostatin, aplidine, asentar, axitinib, recentin, bevacizumab, brivanib alaninat, cilengtide, combretastatin, DAST, endostatin, fenretinide, halofuginone, pazopanib, ranibizumab, rebimastat, removab, revlimid, sorafenib, vatalanib, squalamine, sunitinib, telatinib, thalidomide, ukrain, and vitaxin;
- Antibodies include, but are not limited to, trastuzumab, cetuximab, bevacizumab, rituximab, ticilimumab, ipilimumab, lumiliximab, catumaxomab, atacicept, oregovomab, and alemtuzumab;
- VEGF inhibitors such as, *e*.*g*., sorafenib, DAST, bevacizumab, sunitinib, recentin, axitinib, aflibercept, telatinib, brivanib alaninate, vatalanib, pazopanib, and ranibizumab; Palladia
- EGFR (HER1) inhibitors such as, *e*.*g*., cetuximab, panitumumab, vectibix, gefitinib, erlotinib, and Zactima;
- HER2 inhibitors such as, *e.g.,* lapatinib, tratuzumab, and pertuzumab;
- mTOR inhibitors such as, *e.g.,* temsirolimus, sirolimus/Rapamycin, and everolimus;
- c-Met inhibitors;
- PI3K and AKT inhibitors;
- CDK inhibitors such as roscovitine and flavopiridol;
- Spindle assembly checkpoints inhibitors and targeted anti-mitotic agents such as PLK inhibitors, Aurora inhibitors (*e*.*g*. Hesperadin), checkpoint kinase inhibitors, and KSP inhibitors;
- HDAC inhibitors such as, *e*.*g*., panobinostat, vorinostat, MS275, belinostat, and LBH589;
- HSP90 and HSP70 inhibitors;
- Proteasome inhibitors such as bortezomib and carfilzomib;
- Serine/threonine kinase inhibitors including MEK inhibitors (such as e.g. RDEA 119) and Raf inhibitors such as sorafenib;
- Farnesyl transferase inhibitors such as, *e*.*g*., tipifarnib;
- Tyrosine kinase inhibitors including, *e*.*g*., dasatinib, nilotibib, DAST, bosutinib, sorafenib, bevacizumab, sunitinib, AZD2171, axitinib, aflibercept, telatinib, imatinib mesylate, brivanib alaninate, pazopanib, ranibizumab, vatalanib, cetuximab, panitumumab, vectibix, gefitinib, erlotinib, lapatinib, tratuzumab, pertuzumab, and c-Kit inhibitors; Palladia, masitinib
- Vitamin D receptor agonists;
- Bcl-2 protein inhibitors such as obatoclax, oblimersen sodium, and gossypol;
- Cluster of differentiation 20 receptor antagonists such as, *e*.*g*., rituximab;
- Ribonucleotide reductase inhibitors such as, *e*.*g*., gemcitabine;
- Tumor necrosis apoptosis inducing ligand receptor 1 agonists such as, *e*.*g*., mapatumumab;
- 5-Hydroxytryptamine receptor antagonists such as, *e*.*g*., rEV598, xaliprode, palonosetron hydrochloride, granisetron, Zindol, and AB-1001;
- Integrin inhibitors including alpha5-beta1 integrin inhibitors such as, *e*.*g*., E7820, JSM 6425, volociximab, and endostatin;
- Androgen receptor antagonists including, *e.g.,* nandrolone decanoate, fluoxymesterone, Android, Prost-aid, andromustine, bicalutamide, flutamide, apocyproterone, apo-flutamide, chlormadinone acetate, Androcur, Tabi, cyproterone acetate, and nilutamide;
- Aromatase inhibitors such as, *e.g.,* anastrozole, letrozole, testolactone, exemestane, aminoglutethimide, and formestane;
- Matrix metalloproteinase inhibitors;
- Other anti-cancer agents including, *e*.*g*., alitretinoin, ampligen, atrasentan bexarotene, bortezomib, bosentan, calcitriol, exisulind, fotemustine, ibandronic acid, miltefosine, mitoxantrone, I-asparaginase, procarbazine, dacarbazine, hydroxycarbamide, pegaspargase, pentostatin, tazaroten, velcade, gallium nitrate, canfosfamide, darinaparsin, and tretinoin.

The compounds of the present invention may also be employed in cancer treatment in conjunction with radiation therapy and/or surgical intervention.

Furthermore, the compounds of formula (I) may be utilized, as such or in compositions, in research and diagnostics, or as analytical reference standards, and the like, which are well known in the art.
at least one inventive compound according to general formula (I) and/or pharmaceutically acceptable salts thereof may be combinated with at least one anti-retroviral drug, especially at least one of the drugs mentioned above.

The pharmaceutical compositions according to the present invention comprise at least one compound according to the present invention as an active ingredient together with at least one pharmaceutically acceptable (i.e. non-toxic) carrier, excipient and/or diluent. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, pharmaceutical preparations, which in addition to typical vehicles and/or diluents contain at least one compound according to the present invention and/or a pharmaceutical acceptable salt thereof as active ingredient may be applied as dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application.

The pharmaceutical compositions according to the present invention containing at least one compound according to the present invention and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the 4,6-disubstituted pyrimdine derivative according to the general formula (I) or analogues compound thereof or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix.

Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn, rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat, corn, rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

The inventive compounds are especially useful to prevent formation of metastases. Moreover the inventive compounds are especially useful to treat drug resistant and multi drug resistant cancers and tumours.

### Examples

### Preparation of compounds:

### Abbreviations used in the description of the chemistry and in the Examples that follow are:

CDCl₃ (deuterated chloroform); cHex (cyclohexane); DCM (dichloromethane); DIPEA (di-iso-propylethylamine); DMF (dimethylformamide); DMSO (dimethyl sulfoxide); eq (equivalent); ES (electrospray); EtOAc (ethyl acetate); EtOH (ethanol); iPrOH (iso-propanol); MeOH (methanol); MS (mass spectrometry); NMR (nuclear magnetic resonance); Pd(dppf)Cl₂ ([1,1'-bis(diphenylphosphino)ferrocene]dichloro palladium(II) complex with dichloromethane); iPrOH (iso-propanol); RT (room temperature); sat. aq. (saturated aqueous); SiO₂ (silica gel); TFA (trifluoroacetic acid); THF (tetrahydrofuran).

### Preparative Examples

### Intermediates

### Intermediate 1: 3-[(4-Chloro-1,3,5-triazin-2-yl)amino]benzenemethanesulfon-amide (A1)

To a solution of 2,4-dichloro-1,3,5-triazine (1.0 eq) in dry DMF (0.7 M) at 0°C under N₂ atmosphere was added a solution of (3-aminophenyl)-methanesulfonamide (1.0 eq) in dry DMF (0.7 M). The reaction mixture was stirred for 2.5 h at 0°C. Then water was added and the aqueous solution was neutralized with sat. aq. NaHCO₃ solution. The aqueous layer was extracted with EtOAc and the combined organic layers were dried over Na₂SO₄. Evaporation of solvent gave **A1** as a white solid which was used in the next step without further purification. ¹H NMR (400MHz, d₆-DMSO, 300K) δ 4.26 (s, 2H), 6.89 (s, 2H), 7.15 (d, J = 7.4 Hz, 1H), 7.36 (d, J = 7.4 Hz, 1H), 7.66 (d, J = 7.4 Hz, 1 H), 8.64 (s, 1 H), 9.75 (br. s, 1 H), 10.83 (s, 1 H). MS (ES) C₁₀H₁₀ClN₅O₂S requires: 299, found: 300 (M+H)⁺.

### Intermediate 2: 3-[(4-Chloro-1,3,5-triazin-2-yl)amino]benzenesulfonamide (A2)

**A2** was prepared following the general procedure reported for **A1** using 2,4-dichloro-1,3,5-triazine and 3-aminobenzenesulfonamide as reacting agents. The crude product was purified by flash chromatography on silica gel (cHex/EtOAc = 20:1 to 1:20) to yield the desired product **A2** as a white solid (35%). ¹H NMR (400MHz, d₆-DMSO, 300K) δ 7.41 (s, 2H), 7.59 (m, 2H), 7.85 (d, J = 6.9Hz, 1 H), 8.21 (br.s, 1 H), 8.69 (s, 1 H), 11.02 (s, 1 H). MS (ES) C₉H₈ClN₅O₂S requires: 285, found: 286 (M+H)⁺.

### Intermediate 3: 4-Chloro-N-(3-nitrophenyl)-1,3,5-triazine-2-amine (A3)

**A3** was prepared following the general procedure reported for **A1** using 2,4-dichloro-1,3,5-triazine and 3-nitroaniline as reacting agents. ¹H-NMR (400MHz, d₆-DMSO, 300K) δ 7.67 (t, J = 8.1 Hz, 1H), 7.98 (dd, J = 8.1 Hz, J = 2.2 Hz, 1H), 8.04 (dd, J = 8.1 Hz, J = 2.2 Hz, 1 H), 8.69 (m, 1 H), 8.77 (m, 1 H), 11.17 (s, 1 H). MS (ES) C₉H₆ClN₅O₂ requires: 251, found: 252 (M+H)⁺.

### Intermediate 4: 2-(3-Aminophenyl)ethanesulfonamide (A4)

The compound **A4** has been obtained by reduction of 2-(3-nitrophenyl)-ethanesulfonamide according to the procedure described in WO2009/076140, and the nitro compound from 2-(3-nitrophenyl)ethanol according to J.Med.Chem. 45 (2002), 567-583.

### Intermediate 5: 2-[3-((4-Chloro-1,3,5-triazin-2-yl)amino)phenyl]ethanesulfon-amide (A5)

**A5** was prepared following the general procedure reported for **A1** using 2,4-dichloro-1,3,5-triazine and 2-(3-aminophenyl)ethanesulfonamide **A4** as reacting agents. The crude product was purified by flash chromatography on silica gel (DCM/MeOH = 100:0 to 4:1) to yield the desired product **A5** (54%, 65% purity) as a brown solid. MS (ES) C₁₁H₁₂ClN₅O₂S requires: 313, found: 314 (M+H)⁺.

### Intermediate 6: 3-[(4-Chloro-1,3,5-triazin-2-yl)amino]benzamide (A6)

**A6** was prepared following the general procedure reported for **A1** using 2,4-dichloro-1,3,5-triazine and 3-aminobenzamide as reacting agents. MS (ES) C₁₀H₈ClN₅O requires: 249, found: 250 (M+H)⁺.

### Intermediate 7: rac-S-[3-((4-Chloro-1,3,5-triazin-2-yl)amino)phenyl]-N-ethoxycarbonyl-S-methyl-sulfoximide (A7)

A solution of the sulfoximide **A11** (500 mg, 2.064 mmol) in a mixture of iPrOH (12 ml) and THF (12 ml) was cooled to -20°C. Another pre-cooled solution of 2,4-dichloro-1,3,5-triazine (309.5 mg, 2.064 mmol) in the same mixture of solvents (6 ml each) was added at this temperature. After stirring for 1 hour another batch of the triazine (100 mg, 0.67 mmol) was added and stirring at -20°C was continued for 1.5 hours. The mixture was adjusted to pH7 with sat. aq. NaHCO₃-solution and extracted with EtOAc. The organic layer was dried over Na₂SO₄ and concentrated under reduced pressure to give the product **A7** as a yellow solid. Yield: 561.1 mg (76 %); MS (ES) C₁₃H₁₄CIN₅O₃S requires: 355, found: 356 (M+H)⁺.

### Intermediate 8: 6-[(4-Chloro-1,3,5-triazin-2-yl)amino]-2,3-dihydro-1H-indole-1-sulfonamide (A8)

6-Amino-2,3-dihydro-1H-indole-1-sulfonamide hydrochloride was purchased from UkrOrgSynthesis, and used to prepare the title compound by reaction with 2,4-dichloro-1,3,5-triazine as described for **A7.** The product **A8** was obtained as a brown solid. MS (ES) C₁₁H₁₁ClN₆O₂S requires: 326, found: 327 (M+H)⁺.

### Intermediate 9: 2-[2-((4-Pyridyl)methoxy)phenyl]-4,4,5,5-tetramethyl-1,3,2-dioxaborolane (A9)

To a solution of 2-(4,4,5,5-tetramethyl-1,3,2-dioxaborolan-2-yl)phenol (400 mg, 1.82 mmol) in DMF (6 ml) were added 4-picolyl chloride hydrochloride (446 mg, 2.72 mmol) and K₂CO₃ (1.0 g, 7.24 mmol). The mixture was heated at 150 °C for 1 hour in a microwave oven. After addition of water and EtOAc the organic layer was separated, dried over Na₂SO₄, and the solvent removed under reduced pressure. After chromatographic purification (silica gel, DCM/MeOH gradient 100:0 to 90:10) the title compound **A9** was isolated as a white solid (15%). MS (ES) C₁₈H₂₂BNO₃ requires: 311, found: 312 (M+H)⁺.

### Intermediate 10: 2-[2-(4-(tert-Butoxycarbonylamino)butoxy)phenyl]-4,4,5,5-tetramethyl-1,3,2-dioxaborolane (A10)

The title compound **A10** was prepared from 2-(4,4,5,5-tetramethyl-1,3,2-dioxaborolan-2-yl)phenol and tert-butyl N-(4-bromobutyl)carbamate essentially in the same manner as described for **A9.** MS (ES) C₂₁H₃₄BNO₅ requires: 391, found: 392 (M+H)⁺ and 292 (M-COOC(CH₃)₃+H)⁺.

### Intermediate 11: rac-S-(3-Aminophenyl)-N-ethoxycarbonyl-S-methyl-sulfoximide (A11)

The title compound **A11** was prepared from 3-nitro-thioanisol according to the procedure described in WO2008/006560.

### Intermediate 12: tert-Butyl [4-((3-((4-Chloro-1,3,5-triazin-2-yl)amino)phenyl) methylsulfonamido)butyl]carbamate (A12)

Step1: To an ice-cold solution of (3-nitrobenzyl)sulfonyl chloride (7.00 g, 29.7 mmol) in dry DCM (60 mL) were added successively pyridine (4 mL, 3.91 g, 49.4 mmol) and N-Boc-1,4-diaminobutane (5.60 g, 29.7 mmol). The mixture was stirred for 5.5 hours at RT, diluted with DCM, washed with water, dried over MgSO₄, and concentrated under reduced pressure. It was treated with toluene and the solvent removed again. The sulfonamide solidified upon stirring with ether (200 mL). It was collected by filtration and dried *in vacuo*; yield: 5.90 g (51%).
Step2: Raney-Ni (2 g) was added to a solution of the nitro compound from Step 1 (5.90 g, 15.2 mmol) in methanol (250 mL). The mixture was hydrogenated in a Parr reactor for 36 hours at 50°C and 5 bar of hydrogen. The catalyst was removed by filtration through diatom earth and the filtrate concentrated to dryness under reduced pressure to leave the crude aniline which was used for the next step without further purification; yield: 5.0 g (92 %).
Step3: A solution of the aniline from the previous step (2.75 g, 7.7 mmol) in a mixture of THF / iPrOH 1:1 (5 mL) was cooled to -20°C. After addition of DIPEA (2.6 mL) a solution of 2,4-dichloro-1,3,5-triazine (1.15 g, 7.7 mmol) in THF / iPrOH (10 mL) was added dropwise during 30 minutes. The mixture was stirred for another hour at -20°C, the solvent removed *in vacuo,* and the crude chloro-triazine **A12** was used for the next step without further purification.

### Intermediate 13: 4-Chloro-N-(3-(methylsulfonyl)phenyl)-1,3,5-triazin-2-amine (A13)

A solution of 3-(methylsulfonyl)aniline hydrochloride (277 mg, 1.3 mmol) in THF / iPrOH 1:1 (3 mL) was cooled to -30°C and treated with DIPEA (517 mg, 665 µL, 4 mmol). The mixture was added dropwise at -30°C to a cooled solution of 2,4-dichloro-1,3,5-triazine (200 mg, 1.33 mmol) in THF / iPrOH 1:1 (3 mL). It was stirred for another hour at -30°C, concentrated to dryness *in vacuo,* and the crude intermediate **A13** was used for the next step without further purification.

### Intermediate 14: 4-[(4-Chloro-1,3,5-triazin-2-yl)amino]benzenemethanesulfon-amide (A14)

**A14** was prepared from (4-aminophenyl)methanesulfonyl chloride (250 mg, 1.3 mmol) and 2,4-dichloro-1,3,5-triazine (200 mg, 1.3 mmol) in the presence of DIPEA (345 mg, 440 µL, 2.7 mmol) following the procedure reported for **A13.**

### Example Compounds

### Example 1: 3-[(4-(2-Methoxyphenyl)-1,3,5-triazin-2-yl)amino]benzene-methanesulfonamide (B1)

A mixture of **A1** (1.0 eq), 2-methoxyphenylboronic acid (1.5 eq), and K₃PO₄ (2.0 eq) in dioxane/water (50/1, 0.1M) was degassed with a stream of N₂ for 15min. Pd(dppf)Cl₂ (0.1 eq) was added and the reaction mixture heated to 140°C for 1 h in the microwave oven. The mixture was concentrated under reduced pressure and the residue was purified by reverse phase RP-HPLC (column: C18), using H₂O (0.1%TFA) and MeOH (0.1%TFA) as eluents. The desired fractions were lyophilized to afford the title compound (**B1**) (2%) as a white powder. ¹H NMR (400MHz, d₆-DMSO, 300K) δ 3.87 (s, 3H), 4.25 (s, 2H), 6.87 (s, 2H), 7.10 (m, 2H), 7.21 (d, J = 8.4 Hz, 1 H), 7.37 (t, J = 8.0 Hz, 1 H), 7.55 (m, 2H), 7.71 (s, 1 H), 7.84 (m, 1 H), 8.82 (s, 1 H), 10.52 (s, 1 H). MS (ES) C₁₇H-₁₇N₅O₃S requires: 371, found: 372 (M+H)⁺.

The following Examples 2 - 23 were prepared by essentially the same method as described for **B1.**

### Example 2: 3-[(4-(4-Fluoro-2-methoxyphenyl)-1,3,5-triazin-2-yl)amino]-benzene-methanesulfonamide (B2)

**B2** was prepared following the general procedure reported for **B1** using **A1** and 4-fluoro-2-methoxyphenylboronic acid. MS (ES) C₁₇H₁₆FN₅O₃S requires: 389, found: 390 (M+H)⁺.

### Example 3: 3-[(4-(5-Fluoro-2-methoxyphenyl)-1,3,5-triazin-2-yl)amino]-benzene-methanesulfonamide (B3)

**B3** was prepared following the general procedure reported for **B1** using **A1** and 5-fluoro-2-methoxyphenylboronic acid. MS (ES) C₁₇H₁₆FN₅O₃S requires: 389, found: 390 (M+H)⁺.

### Example 4: 3-[(4-(6-Fluoro-2-methoxyphenyl)-1,3,5-triazin-2-yl)amino]-benzene-methanesulfonamide (B4)

**B4** was prepared following the general procedure reported for **B1** using **A1** and 6-fluoro-2-methoxyphenylboronic acid. MS (ES) C₁₇H₁₆FN₅O₃S requires: 389, found: 390 (M+H)⁺.

### Example 5: 3-[(4-(3,5-Difluoro-2-methoxyphenyl)-1,3,5-triazin-2-yl)amino]-benzenemethanesulfonamide (B5)

**B5** was prepared following the general procedure reported for **B1** using **A1** and 3,5-difluoro-2-methoxyphenylboronic acid. MS (ES) C₁₇H₁₅F₂N₅O₃S requires: 407, found: 408 (M+H)⁺.

### Example 6: 3-[(4-(4-Chloro-2-methoxyphenyl)-1,3,5-triazin-2-yl)amino]-benzene-methanesulfonamide (B6)

**B6** was prepared following the general procedure reported for **B1** using **A1** and 4-chloro-2-methoxyphenylboronic acid. MS (ES) C₁₇H₁₅ClN₅O₃S requires: 405, found: 406 (M+H)⁺.

### Example 7: 3-[(4-(5-Chloro-2-methoxyphenyl)-1,3,5-triazin-2-yl)amino]-benzene-methanesulfonamide (B7)

### B7 was prepared following the general procedure reported for B1 using A1 and 5-chloro-2-methoxyphenylboronic acid. MS (ES) C₁₇H₁₅ClN₅O₃S requires: 405, found: 406 (M+H)⁺.

### Example 8: 3-[(4-(2-Methoxy-4-trifluoromethyl-phenyl)-1,3,5-triazin-2-yl)-amino]-benzenemethanesulfonamide (B8)

**B8** was prepared following the general procedure reported for **B1** using **A1** and 2-methoxy-4-trifluoromethyl-phenylboronic acid. MS (ES) C₁₈H₁₆F₃N₅O₃S requires: 439, found: 440 (M+H)⁺.

### Example 9: 3-[(4-(2-Methoxy-5-trifluoromethyl-phenyl)-1,3,5-triazin-2-yl)-amino]-benzenemethanesulfonamide (B9)

**B9** was prepared following the general procedure reported for **B1** using **A1** and 2-methoxy-5-trifluoromethyl-phenylboronic acid. MS (ES) C₁₈H₁₆F₃N₅O₃S requires: 439, found: 440 (M+H)⁺.

### Example 10: 3-[(4-(5-Hydroxymethyl-2-methoxyphenyl)-1,3,5-triazin-2-yl)-amino]-benzenemethanesulfonamide (B10)

B10 was prepared following the general procedure reported for **B1** using **A1** and 5-hydroxymethyl-2-methoxyphenylboronic acid. MS (ES) C₁₈H₁₉N₅O₄S requires: 401, found: 402 (M+H)⁺.

### Example 11: 3-[(4-(5-Formyl-2-methoxyphenyl)-1,3,5-triazin-2-yl)amino]-benzene-methanesulfonamide (B11)

**B11** was prepared following the general procedure reported for **B1** using **A1** and 5-formyl-2-methoxyphenylboronic acid. MS (ES) C₁₈H₁₇N₅O₄S requires: 399, found: 400 (M+H)⁺.

### Example 12: 3-[(4-(2-Ethoxyphenyl)-1,3,5-triazin-2-yl)amino]benzene-methanesulfonamide (B12)

**B12** was prepared following the general procedure reported for **B1** using **A1** and 2-ethoxyphenylboronic acid. MS (ES) C₁₈H₁₉N₅O₃S requires: 385, found: 386 (M+H)⁺.

### Example 13: 3-[(4-(2-Benzyloxyphenyl)-1,3,5-triazin-2-yl)amino]benzene-methanesulfonamide (B13)

**B13** was prepared following the general procedure reported for **B1** using **A1** and 2-benzyloxyphenylboronic acid. MS (ES) C₂₃H₂₁N₅O₃S requires: 447, found: 448 (M+H)⁺.

### Example 14: 1-(3-{[4-(2-phenoxyphenyl)-1,3,5-triazin-2-yl]amino}phenyl)methane-sulfonamide (B14)

**B14** was prepared following the general procedure reported for **B1** using **A1** and 2-phenoxyphenylboronic acid.
¹H NMR (400MHz, d₆-DMSO, 300K) δ 4.17 (s, 2H), 6.83 (m, 2H), 6.91 (m, 2H), 7.03 (m, 4H), 7.29 (m, 3H), 7.52 (m, 2H), 7.93 (br, 2H), 8.72 (s, 1 H), 10.31 (s, 1 H).

### Example 15: 3-[(4-(1,3-Benzodioxol-4-yl)-1,3,5-triazin-2-yl)amino]benzene-methanesulfonamide (B15)

**B15** was prepared following the general procedure reported for **B1** using **A1** and (1,3-benzodioxol-4-yl)boronic acid. MS (ES) C₁₇H₁₅N₅O₄S requires: 385, found: 386 (M+H)⁺.

### Example 16: 3-[(4-(2-((4-Pyridinyl)methoxy)phenyl)-1,3,5-triazin-2-yl)amino]-benzenemethanesulfonamide (B16)

**B16** was prepared following the general procedure reported for **B1** using **A1** and **A9.** MS (ES) C₂₂H₂₀N₆O₃S requires: 448, found: 449 (M+H)⁺.

### Example 17: 3-[(4-(2-(4-(tert-Butoxycarbonylamino)butoxy)phenyl)-1,3,5-triazin-2-yl)amino]benzenemethanesulfonamide (B17)

**B17** was prepared following the general procedure reported for **B1** using **A1** and **A10.** MS (ES) C₂₅H₃₂N₆O₅S requires: 528, found: 529 (M+H)⁺.

### Example 18: 3-[(4-(4-Methoxypyridin-3-yl)-1,3,5-triazin-2-yl)amino]benzene-methanesulfonamide (B18)

**B18** was prepared following the general procedure reported for **B1** using **A1** and 4-methoxy-3-pyridinyl-boronic acid. MS (ES) C₁₆H₁₆N₆OO₃S requires: 372, found: 373 (M+H)⁺.

### Example 19: 3-[(4-(3-Methoxypyridin-4-yl)-1,3,5-triazin-2-yl)amino]benzene-methanesulfonamide (B19)

**B19** was prepared following the general procedure reported for **B1** using **A1** and 3-methoxy-4-pyridinyl-boronic acid. MS (ES) C₁₆H₁₆N₆O₃S requires: 372, found: 373 (M+H)⁺.

### Example 20: 3-[(4-(2-((Morpholin-4-yl)methyl)phenyl)-1,3,5-triazin-2-yl)amino]-benzenemethanesulfonamide (B20)

**B20** was prepared following the general procedure reported for **B1** using **A1** and (2-((morpholine-4-yl)methyl)phenyl)boronic acid. MS (ES) C₂₁H₂₄N₆O₃S requires: 440, found: 441 (M+H)⁺.

### Example 21: 3-[(4-(2-((Piperidin-1-yl)methyl)phenyl)-1,3,5-triazin-2-yl)amino]-benzenemethanesulfonamide (B21)

**B21** was prepared following the general procedure reported for **B1** using **A1** and (2-((piperidine-1-yl)methyl)phenyl)boronic acid. MS (ES) C₂₂H₂₆N₆O₂S requires: 438, found: 439 (M+H)⁺.

### Example 22: 3-[(4-(2-(Cyclopropylamino-methyl)phenyl)-1,3,5-triazin-2-yl)-amino]-benzenemethanesulfonamide (B22)

**B22** was prepared following the general procedure reported for **B1** using **A1** and (2-(cyclopropylamino-methyl)phenyl)boronic acid. MS (ES) C₂₀H₂₂N₆O₂S requires: 410, found: 411 (M+H)⁺.

### Example 23: 3-[(4-(6-Aminopyridin-3-yl)-1,3,5-triazin-2-yl)amino]benzene-methanesulfonamide (B23)

**B23** was prepared following the general procedure reported for **B1** using **A1** and 5-(4,4,5,5-tetramethyl-1,3,2-dioxaborolan-2-yl)pyridin-2-amine. MS (ES) C₂₀H₂₂N₆O₂S requires: 357, found: 358 (M+H)⁺.

### Example 24: 3-[(4-(2-(Methoxymethyl)phenyl)-1,3,5-triazin-2-yl)amino]benzene-methanesulfonamide (B24)

**B24** was prepared following the general procedure reported for **B1** using **A1** (400 mg, 1.33 mmol) and (2-(methoxymethyl)phenyl)boronic acid (221 mg, 1.33 mmol); yield: 6 mg, 1.2%). MS (ES) C₁₈H₁₉N₅OS requires: 385, found: 386 (M+H)⁺.

### Example 25: 3-[(4-(2-Methoxyphenyl)-1,3,5-triazin-2-yl)amino]benzenesulfonamide (C1)

A mixture of **A2** (1.0 eq), 2-methoxyphenylboronic acid (1.5 eq), and K₃PO₄ (2.0 eq) in dioxane-water (50:1, 0.1M) was degassed with a stream of N₂ for 15min. Pd(dppf)Cl₂ (0.1 eq) was added and the reaction mixture was heated to 140°C for 1 h in the microwave oven. The mixture was concentrated under reduced pressure and the residue was purified by reverse phase RP-HPLC (column: C18), using H₂O (0.1%TFA) and MeOH (0.1%TFA) as eluents. The desired fractions were lyophilized to yield the title compound **C1** (45%) as a white powder. ¹H NMR (400MHz, d₆-DMSO, 300K) δ 3.86 (s, 3H), 7.08 (t, J = 7.4 Hz, 1 H), 7.21 (d, J = 8.2 Hz, 1 H), 7.37 (s, 2H), 7.55 (m, 4H), 7.80 (m, 1H), 8.28 (s, 1 H), 8.88 (s, 1H), 10.70 (s, 1 H). MS (ES) C₁₆H₁₅N₅O₃S requires: 357, found: 358 (M+H)⁺.

### Example 26: 2-[3-((4-(2-Methoxyphenyl)-1,3,5-triazin-2-yl)amino)phenyl]-ethane-sulfonamide (D1)

A mixture of **A5** (1.0 eq, 65% purity), 2-methoxyphenylboronic acid (1.5 eq), and K₃PO₄ (2.0 eq) in dioxane-water (50:1, 0.1M) was degassed with a stream of N₂ for 15min. Pd(dppf)Cl₂ (0.1 eq) was added and the reaction mixture was heated to 140°C for 1 h in the microwave oven. The mixture was concentrated under reduced pressure and the residue was purified by reverse phase RP-HPLC (column: C18), using H₂O(0.1%TFA) and MeOH (0.1%TFA) as eluents. The desired fractions were lyophilized to yield the title compound **D1** (6%) as a white powder. ¹H NMR (400MHz, d₆-DMSO, 300K) δ 3.02 (m, 2H), 3.26 (m, 2H), 3.90 (s, 3H), 6.90 (br. s, 2H), 7.04 (d, J = 7.5 Hz, 1H), 7.12 (t, J = 7.5 Hz, 1 H), 7.22 (d, J = 8.2 Hz, 1 H), 7.32 (t, J = 7.9 Hz, 1 H), 7.58 (t, J = 7.9 Hz, 1 H), 7.68 (m, 2H), 7.85 (br.s, 1 H), 8.86 (s, 1 H), 10.70 (s, 1H) . MS (ES) C₁₈H₁₉N₅O₃S requires: 385, found: 386 (M+H)⁺.

### Example 27: 2-[3-((4-(4-Fluoro-2-methoxyphenyl)-1,3,5-triazin-2-yl)amino)-phenyl]ethanesulfonamide (D2)

**D2** was prepared following the general procedure reported for **D1** using **A5** and 4-fluoro-2-methoxyphenylboronic acid. MS (ES) C₁₈H₁₈FN₅O₃S requires: 403, found: 404 (M+H)⁺.

### Example 28: 3-[(4-(2-Methoxyphenyl)-1,3,5-triazin-2-yl)amino]benzamide (E1)

**E1** was prepared following the general procedure reported for **B1** using **A6** and 2-methoxyphenylboronic acid. After reverse phase RP-HPLC (column: C18), using H₂O and MeOH as eluents, the desired fractions were lyophilized to yield the title compound **E1** (35%) as a white powder. ¹H NMR (400MHz, d₆-DMSO, 300K) δ 3.85 (s, 3H) 6.91 (t, J = 7.3 Hz, 1 H), 6.95 (d, J = 8.3 Hz, 1 H), 7.07 (t, J = 7.3 Hz, 1 H), 7.19 (d, J =8.3 Hz, 1H), 7.36 (m, 1H), 7.42 (t, J = 8.3 Hz, 1H), 7.55 (m, 2H), 7.91 (br. s, 1H), 8.19 (s, 1 H), 8.84 (s, 1 H), 10.61 (s, 1 H). MS (ES) C₁₇H₁₅N₅O₂ requires: 321, found: 322 (M+H)⁺.

### Example 29: 6-[(4-(2-Methoxyphenyl)-1,3,5-triazin-2-yl)amino]-2,3-dihydro-1H-indole-1-sulfonamide (F1)

F1 was prepared following the general procedure reported for **B1** using **A8** and 2-methoxyphenylboronic acid. MS (ES) C₁₈H₁₈N₆O₃S requires: 398, found: 399 (M+H)⁺.

### Example 30: rac-S-[3-((4-(2-Methoxyphenyl)-1,3,5-triazin-2-yl)amino)phenyl]-N-ethoxycarbonyl-S-methyl-sulfoximide (G1)

The title compound **G1** was prepared from **A7** and 2-methoxyphenylboronic acid following the general procedure reported for **B1.** MS (ES) C₂₀H₂₁N₅O₃S requires: 427, found: 428 (M+H)⁺.

### Example 31: 4-(2-Methoxyphenyl)-N-(3-nitrophenyl)-1,3,5-triazine-2-amine (H1)

H1 was prepared following the general procedure reported for **B1** using **A3** and 2-methoxyphenylboronic acid. ¹H NMR (400MHz, d₆-DMSO, 300K) δ 3.84 (s, 3H), 7.08 (d, J = 7.5 Hz, 1H), 7.21 (d, J = 8.3 Hz, 1H), 7.53 (t, J = 8.7 Hz, 1H), 7.63 (t, J = 8.3 Hz, 1 H), 7.79 (br. s, 1 H), 7.90 (d, J = 7.5 Hz, 1 H), 8.24 (br.s, 1 H), 8.88 (s, 1 H), 8.91 (s, 1 H), 10.75 (s, 1 H). MS (ES) C₁₆H₁₃N₅O₃ requires: 323, found: 324 (M+H)⁺.

### Example 32: 3-[(4-(2-(4-Aminobutoxy)phenyl)-1,3,5-triazin-2-yl)amino]-benzene-methanesulfonamide (11)

The triazine **B17** (25 mg, 0.047 mmol) was dissolved in a mixture of TFA (2 ml) and DCM (2 ml). The deprotection was complete after stirring for 1 hour at RT as determined by LC/MS. The solvent was removed under reduced pressure, and the residue purified by reverse phase RP-HPLC (column: C18), using H₂O (0.1%TFA) and MeOH (0.1%TFA) as eluents. The desired fractions were lyophilized to yield the title compound **I1** as a yellow solid. MS (ES) C₂₀H₂₄N₆O₃S requires: 428, found: 429 (M+H)⁺.

### Example 33: N-(3-Aminophenyl)-4-(2-methoxyphenyl)-1,3,5-triazine-2-amine (J1)

A mixture of **H1** (1.0 eq) and Pd/C (10% w/w) in MeOH (0.1 M) was stirred for 16h at RT under H₂ atmosphere (1 atm). The mixture was filtered through Celite^{®} and the solvent was evaporated under reduced pressure. The crude product was purified by flash chromatography on silica gel (cHex/EtOAc = 100:0 to 0:100) to yield the desired product **J1** (18%) as a yellow solid. MS (ES) C₁₆H₁₅N₅O requires: 293, found: 294 (M+H)⁺.

### Example 34: N-[3-((4-(2-Methoxyphenyl)-1,3,5-triazin-2-yl)amino)phenyl]-methane-sulfonamide (K1)

To a mixture of **J1** (1.0 eq) and dry pyridine (3.0 eq) in DCM (0.1 M) at 0°C was slowly added methanesulfonyl chloride (2.0 eq). The mixture was stirred for 16h, quenched by addition of MeOH, and concentrated under reduced pressure. The residue was purified by reverse phase RP-HPLC (column: C18), using H₂O (0.1%TFA) and MeOH (0.1%TFA) as eluents. The desired fractions were lyophilized to yield the title compound **K1** (44%) as a white powder. ¹H NMR (400MHz, d₆-DMSO, 300K) δ 2.96 (s, 3H), 3.83 (s, 3H), 6.90 (d, J = 8.3 Hz, 1 H), 7.06 (t, J = 7.3 Hz, 1 H), 7.17 (d, J = 8.1 Hz, 1 H), 7.28 (t, J =8.1 Hz, 1 H), 7.51 (t, J = 8.3 Hz, 1 H), 7.63 (m, 2H), 7.76 (br. s, 1 H), 8.80 (s, 1 H), 9.75 (s, 1 H), 10.50 (s, 1 H). MS (ES) C₁₇H₁₇N₅O₃S requires: 371, found: 372 (M+H)⁺.

### Example 35: N-[3-((4-(2-Methoxyphenyl)-1,3,5-triazin-2-yl)amino)phenyl]-propanesulfonamide (L1)

**L1** was prepared following the general procedure reported for **K1** using **J1** and propane-1-sulfonyl chloride. MS (ES) C₁₉H₂₁N₅O₃S requires: 399, found: 400 (M+H)⁺.

### Example 36: N-[3-((4-(2-Methoxyphenyl)-1,3,5-triazin-2-yl)amino)phenyl]-acetamide (M1)

**M1** was prepared following the general procedure reported for **K1** using **J1** and acetyl chloride. ¹H NMR (400MHz, d₆-DMSO, 300K) δ 2.02 (s, 3H), 3.83 (s, 3H), 7.06 (t, J = 7.8 Hz, 1H), 7.18 (d, J = 8.9 Hz, 1H), 7.24 (t, J =8.1 Hz, 1 H), 7.29 (m, 1H), 7.52 (t, J = 7.8 Hz, 1H), 7.91 (s, 1H), 8.80 (s, 1H), 9.92 (s, 1 H), 10.40 (s, 1 H). MS (ES) C₁₈H₁₇N₅O₂ requires: 335, found: 336 (M+H)⁺.

### Example 37: N-[3-((4-(2-Methoxyphenyl)-1,3,5-triazin-2-yl)amino)phenyl]-N'-phenyl-urea (N1)

A mixture of **J1** (1.0 eq), phenyl isocyanate (2.0 eq), and pyridine (3.0 eq) in dry DCM (0.01 M) was stirred for 12 h at RT. The mixture was concentrated under reduced pressure and dissolved with a minimum of DMSO. Water was added and the precipitate was filtered off. The white solid was washed with water and dried in vacuo yielding the desired product **N1** (62%), ¹H NMR (400MHz, d₆-DMSO, 300K) δ 3.83 (s, 3H), 6.97 (t, J = 7.0 Hz, 1 H), 7.06 (t, J = 7.4 Hz, 1 H), 7.18 (d, J = 7.8 Hz, 1 H), 7.28 (m, 4H), 7.46 (m, 3H), 7.51 (m, 2H), 7.80 (s, 1H), 8.60 (m, 2H), 8.80 (s, 1H), 10.26 (s, 1H).MS (ES) C₂₃H₂₀N₆O₂ requires: 412, found: 413 (M+H)⁺.

### Example 38: 3-[(4-(2-Methoxy-5-(methylamino-methyl)phenyl)-1,3,5-triazin-2-yl)-amino]benzenemethanesulfonamide (O1)

Sodium borohydride (1.5 eq) was added to a mixture of **B11** and methylamine-solution (MeOH, 2M, 2.0 eq.) in MeOH (0.1M) at 0°C. After 2 h the reaction mixture was stirred for another 12 h at RT. The solution was diluted with EtOAc, and the organic layer was successively washed with sat. aq. NaHCO₃ solution and with brine. After drying over Na₂SO₄ the solvent was removed in vacuo. The residue was purified by reverse phase RP-HPLC (column: C18), using H₂O (0.1%TFA) and MeOH (0.1%TFA) as eluents. The desired fractions were lyophilized to yield the title compound **01** (3%) as a white powder. MS (ES) C₁₉H₂₂N₆O₃S requires: 414, found: 415 (M+H)⁺.

### Example 39: 4-(2-Methoxyphenyl)-N-phenyl-1,3,5-triazine-2-amine (P1)

A mixture of 2-methoxybenzamide (1.0 eq) and N,N-dimethylformamide dimethyl acetal (1.4 eq) was heated at 80°C for 1 h. The excess of reagent was removed under reduced pressure. The crude product was dissolved in 1,4-dioxane (0.2M) followed by addition of phenylguanidine carbonate (0.43 eq) and potassium tert-butoxide (0.41 eq). The reaction mixture was stirred under reflux and N₂ atmosphere for 12 h. After removal of the solvent, the residue was purified by reverse phase RP-HPLC (column: C18), using H₂O (0.1%TFA) and MeOH (0.1%TFA) as eluents. The desired fractions were lyophilized to yield the title compound P1 (20%) as a white powder. ¹H NMR (400MHz, d₆-DMSO, 300K) δ 3.87 (s, 3H), 7.08 (t, J = 7.2 Hz, 2H), 7.20 (d, J = 8.3 Hz, 1 H), 7.35 (t, J = 7.8 Hz, 2H), 7.54 (t, J = 7.8 Hz, 1 H), 7.81 (m, 3H), 8.81 (s, 1 H), 10.50 (s, 1 H). MS (ES) C₁₆H₁₄N₄O requires: 278, found: 279 (M+H)⁺.

### Example 40: tert-Butyl [4-((3-((4-(4-Fluoro-2-methoxyphenyl)-1,3,5-triazin-2-yl)-amino)phenyl)methylsulfonamido)butyl]carbamate (Q1)

To a solution of the crude intermediate **A12** (3.62 g, 7.7 mmol) in dioxane / water 50:1 (75 mL) were added 4-fluoro-2-methoxybenzeneboronic acid (1.96 g, 11.5 mmol) and K₃PO₄ (3.26 g, 15.4 mmol). A stream of nitrogen was passed through the mixture for 15 minutes followed by addition of Pd(dppf)Cl₂ (0.63 g, 0.77 mmol). After another 15 minutes of nitrogen the mixture was stirred overnight at 140°C, cooled to RT, and poured into ice-cold water. It was extracted with EtOAc and the organic layer was dried over MgSO₄. The solvent was removed under reduced pressure and the title compound **Q1** obtained as an amorphous solid (1.1 g, 25%) after column chromatography (silica gel, CHCl₃ / MeOH gradient 100:0 to 90:10). ¹H NMR (300MHz, CDCl₃, 300K) δ 1.10-1.23 (m, 2H), 1.35 (s, 9H), 1.40-1.48 (m, 2H), 1.53 (s, 1H), 2.90-3.11 (m, 4H), 3.85 (s, 3H), 4.20 (s, 2H), 4.48 (bs, 1H), 6.61-6.72 (m, 2H), 6.92 (bs, 1 H), 7.10-7.21 (m, 1H), 7.58 (s, 1H), 7.70-7.97 (m, 3H), 8.56 (bs, 1H). MS (ES) C₂₆H₃₃FN₆O₅S requires: 560, found: 561 (M+H)⁺, 461 (M+H-COOC(CH₃)₃)⁺.

### Example 41: N-(4-Aminobutyl)-1-[3-((4-(4-fluoro-2-methoxyphenyl)-1,3,5-triazin-2-yl)amino)phenyl]methanesulfonamide (R1)

A solution of **Q1** (1.10 g, 1.96 mmol) in DCM (20 mL) was cooled with ice while TFA (2.24 g, 19.6 mmol) was added dropwise during 15 minutes. After stirring at RT for 2 hours the solvent was removed under reduced pressure. The residue was dissolved in EtOAc and the organic solution washed with sat. aq. NaHCO₃, dried over MgSO₄, and concentrated *in vacuo.* The title compound **R1** was obtained as a white powder (66 mg, 7%) after purification by column chromatography (silica gel, CHCl₃ / MeOH gradient 9:1 to 5:1 containing 1% triethylamine). ¹H NMR (300MHz, MeOD, 300K) δ 1.28-1.50 (m, 2H), 1.50-1.68 (m, 2H), 2.71-2.82 (m, 2H), 2.82-2.97 (m, 2H), 3.80 (s, 3H), 4.21 (s, 2H), 6.66-6.78 (m, 1H), 6.81-6.92 (m, 1H), 7.02-7.12 (m, 1H), 7.20-7.36 (m, 1H), 7.60-7.90 (m, 3H), 8.58 (s, 1H). MS (ES) C₂₁H₂₅FN₆O₃S requires: 460, found: 461 (M+H)⁺.

### Example 42: 4-(2-Methoxyphenyl)-N-(3-(methylsulfonyl)phenyl)-1,3,5-triazin-2-amine (S1)

**S1** was prepared from crude **A13** (370 mg, 1.3 mmol) and 2-methoxybenzeneboronic acid (303 mg, 2 mmol) following the procedure described for **Q1.** The title compound **S1** was purified by thick-layer chromatography (silica gel, chloroform / MeOH 9:1) to give a batch of 54 mg which was further purified by reverse phase RP-HPLC (column: C18; H₂O / MeOH gradient containing 0.1%TFA); yield: 1.8 mg, white amorphous solid. ¹H NMR (400MHz, CDCl₃, 300K) δ 3.08 (s, 3H), 3.94 (s, 3H), 7.04-7.11 (m, 2H), 7.50 (dt, J = 7.4Hz, J = 1.8 Hz, 1 H), 7.56 (t, J = 8.0Hz, 1 H), 7.61 (bs, 1 H), 7.68 (bd, J = 7.8Hz, 1 H), 7.90-8.01 (bm, 2H), 8.41 (s, 1H), 8.89 (s, 1H). MS (ES) C₁₇H₁₆N₄O₃S requires: 356, found: 357 (M+H)⁺.

### Example 43: 4-[(4-(2-Methoxyphenyl)-1,3,5-triazin-2-yl)amino]benzenemethane-sulfonamide (T1)

**T1** was prepared from crude **A14** (390 mg, 1.3 mmol) and 2-methoxybenzeneboronic acid (303 mg, 2 mmol) following the procedure described for **Q1.** The title compound **T1** was purified by thick-layer chromatography (silica gel, chloroform / MeOH 9:1) and obtained as a white amorphous solid; yield: 20 mg (4%). ¹H NMR (300MHz, d₆-DMSO, 300K) δ 3.87 (bs, 3H), 4.21 (s, 2H), 6.82 (s, 2H), 7.07 (t, J = 7Hz, 1 H), 7.13-7.25 (m, 1H), 7.34 (d, J = 9Hz, 2H), 7.47-7.59 (m, 1H), 7.70-7.97 (bm, 3H), 8.82 (s, 1H), 10.38 (s, 1 H). MS (ES) C₁₇H₁₇N₅O₃S requires: 371, found: 372 (M+H)⁺.

The following Examples 44 - 53 were prepared by essentially the same method as described for **B1.**

### Example 44: 1-[3-((4-[4-fluoro-2-(trifluoromethyl)phenyl]-1,3,5-triazin-2-yl)amino)-phenyl]methanesulfonamide (U1)

**U1** was prepared following the general procedure reported for **B1** using **A1** and [4-fluoro-2-(trifluoromethyl)phenyl]boronic acid.
¹H NMR (400MHz, d₆-DMSO, 300K) δ 4.23 (s, 2H), 6.85 (s, 2H), 7.10 (m, 1 H), 7.34 (m, 1 H), 7.70 (m, 3H), 7.81 (m, 1 H), 7.91 (m, 1 H), 8.85 (s, 1 H), 10.52 (s, 1 H).

### Example 45: 1-[3-({4-[4-fluoro-2-(propan-2-yloxy)phenyl]-1,3,5-triazin-2-yl}amino)-phenyl]methanesulfonamide (U2)

U2 was prepared following the general procedure reported for **B1** using **A1** and [4-fluoro-2-(propan-2-yloxy)phenyl]boronic acid.
¹H NMR (400MHz, d₆-DMSO, 300K) δ 1.22 (d, 6H), 4.19 (s, 2H), 4.65 (m, 1 H), 6.83 (m, 3H), 7.04 (m, 2H), 7.29 (m, 1 H), 7.70 (br, 3H), 8.76 (s, 1 H), 10.29 (s, 1 H).

### Example 46: 1-(3-{[4-(2-cyano-4-fluorophenyl)-1,3,5-triazin-2-yl]amino}phenyl)-methanesulfonamide (U3)

U3 was prepared following the general procedure reported for **B1** using **A1** and (2-cyano-4-fluorophenyl)boronic acid.
¹H NMR (400MHz, d₆-DMSO, 300K) δ 4.25 (s, 2H), 6.85 (s, 2H), 7.10 (m, 1 H), 7.34 (m, 1 H), 7.73 (br, 3H), 8.01 (m, 1 H), 8.40 (br, 1 H), 8.88 (s, 1 H), 10.54 (s, 1 H).

### Example 47: N-[5-fluoro-2-(4-{[3-(sulfamoylmethyl)phenyl]amino}-1,3,5-triazin-2-yl)phenyl]acetamide (U4)

**U4** was prepared following the general procedure reported for **B1** using **A1** and [2-(acetylamino)-4-fluorophenyl]boronic acid.
¹H NMR (400MHz, d₆-DMSO, 300K) δ 2.10 (br, 3H), 4.26 (s, 2H), 6.88 (s, 2H), 7.05 (m, 1 H), 7.15 (m, 1 H), 7.39 (m, 1 H), 7.70 (br, 2H), 8.39 (m, 1 H), 8.56 (m, 1 H), 8.88 (s, 1 H), 10.52 (s, 1 H), 12.40 (br, 1 H).

### Example 48: 1-[3-({4-[2-(cyclopropylmethoxy)-4-fluorophenyl]-1,3,5-triazin-2-yl}amino)phenyl]methanesulfonamide (U5)

**U5** was prepared following the general procedure reported for **B1** using **A1** and 2-[2-(cyclopropylmethoxy)-4-fluorophenyl]-4,4,5,5-tetramethyl-1,3,2-dioxaborolane.
¹H NMR (400MHz, d₆-DMSO, 300K) δ 0.29 (m, 2H), 0.46 (m, 2H), 1.15 (m, 1H), 3.95 (m, 2H), 4.22 (s, 2H), 6.84 (m, 3H), 7.06 (m, 2H), 7.33 (m, 1 H), 7.74 (br, 3H), 8.79 (s, 1H), 10.31 (s, 1H).

### Example 49: 1-(3-{[4-(3,4-difluoro-2-methoxyphenyl)-1,3,5-triazin-2-yl]amino}-phenyl)methanesulfonamide (U6)

**U6** was prepared following the general procedure reported for **B1** using **A1** and (3,4-difluoro-2-methoxyphenyl)boronic acid.
¹H NMR (400MHz, d₆-DMSO, 300K) δ 3.91 (br, 3H), 4.24 (s, 2H), 6.85 (s, 2H), 7.07 (m, 1 H), 7.35 (m, 2H), 7.73 (m, 3H), 8.84 (s, 1 H), 10.39 (s, 1 H).

### Example 50: 1-(3-{[4-(4,5-difluoro-2-methoxyphenyl)-1,3,5-triazin-2-yl]amino}-phenyl)methanesulfonamide (U7)

**U7** was prepared following the general procedure reported for **B1** using **A1** and (4,5-difluoro-2-methoxyphenyl)boronic acid.
¹H NMR (400MHz, d₆-DMSO, 300K) δ 3.87 (s, 3H), 4.23 (s, 2H), 6.84 (s, 2H), 7.08 (m, 1 H), 7.36 (m, 2H), 7.80 (br, 3H), 8.80 (s, 1 H), 10.39 (s, 1 H).

### Example 51: 4-(4-fluoro-2-methoxyphenyl)-N-[6-(methylsulfonyl)pyridin-3-yl]-1,3,5-triazin-2-amine (U8)

DIPEA (0.23 ml; 1.33 mmol) was added to a solution of 2,4-dichloro-1,3,5-triazine in THF/iPrOH (1.30 ml; 1/1) at -40°C. A suspension of 5-amino-2-methylsulphonylpyridine (115 mg; 0.67 mmol; prepared according to H.S. Forrest and J. Walker, J. Chem. Soc., 1948, 1939-1945) in THF/iPrOH (0.7 ml; 1/1) was added. The reaction mixture was stirred at -40°C for 3.5 hours before it was stirred at 0°C for 3.5 hours. The solvents were carefully removed using a rotovab while the temperature of the water bath remained below 30°C, to give crude 4-chloro-N-[6-(methylsulfonyl)pyridin-3-yl]-1,3,5-triazin-2-amine (420 mg) that was used without further purification.

A mixture of 86 mg of the crude product, (4-fluoro-2-methoxyphenyl)boronic acid (76 mg; 0.45 mmol), and K₃PO₄ (127 mg; 0.60 mmol) in dioxane/water (3 ml; 20/1) was degassed with a stream of N₂ for 15min. Pd(dppf)Cl₂ (123 mg; 0.15 mmol) was added and the reaction mixture heated to 140°C for 1 h in the microwave oven. The mixture was concentrated under reduced pressure and the residue was purified by reverse phase RP-HPLC (column: C18), using H₂O (0.1% HCOOH) and acetonitrile as eluents. The desired fractions were lyophilized to afford the title compound as a white solid.
¹H NMR (400MHz, d₆-DMSO, 300K) δ 3.21 (s, 3H), 3.91 (s, 3H), 6.90 (m, 1 H), 7.11 (m, 1 H), 8.03 (m, 2H), 8.57 (m, 1 H), 8.91 (s, 1 H), 9.17 (br, 1 H), 10.94 (s, 1 H).

### Example 52: 1-(3-{[4-(4-fluoro-2-methoxyphenyl)-1,3,5-triazin-2-yl]amino}phenyl)-methanesulfonamide hydrochloride (B2')

1-(3-{[4-(4-fluoro-2-methoxyphenyl)-1,3,5-triazin-2-yl]amino}phenyl)methanesulfon-amide (914 mg; 2.35 mmol) was suspended in 1 N aqueous hydrogen chloride solution (2.35 ml) and stirred for 4 hours at room temperature. The mixture was concentrated under reduced pressure to give the desired product (980 mg; 2.30 mmol).
¹H NMR (400MHz, d₆-DMSO, 300K) δ 3.90 (s, 3H), 4.23 (s, 2H), 6.91 (m, 3H), 7.13 (m, 2H), 7.37 (m, 1 H), 7.81 (m, 3H), 8.82 (s, 1 H), 10.83 (s, 1 H).

### Example 53: 3-[(4-(2-Methoxyphenyl)-1,3,5-triazin-2-yl)amino]benzenemethane-sulfonamide trifluoroacetate (B1')

A mixture of 3-[(4-Chloro-1,3,5-triazin-2-yl)amino]benzenemethanesulfon-amide (1.0 eq), 2-methoxybenzeneboronic acid (1.5 eq) and K₃PO₄ (2.0 eq) in dioxane/water (50/1, 0.1 M) was degassed with a stream of N₂ for 15min. Pd(dppf)Cl₂ (0.1 eq) was added and the reaction mixture was heated to 140°C for 1 h in the microwave. The mixture was concentrated under reduced pressure and the residue was purified by reverse phase RP-HPLC (column: C18), using H₂0 (0.1%TFA) and MeOH (0.1%TFA) as eluents, the desired fractions were lyophilized to afford the titled compound **(B1')** (2%) as a white powder. ¹H NMR (400MHz, DMSO, 300K) δ 3.87 (s, 3H), 4.25 (s, 2H), 6.87 (s, 2H), 7.10 (m, 2H), 7.21 (d, J = 8.4 Hz, 1H), 7.37 (t, J = 8.0 Hz, 1H), 7.55 (m, 2H), 7.84 (br. s, 1H), 8.82 (s, 1H), 10.52 (s, 1H). MS (ES) C₁₇H₁₇N₅O₃S requires: 371, found: 372 (M+H)⁺.

### Example 54: 3-[(4-(2-Benzyloxyphenyl)-1,3,5-triazin-2-yl)amino]benzenemethanesulfonamide trifluoroacetate (B13')

**B13'** was prepared following the general procedure reported for **B1** using **A1** and 2-benzyloxyphenylboronic acid as described in example 53.
¹H NMR (400MHz, d₆-DMSO, 300K) δ 4.22 (s, 2H), 5.24 (s, 2H), 6.85 (br. s, 2H), 7.08 (m, 2H), 7.19-7.34 (m, 5H), 7.41-7.61 (m, 3H), 7.69 (s, 1H), 7.80 (m, 2H), 8.86 (s, 1H), 10.53 (s, 1 H). MS (ES) C₂₃H₂₁N₅O₃S requires: 447, found: 448 (M+H)⁺.

### Example 55: 3-[(4-(2-Methoxyphenyl)-1,3,5-triazin-2-yl)amino]benzenesulfonamide trifluoroacetate (C1')

A mixture **of A2** (1.0 eq), 2-methoxybenzeneboronic acid (1.5 eq) and K₃PO₄ (2.0 eq) in dioxane-water (50:1, 0.1 M) was degassed with a stream of N₂ for 15min. Pd(dppf) (0.1 eq) was added and the reaction mixture was heated to 140°C for 1 h in the microwave oven. The mixture was concentrated under reduced pressure and the residue was purified by reverse phase RP-HPLC (column: C18), using H₂0 (0.1%TFA) and MeOH (0.1%TFA) as eluents, the desired fractions were lyophilized to afford the titled compound **C1'** (45%) as a white powder.
¹H NMR (400MHz, d₆-DMSO, 300K) δ 3.86 (s, 3H), 7.08 (t, J = 7.4 Hz, 1 H), 7.21 (d, J = 8.2 Hz, 1 H), 7.37 (s, 2H), 7.55 (m, 4H), 7.80 (m, 1 H), 8.28 (s, 1 H), 8.88 (s, 1 H), 10.70 (s, 1 H). MS (ES) C₁₆H₁₅N₅O₃S requires: 357, found: 358 (M+H)⁺.

### Example 56: 3-[(4-(4-Fluoro-2-methoxyphenyl)-1,3,5-triazin-2-yl)amino]benzenemethanesulfonamide trifluoroacetic acid salt (B2")

A mixture of **A2** (1.0 eq), 4-fluoro-2-methoxybenzeneboronic acid (1.5 eq) and K₃PO₄ (2.0 eq) in dioxane-water (50:1, 0.1M) was degassed with a stream of N₂ for 15min. Pd(dppf) (0.1 eq) was added and the reaction mixture was heated to 140°C for 1 h in the microwave oven. The mixture was concentrated under reduced pressure and the residue was purified by reverse phase RP-HPLC (column: C18), using H₂0 (0.1%TFA) and MeOH (0.1%TFA) as eluents, the desired fractions were lyophilized to afford the titled compound **B2"** (23%) as a white powder. ¹H NMR (400MHz, d₆-DMSO, 300K) δ 3.89 (s, 3H), 4.24 (s, 2H), 6.90 (m, 3H), 7.11 (m, 2H), 7.36 (t, J = 7.9Hz, 1 H), 7.72 (m, 1H), 7.90 (m, 2H), 8.81 (s, 1H), 10.63 (s, 1H). MS (ES) C₁₇H₁₆FN₅O₃S requires: 389, found: 390 (M+H)⁺.

### Materials and Methods:

### 1. Measurement of Binding Affinities to CDKs

This protocol describes how the LanthaScreen Eu Kinase Binding Assay was performed to determine dissociation constants (K_{d}) of compounds of general formula (I) and CDK/Cyclin complexes. The principle behind this assay is based upon the binding and displacement of an Alexa Fluor 647-labeled tracer, which binds to the active site of kinases. Binding of the tracer to the kinase is detected using a Eu-labeled antibody. Simultaneous binding of both the tracer and antibody to the kinase gives rise to a FRET-signal. Binding of an inhibitor to the kinase competes for binding with the tracer, resulting in a loss of FRET.

**Table 2: Reagents, stock concentrations and final assay concentrations**

| **Kinase** | **Supplier** | **Kinase-conc. [nM]** | **Tracer** | **Supplier** | **Tracer-conc. [nM]** | **Antibody** | **Supplier** | **Antibody-conc.** |
|---|---|---|---|---|---|---|---|---|
| CDK2/Cyclin A (135 kDa) | Proqinase | 5 | 236 | Invitrogen | 20 | Eu-Anti-GST | Cisbio | 1:750 |
| CDK7/CyclinH/Mat1 (126 kDa) | Carna Biosciences | 5 | 236 | Invitrogen | 60 | Eu-Anti-GST | Invitrogen | 2nM |
| CDK8/Cyclin C (97kDa) | Invitrogen | 5 | 236 | Invitrogen | 20 | Eu-Anti-GST | Invitrogen | 2nM |
| CDK9/Cyclin T1 (132 kDa) | Invitrogen | 5 | 236 | Invitrogen | 30 | Eu-Anti-GST | Cisbio | 1:250 |
| CDK9/Cyclin K (92 kDa) | Invitrogen | 10 | 236 | Invitrogen | 35 | Eu-Anti-GST | Invitrogen | 4nM |

The compounds of general formula (I) summarized in Table 1 were diluted from a 10 mM DMSO stock solution 1:10 in a total volume of 15 µL DMSO. This compound predilution was then serial diluted 1:3 over 8 steps in DMSO and briefly spun down. Each compound solution was now diluted 1:33.33 in kinase buffer (HEPES: 20 mM, pH: 8.0; MgCl₂: 10 mM; DTT: 1 mM; Brij-35: 0.01 %), mixed thoroughly and spun down. For every sample, 5 µL of the diluted compound were mixed with 5 µL tracer working solution (e.g. 60 nM tracer 236 in kinase buffer for CDK2/Cyclin A) and 5 µL CDK/Cyclin / Anti-GST-AB-working solution (e.g. 15 nM CDK2/Cyclin A, 1:250 dilution of Anti-GST-AB in kinase buffer) in a well of a small volume 384 well plate (Corning Incorporated, Corning, NY, USA; order no. 3673). The tracer concentration was adjusted to its dissociation constant (K_{d}) for the CDK/Cyclin, which was 30 nM for CDK2/Cyclin A, CDK7/Cyclin H and CDK9/Cyclin T1, 20 nM for CDK8/Cyclin C, and 35 nM for CDK9 / Cyclin K. For negative controls, in each well 5 µL of DMSO working solution (3% DMSO diluted in kinase buffer) was mixed with 5 µL Anti-GST-AB working solution (e.g. 1:250 dilution of Anti-GST-AB in kinase buffer for CDK2/Cyclin A) and 5 µL Tracer working solution (e.g. 60 nM Tracer 236 in kinase buffer for CDK2/Cyclin A). For positive controls, in each well 5 µL of DMSO working solution (3% DMSO diluted in kinase buffer) was mixed with 5 µL CDK/Cyclin/Anti-GST-AB-working solution: (e.g. 15 nM CDK2/Cyclin A, 1:250 dilution of Anti-GST-AB in kinase buffer) and 5 µL Tracer working solution (e.g. 60 nM Tracer 236 in kinase buffer for CDK2/Cyclin A). Positive and negative controls were calculated from at least 8 different sample wells. The 384 well plates were mixed in a Teleshaker plate mixer (Beckman Coulter, Brea, CA, USA) at 2000 rpm for 40 sec, and incubated for 1 h at room temperature before reading. The FRET signal was measured at 340 nm excitation, 665 nm and 615 nm emission (for the kinase tracer and LanthaScreen Eu-AB, respectively) with an Envision spectrophotometer (Perkin Elmer, Waltham, MA, USA) with 50 µs delay and 300 µs integration time. K_{d} values were determined from the sigmoidal dose response curves with the software Quattro Workflow (Quattro GmbH, Munich, Germany). Results are presented in Table 4.

### 2. Measurement of half maximal inhibitory concentration to CDKs

This protocol describes how the Lance Ultra KinaSelect Assay was performed to determine half maximal inhibitory concentration (IC₅₀) of compounds of general formula (I) and CDK/Cyclin complexes. The principle behind this enzymatic assay is based upon the phosphorylation of the U*Light*-Peptide Substrat. It is detected by using a specific EU-Iabeled anti-phospho peptide antibody. The binding of the Eu-labeled anti-phospho peptide antibody to the phosphorylated U*Light* labeled peptide gives rise to a FRET-signal. Binding of an inhibitor to the kinase prevents phosphorylation of the U*Light-*MBP Substrat, resulting in a loss of FRET.

**Table 3: Reagents, stock concentrations and final assay concentrations**

| **Kinase** | **Supplier** | **Kinase-conc. [nM]** | **ATP-conc. [µM]** | **Substrat** | **Supplier** | **Substrat-conc. [nM]** | **Antibody** | **Supplier** | **Antibody-conc. [nM]** |
|---|---|---|---|---|---|---|---|---|---|
| CDK1/CyclinB1 (91 kDa) | Carna | 2 | 20 | Ulight MBP | Perkin Elmer | 50 | Eu-anti-P-MBP | Perkin Elmer | 0,25 |
| CDK2/CyclinA (135 kDa) | Proqinase | 5 | 3 | Ulight MBP | Perkin Elmer | 50 | Eu-anti-P-MBP | Perkin Elmer | 0,25 |
| CDK4/CyclinD1 (123 kDa) | Invirtogen | 10 | 90 | Ulight MBP | Perkin Elmer | 50 | Eu-anti-P-MBP | Perkin Elmer | 0,25 |
| CDK6/CyclinD3 (123 kDa) | Carna | 5 | 55 | Ulight MBP | Perkin Elmer | 50 | Eu-anti-P-MBP | Perkin Elmer | 0,025 |
| CDK7/CyclinH/MAT1 (126 kDa) | Invitrogen | 10 | 25 | Ulight MBP | Perkin Elmer | 50 | Eu-anti-P-MBP | Perkin Elmer | 0,25 |
| CDK9/CyclinT1 (132 kDa) | Invitrogen | 10 | 25 | Ulight MBP | Perkin Elmer | 50 | Eu-anti-P-MBP | Perkin Elmer | 0,25 |
| CDK9/CyclinK (92 kDa) | Invitrogen | 10 | 125 | Ulight MBP | Perkin Elmer | 50 | Eu-anti-P-MBP | Perkin Elmer | 0,25 |

The compounds of general formula (I) summarized in Table 5 were diluted from a 10 mM DMSO stock solution 1:10 in a total volume of 15 µL DMSO. This compound predilution was then serial diluted 1:3 over 8 steps in DMSO and briefly spun down. Each compound solution was now diluted 1:20 in Enzymatic Buffer (HEPES: 50 mM, pH: 7.5; MgCl₂: 10 mM; EGTA: 1 mM; DTT: 2mM; Tween-20: 0.01%), mixed thoroughly and spun down. For every sample, 2 µL of the diluted compound were mixed with 6 µL CDK/Cyclin/Substrat solution and 2 µL ATP solution in a well of a small volume 384 well plate (Corning Incorporated, Corning, NY, USA; order no. 3673). The CDK/Cyclin was diluted to the appropriate concentration (see Table 3) and the ATP concentration was adjusted to its IC₅₀ concentration for the CDK/Cyclin, which was 3 µM for CDK2/Cyclin A, 20 µM for CDK1/Cyclin B1, 25 µM for CDK7/Cyclin H and CDK9/Cyclin T1, 55 µM for CDK6/Cyclin D3, 90 µM for DK4/Cyclin D1 and 125 µM for CDK9/Cyclin K. For negative controls, in each well 2 µL of DMSO solution (1% final DMSO assay concentration) was mixed with 6 µL substrate solution (50 nM U*Light* MBP final assay concentration) and 2 µL ATP solution (appropriate final concentration see Table 3). For positive controls, in each well 2 µL of DMSO solution (1% final DMSO assay concentration) was mixed with 6 µL CDK/Cyclin/Substrat (appropriate final concentration see Table 3) and 2 µL Tracer ATP solution (appropriate final concentration see Table 3). Positive and negative controls were calculated from at least 8 different sample wells. The 384 well plates were mixed in a Teleshaker plate mixer (Beckman Coulter, Brea, CA, USA) at 2000 rpm for 40 sec, and incubated for 1 h at room temperature. Before reading, 10 µL detection buffer (Lance Detection Buffer 1X; EDTA: 20nM; Eu-Anti-P-MBP: see Table 3) was added. The FRET signal was measured at 340 nm excitation, 665 nm and 615 nm emission (for the kinase tracer and LanthaScreen Eu-AB, respectively) with an Envision spectrophotometer (Perkin Elmer, Waltham, MA, USA) with 50 µs delay and 300 µs integration time. IC₅₀ values were determined from the sigmoidal dose response curves with the software Quattro Workflow (Quattro GmbH, Munich, Germany). Results are presented in Table 5.

### 3. Cellular Assays

### 3.1 RNA-Polymerase II Ser2 Cellular Phosphorylation Assay:

HCT-116 cells (DSMZ, Braunschweig, Germany) were maintained in Mc Coy's cell culture medium + glutamine (PAN Biotech GmbH, Aidenbach, Germany) supplemented with 10% fetal calf serum (PAA Laboratories GmbH, Pasching, Austria) and grown at 37°C, 5% CO₂, For the cellular phosphorylation assay, cells were seeded with 2x105 cells/well/1ml in 24-well plates (Greiner Bio - One, Frickenhausen, Germany; catalog # 662160). The compounds of general formula (I) summarized in Table 6 were diluted from a 10 mM DMSO stock solution 1:10 in a total volume of 15 µL DMSO. After overnight incubation at 37°C/5% CO₂, 1.5 µL of a compound diluted in DMSO was added to each sample well. Wells with cells and 0.15 % DMSO in culture medium were used as positive controls, wells without cells and 0.15 % DMSO in culture medium were used as negative controls. The cells were incubated with the compounds for 72h at 37°C/5% CO₂, Before lysis, cells were washed with phosphate buffered saline. Phosphorylation of RNA Polymerase II Ser2 and tubulin levels for normalization were analyzed afterwards with the Multi-Array technology (Meso Scale Discovery, Gaithersburg, Maryland, USA), a combination of antibody coupled electrochemiluminescence detection and patterned arrays. Manufacturer's instructions were followed and all solutions were purchased from Meso Scale Discovery. In brief, cells were lysed by 30 min incubation in CLB1 lysis buffer (Zeptosens, Witterswil, Switzerland; 60 µL per well), and supernatants were cleared by centrifugation. For analysis of RNA Polymerase II Ser2-phosphorylation, lysates were diluted 1:50 with Meso Scale Lysis Buffer supplemented with phosphatase- and protease-inhibitors, and 25 µL of each sample was pipetted in a well of a MSD Multi-Array 96-Well Plate Sector^{®} Imager High Bind Plate (Meso Scale Discovery; catalog # L15XB-3/L11XB-3), and incubated for 2h at room temperature. 150µL Meso Scale Tris Wash Buffer supplemented with 3% w/v Meso Scale Blocker A were added per well, then plates were sealed and incubated 1 h with vigorous shaking. Plates were washed with 1x Tris Wash Buffer (10x Meso Scale Wash Buffer diluted 1:10 in destilled water), 25 µL of antibody solution was added (CTD7 3E10 antibody from Helmholtz Zentrum Munich, Germany, diluted 1:100 in Meso Scale Tris Wash Buffer supplemented with 1% w/v Meso Scale Blocker A), and plates were washed three times in 1x Tris Wash Buffer. 25 µL of MSD^{®} SULFO-TAG™ Goat - Anti - Rat - Antibody (Meso Scale Discovery, catalog # R32AH-1, diluted 1:125 in Tris Wash Buffer with 1% (w/v) blocker A) were added per well, plates were sealed and incubated with vigorous shaking for 1 h at room temperature. Finally, plates were washed three times with Tris Wash Buffer, 150 µl 2x Read Buffer (Meso Scale Discovery) were added per well and plates were analyzed immediately in a Sector Imager from Meso Scale Discovery. For determination of tubulin protein levels, samples were analyzed with the protocol for RNA Polymerase II Ser2-phosphorylation, with an anti-tubulin antibody (rabbit; BIODESIGN International, catalog # T59840R, diluted 1:100) and a MSD^{®} SULFO-TAG™ Goat - Anti - Rat - Antibody (Meso Scale Discovery, catalog # R32AH-1, diluted 1:125). RNA Polymerase II Ser2 phosphorylation was normalized with tubulin protein levels, and EC₅₀ values were calculated with the software XLFit (IDBS, Guildford, UK) from 2-fold dilution series comprising 6 concentrations in duplicates. Results are presented in Table 6.

### 3.2 NF-kappaB Reporter Assay

Cells were maintained in RPMI cell culture medium + glutamine (PAN Biotech GmbH, Aidenbach, Germany) supplemented with 10% fetal calf serum (PAA Laboratories GmbH, Pasching, Austria) and grown at 37°C, 5% CO₂. HEK293 cells grown to 50% confluence were transfected with the Amaxa^{®} Cell Line Nucleofector^{®} Kit V (Lonza, Basel, Switzerland, catalog # VCA-1003). Transfections were performed according to manufacturer's optimized protocol for transfection of HEK293 cells. In brief, 2x10⁵ cells were transfected with 5 µg highly purified plasmid DNA. Cells were transfected with a NF-kappa B reporter plasmid (pNFkBluc), pTALluc for control, or pMAXGFP for transfection control. After transfection, cells were taken up in 500 µL RPMI1640 cell culture medium, incubated for 1 h at 37°C, and 4.5 ml DMEM without phenol red were added per transfection. Transfected cells were seeded in 96 well plates (Greiner Bio-One, Frickenhausen, Germany, catalog # 655098) with 100 µL cell suspension per well and incubated for 48h. To each well, 100 µL DMEM with 2x concentrated compound diluted from 10 mM DMSO stocks, or 100 µL DMEM with 0.4% DMSO for control wells, was added. The compounds of general formula (I) summarized in Table 6 were used in this assay. Cells were stimulated with 20ng/ml TNF alpha, and plates were incubated for 5h at 37°C/5% CO₂, Cell culture supernatants were removed to leave 100 µl medium per well, followed by addition of 100 µl Bright Glo luciferase assay reagent (Promega, Madison, WI, USA, catalog # E2620), and shaking for 5 minutes in the dark. Luminescence was measured with a Victor Photospectrometer (Perkin Elmer, Waltham, MA, USA). EC₅₀ values were calculated with the software Excel Fit (IDBS, Guildford, UK) from 2-fold dilution series comprising at least 10 concentrations in duplicates. Results are presented in Table 6.

### 3.3 TNF alpha Release Assay

Freshly isolated peripheral blood mononuclear cells (PBMCs) were seeded in 96-well cell culture plates with 200,000 cells in 100 µl cell culture medium (DMEM cell culture medium + glutamine from PAN Biotech GmbH, Aidenbach, Germany) supplemented with 10% fetal calf serum (PAA Laboratories GmbH, Pasching, Austria) per well and incubated overnight at 37°C, 5% CO₂, To each well, 100 µL cell culture medium with 2x concentrated test compounds diluted from 10 mM DMSO stocks, or 100 µL DMEM with 0.4% DMSO for control wells, was added. The compounds of general formula (I) summarized in Table 6 were used in this assay. After incubation for 1 h at 37°C, 5% CO₂, cells were stimulated with 1µg/mL LPS (Lipopolysaccharides, Sigma, catalog # L4391-1 MG; 1mg/ml stock solution), or left untreated for negative controls, and plates were incubated for 6h at 37°C/5% CO₂, The cell culture plates were centrifuged at 2000 rpm for 5 minutes, and supernatants were transferred to fresh 96-well polypropylene plates. 25µL of supernatants were transferred into 96-well-plates of the human TNF alpha-tissue culture kit (Meso Scale Discovery, Gaithersburg, Maryland, USA), and manufacturer's instructions were followed for analysis of TNF alpha levels. Chemoluminescence was measured in the Mesoscale Sector Imager, and EC₅₀ values were calculated with the software Excel Fit (IDBS, Guildford, UK) from 2-fold dilution series comprising at least 6 concentrations in duplicates. Results are presented in Table 6.

### 3.4 Cell Viability Assays

HeLa- or MDAMB468-Cells or MATU_ADR, H460, DU145, CACO-2 or B16F10 cells were maintained in RPMI 1640 or McCoy's 5A cell culture medium + glutamine (PAN Biotech GmbH, Aidenbach, Germany; order no. P04-22100; P04-05500) supplemented with 10% fetal calf serum "Gold" (PAA Laboratories GmbH, Pasching, Austria; order no. A15-151) and grown at 37°C, 5% CO₂, For the cell viability assay, cells were seeded with a density of 400 (Hela cells, DSMZ Braunschweig order no. ACC57) or 800 (MDAMB468 cells, ATCC order no. HTB-132) per well in 25 µL in 384-well plates (Greiner Bio-One, Frickenhausen, Germany; order no. 781080). After overnight incubation at 37°C/5% CO₂, 25nL or 75nL compound were added to each sample well by using BIOMEK FXP Laboratory Automation Workstation (Beckman Coulter, USA). Wells with cells and 0.1 % or 0.3% DMSO in culture medium were used as positive controls, wells with cells and 10µM staurosporine in culture medium were used as negative controls. The cells were incubated with the compounds for 72h at 37°C/5% CO₂. For measurement of cell viability 25 µL Cell Titer Glo reagent (Promega, Madison, USA; order no. G7573), 1:2 diluted with cell culture medium, was added to each well. The 384well-plates were placed for 2 min on an orbital microplate shaker and incubated for further 10 min at room temperature to stabilize the luminescence signal. Luminescence was measured by Envision Plate Reader (Perkin Elmer, USA). EC₅₀ values were calculated with the software Excel Fit (IDBS, Guildford, UK) from 3-fold dilution series comprising at least 8 concentrations in duplicates. Results are presented in Tables 6 and 7.

### 3.5. CDK2/CycE kinase assay

CDK2/CycE-inhibitory activity of compounds of the present invention was quantified employing the CDK2/CycE TR-FRET assay as described in the following paragraphs.

Recombinant fusion proteins of glutathione S-transferase (GST) and human CDK2 and of GST and human CycE, expressed in insect cells (Sf-9) and purified by glutathion-sepharose affinity chromatography, were purchased from ProQinase GmbH (Freiburg, Germany). A biotinylated peptide biotin-Ttds-YISPLKSPYKISEG (C-terminus in amide form), which can be purchased from e.g the company JERINI Peptide Technologies (Berlin, Germany), was used as a substrate for the kinase reaction.

For the assay, 50 nl of a 100-fold concentrated solution of the test compound in DMSO was pipetted into a well of a black low-volume 384-well microtiter plate (Greiner Bio-One, Frickenhausen, Germany), 2 µl of a solution of CDK2/CycE in aqueous assay buffer [50 mM Tris/HCl pH 8.0], 10 mM MgCl₂, 1.0 mM dithiothreitol, 0.1 mM sodium ortho-vanadate, 0.01% (v/v) Nonidet-P40 (Sigma)] were added. The mixture was incubated at 22 °C for 15 min to allow pre-binding of the test compounds to the enzyme before the start of the kinase reaction. Then, the kinase reaction was initiated by the addition of 3 µl of a solution of adenosine-triphosphate (ATP, 16.7 µM, resulting in a final concentration of 10 µM for an assay volume of 5 µl assay) and substrate (1.25 µM resulting in a final concentration of 0.75 µM for an assay volume of 5 µl assay) in assay buffer. The resulting mixture was incubated at 22 °C for 25 minutes. The concentration of CDK2/CycE was adjusted depending on the activity of the enzyme lot and was chosen appropriately for measurements in the linear range of the assay. Typical concentrations were in the range of 130 ng/ml. The reaction was stopped by the addition of 5 µl of a solution of TR-FRET detection reagents (0.2 µM streptavidine-XL and 1 nM anti-RB(pSer807/pSer811)-antibody from BD Pharmingen [# 558389] and 1.2 nM LANCE EU-W1024 labeled anti-mouse IgG antibody [Perkin-Elmer, product no. AD0077]) in an aqueous EDTA-solution (100 mM EDTA, 0.2 % (w/v) bovine serum albumin in 100 mM HEPES/NaOH, pH 7.0).

The resulting mixture was incubated at 22 °C for one hour to allow the formation of a complex between the phosphorylated biotinylated peptide and the detection reagents. Subsequently, the amount of phosphorylated substrate was evaluated by measuring the resonance energy transfer from the Eu-chelate to the streptavidine-XL. Therefore, the fluorescence emissions at 620 nm and 665 nm after excitation at 350 nm were measured in a high time-resolved fluorometry (HTRF) reader, e.g. a Rubystar (BMG Labtechnologies, Offenburg, Germany) or a Viewlux (Perkin-Elmer). The ratio of the emissions at 665 nm and at 622 nm was taken as a measure for the amounts of phosphorylated substrate. The data were normalised, i.e. enzyme reaction without inhibitor corresponds to 0 % inhibition, and all other assay components in the absence of the enzyme correspond to 100 % inhibition. IC₅₀ values were calculated by a 4-parameter fit using in-house software.

### 3.6. CDK9/CycT1 kinase assay

CDK9/CycT1-inhibitory activity of compounds of the present invention was quantified employing the CDK9/CycT1 TR-FRET assay as described in the following paragraphs.

Recombinant full-length His-tagged human CDK9 and CycT1, expressed in insect cells and purified by Ni-NTA affinity chromatography, were purchased from Invitrogen (catalogue number PV4131). A biotinylated peptide biotin-Ttds-YISPLKSPYKISEG (C-terminus in amide form), which can be purchased from e.g the company JERINI Peptide Technologies (Berlin, Germany), was used as a substrate for the kinase reaction.

For the assay, 50 nl of a 100-fold concentrated solution of the test compound in DMSO was pipetted into a well of a black low-volume 384-well microtiter plate (Greiner Bio-One, Frickenhausen, Germany), 2 µl of a solution of CDK9/CycT1 in aqueous assay buffer [50 mM Tris/HCl pH 8.0], 10 mM MgCl₂, 1.0 mM dithiothreitol, 0.1 mM sodium ortho-vanadate, 0.01% (v/v) Nonidet-P40 (Sigma)] were added. The mixture was incubated at 22 °C for 15 min to allow pre-binding of the test compounds to the enzyme before the start of the kinase reaction. Then, the kinase reaction was started by the addition of 3 µl of a solution of adenosine-triphosphate (ATP, 16.7 µM, resulting in a final concentration of 10 µM for an assay volume of 5 µl assay) and substrate (1.25 µM resulting in a final concentration of 0.75 µM for an assay volume of 5 µl assay) in assay buffer. The resulting mixture was incubated at 22 °C for a reaction time of 25 minutes. The concentration of CDK9/CycT1 was adjusted depending of the activity of the enzyme lot and was was chosen appropriately for measurements in the linear range of the assay. Typical concentrations were in the range of 1 µg/ml. The reaction was stopped by the addition of 5 µl of a solution of TR-FRET detection reagents (0.2 µM streptavidine-XL and 1 nM anti-RB(pSer807/pSer811)-antibody from BD Pharmingen [# 558389] and 1.2 nM LANCE EU-W1024 labeled anti-mouse IgG antibody [Perkin-Elmer, product no. AD0077]) in an aqueous EDTA-solution (100 mM EDTA, 0.2 % (w/v) bovine serum albumin in 100 mM HEPES/NaOH, pH 7.0).

The resulting mixture was incubated at 22 °C for one hour to allow the formation of a complex between the phosphorylated biotinylated peptide and the detection reagents. Subsequently, the amount of phosphorylated substrate was evaluated by measurement of the resonance energy transfer from the Eu-chelate to the streptavidine-XL. Therefore, the fluorescence emissions at 620 nm and 665 nm after excitation at 350 nm were measured in a HTRF reader, e.g. a Rubystar (BMG Labtechnologies, Offenburg, Germany) or a Viewlux (Perkin-Elmer). The ratio of the emissions at 665 nm and at 622 nm was taken as a measure for the amounts of phosphorylated substrate. The data were normalised, i.e. enzyme reaction without inhibitor corresponds to 0 % inhibition, and all other assay components in the absence of the enzyme correspond to 100 % inhibition. IC₅₀ values were calculated by a 4-parameter fit using in-house software.

### 4. Biological assay: Proliferation Assay

Cultivated tumour cells (NCI-H460, human non-small cell lung carcinoma cells, ATCC HTB-177; DU 145, hormone-independent human prostate carcinoma cells, ATCC HTB-81; HeLa-MaTu, human cervical carcinoma cells, EPO-GmbH, Berlin; HeLa-MaTu-ADR, multidrug-resistant human cervical carcinoma cells, EPO-GmbH, Berlin; HeLa human cervical tumour cells, ATCC CCL-2; Caco-2 human colorectal carcinoma, ATCC HTB-37; B16F10 mouse melanoma cells, ATCC CRL-6475) were plated at a density of 5,000 cells/well (DU145, HeLa-MaTu-ADR), 3,000 cells/well (NCl-H460, HeLa-MaTu, HeLa), 1,500 cells/well (Caco-2), or 1,000 cells/well (B16F10) in a 96-well multititer plate in 200 µL of their respective growth medium supplemented with 10% fetal calf serum. After 24 hours, the cells of one plate (zero-point plate) were stained with crystal violet (see below), while the medium of the other plates was replaced by fresh culture medium (200 µl), to which the test substances were added in various concentrations (0 µM as well as in the range of 0.001 to 10 µM; the final concentration of the solvent dimethyl sulfoxide was 0.5%). The cells were incubated in the presence of test substances for 4 days. Cell proliferation was determined by staining the cells with crystal violet: The cells were fixed by adding 20 µl/measuring point of an 11% glutaric aldehyde solution at room temperature for 15 minutes. After three washing cycles of the fixed cells with water, the plates were dried at room temperature. The cells were stained by adding 100 µl/measuring point of a 0.1 % crystal violet solution (pH 3.0). After three washing cycles of the stained cells with water, the plates were dried at room temperature. The dye was dissolved by adding 100 µl/measuring point of a 10% acetic acid solution. The extinction was determined by photometry at a wavelength of 595 nm. The change of cell number, in percent, was calculated by normalization of the measured values to the extinction values of the zero-point plate (equivalent to 0%) and the extinction of the untreated (0 µm) cells (equivalent to 100%). The IC₅₀ values were determined by means of a 4 parameter fit using the company's own software.

### Results:

### 1. Measurement of Binding Affinities to CDKs

The dissociation constants K_{d} of the compounds according to the present invention for binding to CDK9, CDK8, CDK7, and CDK2, respectively, are summarized in Table 4. Comparison of binding constants of a special compound of formula (I) for a number of different CDKs shows that binding of a compound to CKD9 is always stronger than binding to other CDKs. Thus, a compound of formula (I) binds or interacts specificially with CKD9 and at least selectively with CDK9.

**Table 4: Affinity for CDK9, CDK8, CDK7, and CDK2 of compounds according to the present invention**

| All values are measured in nM using the assay as described under 2. of Materials and Methods; "n.t." means that the compounds have not been tested in this assay. | | | | | | |
|---|---|---|---|---|---|---|
| ① | **Nomenclature** | ② | ③ | ④ | ⑤ | ⑥ |
| **B1** | 3-[(4-(2-Methoxyphenyl)-1,3,5-triazin-2-yl)amino]benzenemethanesulfonamide | 5 | n.t. | n.t. | > 10000 | n.t. |
| **B1'** | 3-[(4-(2-Methoxyphenyl)-1,3,5-triazin-2- yl)amino]benzenemethanesulfonamide trifluoroacetic acid salt | 7.6 | 48.7 | n.t. | > 10000 | 798 |
| **B10** | 3-[(4-(5-Hydroxymethyl-2-methoxyphenyl)-1,3,5-triazin-2- yl)amino]benzenemethanesulfonamide | 309 | 110 | n.t. | > 10000 | n.t. |
| **B11** | 3-[(4-(5-Formyl-2-methoxyphenyl)-1,3,5-triazin-2- yl)amino]benzenemethanesulfonamide | n.t. | n.t. | n.t. | n.t. | n.t. |
| **B12** | 3-[(4-(2-Ethoxyphenyl)-1,3,5-triazin-2-yl)amino]benzenemethanesulfonamide | 10 | 11.1 | > 10000 | > 10000 | 2347 |
| **B13** | 3-[(4-(2-Benzyloxyphenyl)-1,3,5-triazin-2-yl)amino]benzenemethanesulfonamide | 5 | n.t. | n.t. | > 10000 | 687 |
| **B13'** | 3-[(4-(2-Benzyloxyphenyl)-1,3 5-triazin-2-yl)amino]benzenemethanesulfonamide trifluoroacetic acid salt | 5.5 | 5 | 4857 | > 10000 | 88 |
| **B14** | 3-[(4-(2-Phenoxyphenyl)-1,3,5-triazin-2-yl)amino]benzenemethanesulfonamide | 25.5 | 26.4 | 5704 | > 10000 | 3453c |
| **B15** | 3-[(4-(1,3-Benzodioxol-4-yl)-1,3,5-triazin-2-yl)amino]benzenemethanesulfonamide | 141 | 2456 | > 10000 | > 10000 | 6464 |
| **B16** | 3-[(4-(2-((4-Pyridinyl)methoxy)phenyl)-1,3,5-triazin-2-yl)amino]benzenemethanesulfonamide | < 5 | 5.7 | > 10000 | > 10000 | 1693 |
| **B17** | 3-[(4-(2-(4-(tert-Butoxycarbonylamino)butoxy)phenyl)-1,3,5-triazin-2-yl)amino]benzenemethanesulfonamide | 15 | 6.4 | n.t. | 12820 | 2850 |
| **B18** | 3-[(4-(4-Methoxypyridin-3-yl)-1,3,5-triazin-2-yl)amino]benzenemethanesulfonamide | 104 | 302 | 1761 | > 10000 | n.t. |
| **B19** | 3-[(4-(3-Methoxypyridin-4-yl)-1,3,5-triazin-2-yl)amino]benzenemethanesulfonamide | 7 | 11.5 | n.t. | > 10000 | n.t. |
| **B2** | 3-[(4-(4-Fluoro-2-methoxyphenyl)-1,3,5-triazin-2-yl)amino]benzenemethanesulfonamide | 5 | n.t. | n.t. | > 10000 | n.t. |
| **B2'** | 3-[(4-(4-Fluoro-2-methoxyphenyl)-1,3,5-triazin-2-yl)amino]benzenemethanesulfonamide hydrochloride | 5.6 | n.t. | n.t. | > 10000 | 207 |
| **B2"** | 3-[(4-(4-Fluoro-2-methoxyphenyl)-1,3,5-triazin-2-yl)amino]benzenemethanesulfonamide trifluoroacetic acid salt | 6.6 | 5 | > 10000 | > 10000 | n.t. |
| **B22** | 3-[(4-(2-(Cyclopropylamino-methyl)phenyl)-1,3,5-triazin-2-yl)amino]benzenemethanesulfonamide | 425 | 373 | n.t. | n.t. | n.t. |
| **B23** | 3-[(4-(6-Aminopyridin-3-yl)-1,3,5-triazin-2-yl)amino]benzenemethanesulfonamide | 99 | 26.2 | n.t. | > 10000 | n.t. |
| **B24** | 3-[(4-(2-(Methoxymethyl)phenyl)-1,3,5-triazin-2-yl)amino]benzenemethanesulfonamide | 907 | 1106 | n.t. | > 10000 | n.t. |
| **B3** | 3-[(4-(5-Fluoro-2-methoxyphenyl)-1,3,5-triazin-2-yl)amino]benzenemethanesulfonamide | 16 | 5 | n.t. | > 10000 | 375 |
| **B4** | 3-[(4-(6-Fluoro-2-methoxyphenyl)-1,3,5-triazin-2-yl)amino]benzenemethanesulfonamide | 54 | 20.9 | > 10000 | > 10000 | 704 |
| **B5** | 3-[(4-(3,5-Difluoro-2-methoxyphenyl)-1,3,5-triazin-2-yl)amino]benzenemethanesulfonamide | 835 | 286 | n.t. | > 10000 | n.t. |
| **B6** | 3-[(4-(4-Chloro-2-methoxyphenyl)-1,3,5-triazin-2-yl)amino]benzenemethanesulfonamide | 11 | 12.5 | 6784 | > 10000 | n.t. |
| **B7** | 3-[(4-(5-Chloro-2-methoxyphenyl)-1,3,5-triazin-2-yl)amino]benzenemethanesulfonamide | 55 | 101 | > 10000 | > 10000 | n.t. |
| **B8** | 3-[(4-(2-Methoxy-4-trifluoromethyl-phenyl)-1,3,5-triazin-2yl)amino]benzenemethanesulfonamide | 1225 | 709 | n.t. | > 10000 | n.t. |
| **B9** | 3-[(4-(2-Methoxy-5-trifluoromethyl-phenyl)-1,3,5-triazin-2-yl)amino]benzenemethanesulfonamide | 398 | 1593 | n.t. | > 10000 | n.t. |
| **C1** | 3-[(4-(2-Methoxyphenyl)-1,3,5-triazin-2-yl)amino]benzenesulfonamide | n.t. | n.t. | n.t. | n.t. | n.t. |
| **C1'** | 3-[(4-(2-Methoxyphenyl)-1,3 5-triazin-2-yl)amino]benzenesulfonamide trifluoroacetic acid salt | < 5 | 5 | n.t. | > 10000 | 220 |
| **D1** | 2-[3-((4-(2-Methoxyphenyl)-1,3,5-triazin-2-yl)amino)phenyl]ethanesulfonamide | 32.8 | 99.5 | n.t. | > 10000 | 310 |
| **D2** | 2-[3-((4-(4-Fluoro-2-methoxyphenyl)-1,3,5-triazin-2-yl)amino)phenyl]ethanesulfonamide | 9.5 | 5.0 | n.t. | > 10000 | < 5 |
| **E1** | 3-[(4-(2-Methoxyphenyl)-1,3,5-triazin-2-yl)amino]benzamide | 136 | 301 | n.t. | > 10000 | 2164 |
| **F1** | 6-[(4-(2-Methoxyphenyl)-1,3,5-triazine-2-yl)amino]-2,3-dihydro-1H-indole-1-sulfonamide | 131 | 39.7 | n.t. | 13372 | 1098 |
| **G1** | rac-S-[3-((4-(2-Methoxyphenyl)-1,3,5-triazin-2-yl)amino)phenyl]-N-ethoxycarbonyl-S-methyl-sulfoximide | 105 | 87.2 | n.t. | > 10000 | > 10000 |
| **H1** | 4-(2-Methoxyphenyl)-N-(3-nitrophenyl)-1,3,5-triazine-2-amine | 17.8 | 15.8 | n.t. | > 30000 | 68 |
| **I1** | 3-[(4-(2-(4-Aminobutoxy)phenyl)-1,3,5-triazin-2-yl)amino]benzenemethanesulfonamide | 101 | 52 | n.t. | > 30000 | n.t. |
| **K1** | N-[3-((4-(2-Methoxyphenyl)-1,3,5-triazine-2-yl)amino)phenyl]-methanesulfonamide | 38.5 | 35.9 | n.t. | > 30000 | 283 |
| **L1** | N-[3-((4-(2-Methoxyphenyl)-1,3,5-triazine-2-yl)amino)phenyl]-propanesulfonamide | 45.5 | n.t. | n.t. | > 30000 | n.t. |
| **M1** | N-[3-((4-(2-Methoxyphenyl)-1,3,5-triazine-2-yl)amino)phenyl]acetamide | 146 | 71.2 | n.t. | > 30000 | 1454 |
| **N1** | N-[3-((4-(2-Methoxyphenyl)-1,3,5-triazine-2-yl)amino)phenyl]-N'-phenyl-urea | 478 | 159 | > 10000 | > 30000 | 2359 |
| **O1** | 3-[(4-(2-Methoxy-5-(methylamino-methyl)phenyl)-1,3,5-triazin-2-yl)amino]benzenemethanesulfonamide | 5407 | 5978 | n.t. | > 30000 | n.t. |
| **P1** | 4-(2-Methoxyphenyl)-N-phenyl-1,3,5-triazine-2-amine | 126 | n.t. | n.t. | > 30000 | 709 |
| **Q1** | tert-Butyl [4-((3-((4-(4-Fluoro-2-methoxyphenyl)-1,3,5-triazin-2-yl)amino)phenyl)methylsulfonamido) butyl]carbamate | 12.9 | 5 | n.t. | > 10000 | 2086 |
| **R1** | N-(4-Aminobutyl)-1-[3-((4-(4-fluoro-2-methoxyphenyl)-1,3,5-triazin-yl)amino)phenyl]methanesulfonamide | 5 | 5 | n.t. | > 10000 | 2925 |
| **S1** | 4-(2-Methoxyphenyl)-N-(3-(methylsulfonyl)phenyl)-1,3,5-triazin-2-amine | 6.9 | n.t. | n.t. | > 10000 | 851 |
| **T1** | 4-[(4-(2-Methoxyphenyl)-1,3,5-triazin-2-yl)amino]benzenemethane-sulfonamide | 43.7 | n.t. | n.t. | > 10000 | 237 |

| | | | | | | |
|---|---|---|---|---|---|---|
| ①: Compound Number ②: CDK9 / Cyclin T1 ③: CDK9 / Cyclin K ④: CDK8 ⑤: CDK7 ⑥: CDK2 | | | | | | |

### 2. Measurement of half maximal inhibitory concentration to CDKs in enzymatic assays

The inhibitory activities of the compounds according to the present invention are shown in Table 5 as half-maximal inhibition constant (IC₅₀) values for inhibition of CDK9, CDK1, CDK2, CDK4, CDK6, and CDK7, respectively.

**Table 5: Inhibition for CDK9, CDK1, CDK2, CDK4, CDK6, and CDK7 of compounds according to the present invention**

| All values are measured in nM using the LANCE assay as described under 2. of Materials and Methods; "n.t." means that the compounds have not been tested in this assay. | | | | | | | |
|---|---|---|---|---|---|---|---|
| ① | **Nomenclature** | ② | ③ | ④ | ⑤ | ⑥ | ⑦ |
| **B1'** | 3-[(4-(2-Methoxyphenyl)-1,3,5-triazin-2-yl)amino]benzenemethanesulfonamide trifluoroacetic acid salt | 96.5 | 12050 | 3083 | > 10000 | > 10000 | > 10000 |
| **B12** | 3-[(4-(2-Ethoxyphenyl)-1,3,5-triazin-2-yl)amino]benzenemethanesulfonamide | 102 | 11550 | 2930 | > 10000 | > 10000 | > 10000 |
| **B13'** | 3-[(4-(2-Benzyloxyphenyl)-1,3,5-triazin-2-yl)amino]benzenemethanesulfonamide trifluoroacetic acid salt | 5.7 | 3398 | 1771 | 681 | > 10000 | > 10000 |
| **B14'** | 3-[(4-(2-Phenoxyphenyl)-1,3,5-triazin-2-yl)amino]benzenemethanesulfonamide | 9 | n.t. | n.t. | n.t. | n.t. | n.t. |
| **B16'** | 3-[(4-(2-((4-Pyridinyl)methoxy)phenyl)-1,3,5-triazin-2-yl)amino]benzenemethanesulfonamide | 9 | 6772 | 1067 | > 10000 | > 10000 | > 10000 |
| **B2** | 3-[(4-(4-Fluoro-2-methoxyphenyl)-1,3,5-triazin-2-yl)amino]benzenemethanesulfonamide | 10.6 | n.t. | n.t. | n.t. | n.t. | n.t. |
| **B2'** | 3-[(4-(4-Fluoro-2-methoxyphenyl)-1,3,5-triazin-2-yl)amino]benzenemethanesulfonamide hydrochloride | 10.4 | n.t. | n.t. | n.t. | n.t. | n.t. |
| **B2"** | 3-[(4-(4-Fluoro-2-methoxyphenyl)-1,3,5-triazin-2-yl)amino]benzenemethanesulfonamide trifluoroacetic acid salt | 11.6 | 899 | 568 | 3205 | > 10000 | > 10000 |
| **B4** | 3-[(4-(6-Fluoro-2-methoxyphenyl)-1,3,5-triazin-2-yl)amino]benzenemethanesulfonamide | 201 | 5682 | 1445 | > 10000 | > 10000 | > 10000 |
| **B6** | 3-[(4-(4-Chloro-2-methoxyphenyl)-1,3,5-triazin-2-yl)amino]benzenemethanesulfonamide | 16.2 | > 10000 | > 10000 | > 10000 | > 10000 | > 10000 |
| **C1'** | 3-[(4-(2-Methoxyphenyl)-1,3,5-triazin-2-yl)amino]benzenesulfonamide trifluoroacetic acid salt | 20.7 | 1349 | 781 | 1505 | > 10000 | > 10000 |
| **D1** | 2-[3-((4-(2-Methoxyphenyl)-1,3,5-triazin-2-yl)amino)phenyl]ethanesulfonamide | 139 | 2572 | 515 | 9322 | > 10000 | > 10000 |
| **D2** | 2-[3-((4-(4-Fluoro-2-methoxyphenyl)-1,3,5-triazin-2-yl)amino)phenyl]ethanesulfonamide | 12.8 | > 10000 | 423 | > 10000 | > 10000 | > 10000 |
| **E1** | 3-[(4-(2-Methoxyphenyl)-1,3,5-triazin-2-yl)amino]benzamide | 837 | n.t. | n.t. | n.t. | n.t. | n.t. |
| **H1** | 4-(2-Methoxyphenyl)-N-(3-nitrophenyl)-1,3,5-triazine-2-amine | 194 | n.t. | n.t. | n.t. | n.t. | n.t. |

| ① | **Nomenclature** | ② | ③ | ④ | ⑤ | ⑥ | ⑦ |
|---|---|---|---|---|---|---|---|
| **K1** | N-[3-((4-(2-Methoxyphenyl)-1,3,5-triazine-2-yl)amino)phenyl]-methanesulfonamide | 72 | > 10000 | 826 | > 10000 | > 10000 | > 10000 |
| **L1** | N-[3-((4-(2-Methoxyphenyl)-1,3,5-triazine-2-yl)amino)phenyl]-propanesulfonamide | 111 | > 10000 | > 10000 | > 10000 | > 10000 | > 10000 |
| **P1** | 4-(2-Methoxyphenyl)-N-phenyl-1,3,5-triazine-2-amine | 937 | n.t. | n.t. | n.t. | n.t. | n.t. |
| **S1** | 4-(2-Methoxyphenyl)-N-(3-(methylsulfonyl)phenyl)-1,3,5-triazin-2-amine | 24.7 | n.t. | 1767 | n.t. | n.t. | > 10000 |
| **T1** | 4-[(4-(2-Methoxyphenyl)-1,3,5-triazin-2-yl)amino]benzenemethane-sulfonamide | 190 | n.t. | 259 | n.t. | n.t. | > 10000 |

| | | | | | | | |
|---|---|---|---|---|---|---|---|
| ①: Compound Number ②: CDK9 ③: CDK1 ④: CDK2 ⑤: CDK4 ⑥: CDK6 ⑦: CDK7 | | | | | | | |

**Table 5a: Inhibition for CDK9 and CDK2 of compounds according to the present invention**

| The IC₅₀ (inhibitory concentration at 50% of maximal effect) values are indicated in nM or µM, "n.t." means that the compounds have not been tested in this assay. | | | |
|---|---|---|---|
| ① | **Nomenclature** | ② | ③ |
| **U1** | 1-[3-({4-[4-fluoro-2-(trifluoromethyl)phenyl]-1,3,5-triazin-2-yl}amino)phenyl]methanesulfonamide | 15 µM | 15 µM |
| **U2** | 1-[3-({4-[4-fluoro-2-(propan-2-yloxy)phenyl]-1,3,5-triazin-2-yl}amino)phenyl]methanesulfonamide | 28 nM | 880 nM |
| **U3** | 1-(3-{[4-(2-cyano-4-fluorophenyl)-1,3,5-triazin-2-y1]amino}phenyl)methanesulfonamide | 3.3 µM | 14 µM |
| **U4** | N-[5-fluoro-2-(4-{[3-(sulfamoylethyl)phenyl]amino}-1,3,5-triazin-2-yl)phenyl]acetamide | 3.6 µM | 3,0 µM |
| **U5** | 1-[3-({4-[2-(cyclopropylmethoxy)-4-fluorophenyl]-1,3,5-triazin-2-yl}amino)phenyl]methanesulfonamide | 34 nM | 1,6 µM |
| **U6** | 1-(3-{[4-(3,4-difluoro-2-methoxyphenyl)-1,3,5-triazin-2-yl]amino}phenyl)methanesulfonamide | 370 nM | 12 µM |
| **U7** | 1-(3-{[4-(4,5-difluoro-2-methoxyphenyl)-1,3,5-triazin-2-yl]amino}phenyl)methanesulfonamide | 14 nM | 930 nM |
| **U8** | 4-(4-fluoro-2-methoxyphenyl)-N-[6-(methylsulfonyl)pyridin-3-yl]-1,3,5-triazin-2-amine | 320 nM | 170 nM |
| **B14** | 3-[(4-(2-Phenoxyphenyl)-1,3,5-triazin-2-yl)amino]benzenemethanesulfonamide | 62 nM | 4,0 µM |
| **B13** | 3-[(4-(2-Benzyfoxyphenyl)-1,3,5-triazin-2-yl)amino]benzenemethanesulfonamide | 3,2nM | 380 nM |
| **B1** | 3-[(4-(2-Methoxyphenyl)-1,3,5-triazin-2-yl)amino]benzenemethanesulfonamide | 21 nM | 1,8 µM |
| **B2** | 1-(3-{[4-(4-fluoro-2-methoxyphenyl)-1,3,5-triazin-2-yl]amino}phenyl)methanesulfonamide | 8,1 nM | 930 nM |
| **B2'** | 1-(3-{[4-(4-fluoro-2-methoxyphenyl)-1,3,5-triazin-2-yl]amino}phenyl)methanesulfonamide hydrochloride | 14 nM | 670 nM |
| **C1** | 3-[(4-(2-Methoxyphenyl)-1,3,5-triazin-2-yl)amino]benzenesulfonamide | 10 nM | 450 nM |
| **Ref1** | C-{3-[6-(2-Methoxy-phenyl)-pyrimidin-4-ylamino]-phenyl}-methanesulfonamide | 43 nM | 2,1 µM |
| **Ref2** | C-{3-[6-(2-Methoxy-phenyl)-pyrimidin-4-ylamino]-phenyl}-N-methylmethanesulfonamide | 40 nM | 1,6 µM |
| **Ref3** | [6-(5-Chloro-2-methyl-phenyl)-[1,3,5]triazin-4-yl]-(4-chloro-phenyl)-amine | >20 µM | >20 µM |
| **Ref4** | C-{3-[6-(2-Methoxy-phenyl)-pyrimidin-4-ylamino]-phenyl}-N,N-dimethylmethanesulfonamide | 49 nM | 2,7µM |
| **Ref5** | 4-[4-(2-Benzoylaminophenyl)-[1,3,5]triazin-2-ylamino]benzamide | 8,4 µM | 14 µM |

| | | | |
|---|---|---|---|
| ①: Compound Number ②: CDK9 CDK9/CycT1 kinase assay as described under 3.6 of Materials and Methods ③: CDK2 CDK2/CycE kinase assay as described under 3.5 of Materials and Methods | | | |

Source for Reference Examples Ref1 to Ref5:
Ref1: Example 85 on page 45 of WO20081290801
Ref2: Example 49 on page 38 of WO2008129080A1
Ref3: Example 33 on page 32 of WO2003/037346A1
Ref4: Example 20 on page 32 of WO200812908A1
Ref5: Example 152 on page 32 of US20040116388

### 3. Cellular Assays

The cellular activity of the compounds according to the present invention are shown in Table 6 as half-maximal inhibition constant (IC₅₀) values on LPS-induced TNF alpha release in PBMCs, NF-kappaB reporter gene activation, cellular CDK9 activity (RNA Polymerase II Ser2 phosphorylation), and cell viability in Hela- or MDAMB468-Cells, respectively. The cellular activities of the compounds according to the present invention are shown in Table 7 as half-maximal inhibition constant (IC₅₀) values of cell viability in MaTu/ADR, H460, DU145, CACO-2 or B16F10 cells.

**Table 6: Inhibition of LPS-induced TNF alpha release in PBMCs, NF-kappaB reporter gene activation, cellular CDK9 activity (RNA Polymerase II Ser2 phosphorylation), and cell viability in MDAMB468-Cells by compounds according to the present invention. All IC₅₀ (inhibitory concentration at 50% of maximal effect) values are indicated in nM; "n.t." means that the compounds have not been tested in this assay.**

| ① | **Nomenclature** | ② | ③ | ④ | ⑤ |
|---|---|---|---|---|---|
| **B1** | 3-[(4-(2-Methoxyphenyl)-1,3,5-triazin-2-yl)aminolbenzenemethanesulfonamide | n.t. | n.t. | n.t. | 2.20 |
| **B1'** | 3-[(4-(2-Methoxyphenyl)-1,3,5-triazin-2-yl)amino]benzenemethanesulfonamide trifluoroacetic acid salt | 2.71 | 3.56 | 10.86 | 2.99 |
| **B2** | 3-[(4-(4-Fluoro-2-methoxyphenyl)-1,3,5-triazin-2-yl)aminolbenzenemethanesulfonamide | 1.03 | n.t. | n.t. | 0.42 |
| **B2"** | 3-[(4-(4-Fluoro-2-methoxyphenyl)-1,3,5-triazin-2-yl)amino]benzenemethanesulfonamide trifluoroacetic acid salt | 0.64 | 1.6 | 3.64 | 0.66 |
| **B3** | 3-[(4-(5-Fluoro-2-methoxyphenyl)-1,3,5-triazin-2-yl)amino]benzenemethanesulfonamide | 3.24 | 2.47 | 9.62 | n.t. |
| **B4** | 3-[(4-(6-Fluoro-2-methoxyphenyl)-1,3,5-triazin-2-yl)amino]benzenemethanesulfonamide | 6.53 | 9.71 | 49.82 | n.t. |
| **B6** | 3-[(4-(4-Chloro-2-methoxyphenyl)-1,3,5-triazin-2-yl)amino]benzenemethanesulfonamide | n.t. | 2.49 | 15.3 | n.t. |
| **B12** | 3-[(4-(2-Ethoxyphenyl)-1,3,5-triazin-2-yl)aminolbenzenemethanesulfonamide | n.t. | 3.67 | 17.48d | n.t. |
| **B13'** | 3-[(4-(2-Benzyloxyphenyl)-1,3,5-triazin-2-yl)amino] benzenemethanesulfonamide trifluoroacetic acid salt | 0.12 | 0.36 | n.t. | 0.16 |
| **B13** | 3-[(4-(2-Benzyloxyphenyl)-1,3,5-triazin-2-yl)amino]benzenemethanesulfonamide | n.t. | n.t. | n.t. | n.t. |
| **B14** | 3-[(4-(2-Phenoxyphenyl)-1,3,5-triazin-2-yl)amino]benzenemethanesulfonamide | n.t. | 3.96 | 14.49 | n.t. |
| **B16** | 3-[(4-(2-((4-Pyridinyl)methoxy)phenyl)-1,3,5-triazin-2-yl)amino]benzenemethanesulfonamide | n.t. | 0.96 | 11.92 | n.t. |
| **B19** | 3-[(4-(3-Methoxypyridin-4-yl)-1,3,5-triazin-2-yl)amino]benzenemethanesulfonamide | 10.57 | 19.86 | 15.51 | n.t. |
| **B23** | 3-[(4-(6-Aminopyridin-3-yl)-1,3,5-triazin-2-yl)amino]benzenemethanesulfonamide | n.t. | > 30 | > 30 | n.t. |
| **C1** | 3-[(4-(2-Methoxyphenyl)-1,3,5-triazin-2-yl)amino]benzenesulfonamide | n.t. | n.t. | n.t. | n.t. |
| **C1'** | 3-[(4-(2-Methoxyphenyl)-1,3,5-triazin-2-yl)aminolbenzenesulfonamide trifluoroacetic acid salt | 0.50 | 1.47 | 2.87 | 0.80 |
| **D1** | 2-[3-((4-(2-Methoxyphenyl)-1,3,5-triazin-2-yl)amino)phenyl]ethanesulfonamide | 5.37 | 4.98 | n.t. | 4.61 |
| **D2** | 2-[3-((4-(4-Fluoro-2-methoxyphenyl)-1,3,5-triazin-2-yl)amino)phenyl]ethanesulfonamide | n.t. | 1.63 | 10.76 | n.t. |
| **E1** | 3-[(4-(2-Methoxyphenyl)-1,3,5-triazin-2-yl)amino]benzamide | n.t. | n.t. | n.t. | 23.03 |
| **H1** | 4-(2-Methoxyphenyl)-N-(3-nitrophenyl)-1,3,5-triazine-2-amine | 2.92 | 2.67 | n.t. | 0.83 |
| **K1** | N-[3-((4-(2-Methoxyphenyl)-1,3,5-triazin-2-yl)amino)phenyl]-methanesulfonamide | 4.42 | 5.52 | n.t. | n.t. |
| **L1** | N-[3-((4-(2-Methoxyphenyl)-1,3,5-triazine-2-yl)amino)phenyl]-propanesulfonamide | 2.41 | 6.53 | > 30 | 4.50 |
| **N1** | N-[3-((4-(2-Methoxyphenyl)-1,3,5-triazin-2-yl)amino)phenyl]-N'-phenyl-urea | 6.71 | 18.4 | n.t. | n.t. |
| **P1** | 4-(2-Methoxyphenyl)-N-phenyl-1,3,5-triazine-2-amine | 13.9 | 18.77 | n.t. | 13.33 |

| | | | | | |
|---|---|---|---|---|---|
| ①: Compound Number ②: TNF alpha release ③: NF-kappaB activation ④: RNA Polymerase II Ser2 Phosphorylation ⑤: Cell Viability - MDAMB468 cells | | | | | |

**Table 7: Inhibition of cell viability in HeLa, MaTu/ADR, H460, DU145, CACO-2 and B16F10 cells by compounds according to the present invention.**

| All IC₅₀ (inhibitory concentration at 50% of maximal effect) values are indicated in µM, "n.t." means that the compounds have not been tested in this assay. | | | | | | | |
|---|---|---|---|---|---|---|---|
| ① | **Nomenclature** | ② | ③ | ④ | ⑤ | ⑥ | ⑦ |
| **B1'** | 3-[(4-(2-Methoxyphenyl)-1,3,5-triazin-2-yl)amino] benzenemethanesulfonamide trifluoroacetic acid salt | 3.2 | 3.2 | 3.5 | 3.8 | 4.3 | 4.4 |
| **B2'** | 1-(3-{[4-(4-fluoro-2-methoxyphenyl)-1,3,5-triazin-2-yl]amino}phenyl)methanesulfonamide hydrochloride | 1 | 1 | 1.5 | 1 | 1.5 | 1.1 |
| **B2** | 3-[(4-(4-Fluoro-2-methoxyphenyl)-1,3,5-triazin-2-yl)amino] benzenemethanesulfonamide | 1 | 1 | 1.3 | 1.5 | 1.6 | 1.6 |
| **B13'** | 3-[(4-(2-Benzyloxyphenyl)-1,3,5-triazin-2-yl)amino] benzenemethanesulfon-amide trifluoroacetic acid salt | 0.31 | 0.29 | 0.32 | 0.33 | 0.37 | 0.32 |
| **C1'** | 3-[(4-(2-Methoxyphenyl)-1,3,5-triazin-2-yl)amino]benzenesulfonamide trifluoroacetic acid salt | 1 | 0.76 | 1.1 | 1 | 1.1 | 1 |
| **U2** | 1-[3-({4-[4-fluoro-2-(propan-2-yloxy)phenyl]-1,3,5-triazin-2-yl}amino)phenyl]methanesulfonamide | 2.7 | n.t. | n.t. | n.t. | n.t. | n.t. |
| **U3** | 1-(3-{[4-(2-cyano-4-fluorophenyl)-1,3,5-triazin-2-yl]amino}phenyl)methanesulfonamide | 10 | n.t. | n.t. | n.t. | n.t. | n.t. |
| **U7** | 1-(3-{[4-(4,5-difluoro-2-methoxyphenyl)-1,3,5-triazin-2-yl]amino}phenyl)methanesulfonamide | 1.2 | n.t. | n.t. | n.t. | n.t. | n.t. |
| **U8** | 4-(4-fluoro-2-methoxyphenyl)-N-[6-(methylsulfonyl)pyridin-3-yl-1,3,5-triazin-2-amine | 4.2 | n.t. | n.t. | n.t. | n.t. | n.t. |
| **Ref1** | C-{3-[6-(2-Methoxy-phenyl)-pyrimidin-4-ylamino]-phenyl]-methanesulfonamide | 3.4 | n.t. | n.t. | n.t. | n.t. | n.t. |
| **Ref2** | C-{3-[6-(2-Methoxy-phenyl)-pyrimidin-4-ylamino]-phenyl}-N-methylmethanesulfonamide | 4.6 | n.t. | n.t. | n.t. | n.t. | n.t. |
| **Ref4** | C-{3-[6-(2-Methoxy-phenyl)-pyrimidin-4-ylamino]-phenyl}-N,N-dimethylmethanesulfonamide | 6.1 | n.t. | n.t. | n.t. | n.t. | n.t. |

| | | | | | | | |
|---|---|---|---|---|---|---|---|
| ①: Compound Number ②: Cell Viability - HeLa cells ③: Cell Viability - MaTu/ADR cells ④: Cell Viability - H460 cells ⑤: Cell Viability - DU145 cells ⑥: Cell Viability - CACO-2 cells ⑦: Cell Viability - B16F10 cells | | | | | | | |

## Claims

1. Compound of the general formula (I) 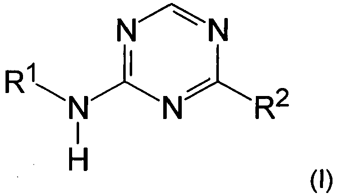 wherein
**R¹** represents 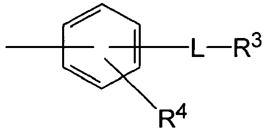 in which
L is-CH₂-, -CH₂CH₂-, or -CF₂-;
**R³** is -SO₂NH₂, -SO₂NH(CH₃), -SO₂N(CH₃)₂, -SO₂NH(CH₂CH₂OCH₃), -NHSO₂CH₃, -NHSO₂CH₂CH₃, -NHSO₂CH₂CH₂CH₃, -NHSO₂CF₃, -SO₂CH₃, -NHSO₂NH₂, -SO(NH)CH₃;
**R⁴** is -H, -CH₃, -F, -Cl, or -CF₃;
**R²** represents 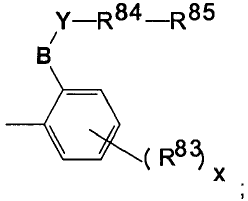 in which the group **-Y-R⁸⁴-R⁸⁵** is -OCH₃, -OCH₂CH₃, -OCH₂CH₂CH₃, -OCH₂CH₂CH₂CH₃, -OCH(CH₃)₂, -OPh, -OCH₂Ph, -OCH₂(4-pyridyl);
**R⁸³** is -H, -F, or -Cl;
x is 0, 1, or 2;
or enantiomers, stereoisomeric forms, mixtures of enantiomers, diastereomers, mixtures of diastereomers, hydrates, solvates, acid salt forms, tautomers, and racemates and pharmaceutically acceptable salts thereof.

2. The compound according to claim 1, wherein
**R¹** represents 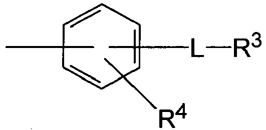 in which
the substituent **-L-R³** is -CH₂SO₂NH₂, -CH₂CH₂SO₂NH₂, -CF₂SO₂NH₂, -CH₂NHSO₂NH₂, -CH₂SO(NH)CH₃;
**R⁴** is -H;
**R²** represents 2-methoxyphenyl, 4-fluoro-2-methoxyphenyl, or 6-fluoro-2-methoxyphenyl.

3. The compound according to claim 1, wherein
**R¹** represents 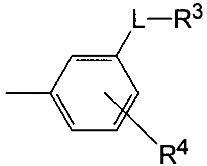 in which
the substituent **-L-R³** is -CH₂SO₂NH₂,
**R⁴** is -H;
**R²** represents 2-methoxyphenyl, 4-fluoro-2-methoxyphenyl or 2-benzyloxyphenyl,
or their salts, solvates or salts of solvates and especially the hydrochloride salt or the trifluoroacetate salt.

4. The compound according to claim 1, wherein the compound is selected from the group of compounds consisting of:
3-[(4-(2-Methoxyphenyl)-1,3,5-triazin-2-yl)amino]benzenemethanesulfonamide,
3-[(4-(4-Fluoro-2-methoxyphenyl)-1,3,5-triazin-2-yl)amino]benzenemethanesulfonamide,
3-[(4-(5-Fluoro-2-methoxyphenyl)-1,3,5-triazin-2-yl)amino]benzenemethanesulfonamide,
3-[(4-(6-Fluoro-2-methoxyphenyl)-1,3,5-triazin-2-yl)amino]benzenemethanesulfonamide,
3-[(4-(3,5-Difluoro-2-methoxyphenyl)-1,3,5-triazin-2-yl)amino]benzenemethanesulfonamide,
3-[(4-(4-Chloro-2-methoxyphenyl)-1,3,5-triazin-2-yl)amino]benzenemethanesulfonamide,
3-[(4-(5-Chloro-2-methoxyphenyl)-1,3,5-triazin-2-yl)amino]benzenemethanesulfonamide,
3-[(4-(2-Methoxy-4-trifluoromethyl-phenyl)-1,3,5-triazin-2-yl)amino]benzenemethanesulfonamide,
3-[(4-(2-Methoxy-5-trifluoromethyl-phenyl)-1,3,5-triazin-2-yl)amino]benzenemethanesulfonamide,
3-[(4-(5-Hydroxymethyl-2-methoxyphenyl)-1,3,5-triazin-2-yl)amino]benzenemethanesulfonamide,
3-[(4-(5-Formyl-2-methoxyphenyl)-1,3,5-triazin-2-yl)amino]benzenemethanesulfonamide,
3-[(4-(2-Ethoxyphenyl)-1,3,5-triazin-2-yl)amino]benzenemethanesulfonamide,
3-[(4-(2-Benzyloxyphenyl)-1,3,5-triazin-2-yl)amino]benzenemethanesulfonamide,
1-(3-{[4-(2-phenoxyphenyl)-1,3,5-triazin-2-yl]amino}phenyl)methanesulfonamide,
3-[(4-(1,3-Benzodioxol-4-yl)-1,3,5-triazin-2-yl)amino]benzenemethanesulfonamide,
3-[(4-(2-((4-Pyridinyl)methoxy)phenyl)-1,3,5-triazin-2-yl)amino]benzenemethanesulfonamide,
3-[(4-(2-(4-(tert-Butoxycarbonylamino)butoxy)phenyl)-1,3,5-triazin-2-yl)amino]-benzenemethanesulfonamide,
3-[(4-(2-((Morpholin-4-yl)methyl)phenyl)-1,3,5-triazin-2-yl)amino]benzene-methanesulfonamide,
3-[(4-(2-((Piperidin-1-yl)methyl)phenyl)-1,3,5-triazin-2-yl)amino]benzene-methanesulfonamide,
3-[(4-(2-(Cyclopropylam ino-methyl)phenyl)-1, 3, 5-triazin-2-yl)amino]benzenemethanesulfonamide,
3-[(4-(2-(Methoxymethyl)phenyl)-1,3,5-triazin-2-yl)amino]benzenemethanesulfonamide,
2-[3-((4-(2-Methoxyphenyl)-1,3,5-triazin-2-yl)amino)phenyl]ethanesulfonamide,
2-[3-((4-(4-Fluoro-2-methoxyphenyl)-1,3,5-triazin-2-yl)amino)phenyl]ethane-sulfonamide,
3-[(4-(2-(4-Aminobutoxy)phenyl)-1,3,5-triazin-2-yl)amino]benzenemethane-sulfonamide,
3-[(4-(2-Methoxy-5-(methylamino-methyl)phenyl)-1,3,5-triazin-2-yl)amino]-benzenemethanesulfonamide,
tert-Butyl-[4-((3-((4-(4-Fluoro-2-methoxyphenyl)-1,3,5-triazin-2-yl)amino)phenyl)-methylsulfonamido) butyl]carbamate,
N-(4-Aminobutyl)-1-[3-((4-(4-fluoro-2-methoxyphenyl)-1,3,5-triazin-2-yl)amino)-phenyl]methanesulfonamide,
4-[(4-(2-Methoxyphenyl)-1,3,5-triazin-2-yl)amino]benzenemethane-sulfonamide,
1-[3-({4-[4-fluoro-2-(trifluoromethyl)phenyl]-1,3,5-triazin-2-yl}amino)phenyl]-methanesulfonamide,
1-[3-({4-[4-fluoro-2-(propan-2-yloxy)phenyl]-1,3,5-triazin-2-yl}amino)phenyl]-methanesulfonamide,
1-(3-{[4-(2-cyano-4-fluorophenyl)-1,3,5-triazin-2-yl]amino}phenyl)methanesulfonamide,
1-[3-({4-[2-(cyclopropylmethoxy)-4-fluorophenyl]-1,3,5-triazin-2-yl}amino)phenyl]-methanesulfonamide,
1-(3-{[4-(3,4-difluoro-2-methoxyphenyl)-1,3,5-triazin-2-yl]amino}phenyl)methanesulfonamide,
1-(3-{[4-(4,5-difluoro-2-methoxyphenyl)-1,3,5-triazin-2-yl]amino}phenyl)methanesulfonamide,
3-[(4-(2-Methoxyphenyl)-1,3,5-triazin-2-yl)amino]benzenemethanesulfonamide trifluoroacetic acid salt,
1-(3-{[4-(4-fluoro-2-methoxyphenyl)-1,3,5-triazin-2-yl]amino}phenyl)methanesulfonamide hydrochloride,
3-[(4-(4-Fluoro-2-methoxyphenyl)-1,3,5-triazin-2-yl)amino]benzenemethanesulfonamide trifluoroacetic acid salt,
3-[(4-(2-Benzyloxyphenyl)-1,3,5-triazin-2-yl)amino] benzenemethanesulfon-amide trifluoroacetic acid salt.

5. A compound according to any one of claims 1 to 4 for use as pharmaceutically active agent.

6. Compound of general formula (I) as defined in any one of claims 1 - 4 for use in the prophylaxis and / or treatment of infectious diseases, including opportunistic diseases, immunological diseases, autoimmune diseases, cardiovascular diseases, cell proliferative diseases, inflammation, erectile dysfunction and stroke.

7. Compound for use according to claim 6, wherein the proliferative disease is selected from the group comprising:
adenocarcinoma, choroidal melanoma, acute leukemia, acoustic neurinoma, ampullary carcinoma, anal carcinoma, astrocytoma, basal cell carcinoma, pancreatic cancer, desmoid tumor, bladder cancer, bronchial carcinoma, breast cancer, Burkitt's lymphoma, corpus cancer, CUP-syndrome (carcinoma of unknown primary), colorectal cancer, small intestine cancer, small intestinal tumors, ovarian cancer, endometrial carcinoma, ependymoma, epithelial cancer types, Ewing's tumors, gastrointestinal tumors, gastric cancer, gallbladder cancer, gall bladder carcinomas, uterine cancer, cervical cancer, glioblastomas, gynecologic tumors, ear, nose and throat tumors, hematologic neoplasias, hairy cell leukemia, urethral cancer, skin cancer, skin testis cancer, brain tumors (gliomas), brain metastases, testicle cancer, hypophysis tumor, carcinoids, Kaposi's sarcoma, laryngeal cancer, germ cell tumor, bone cancer, colorectal carcinoma, head and neck tumors (tumors of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumors gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, oligodendroglioma, esophageal carcinoma, osteolytic carcinomas and osteoplastic carcinomas, osteosarcomas, ovarial carcinoma, pancreatic carcinoma, penile cancer, plasmocytoma, prostate cancer, pharyngeal cancer, rectal carcinoma, retinoblastoma, vaginal cancer, thyroid carcinoma, Schneeberg disease, esophageal cancer, spinalioms, T-cell lymphoma (mycosis fungoides), thymoma, tube carcinoma, eye tumors, urethral cancer, urologic tumors, urothelial carcinoma, vulva cancer, wart appearance, soft tissue tumors, soft tissue sarcoma, Wilm's tumor, cervical carcinoma, tongue cancer, invasive ductal carcinoma, invasive lobular carcinoma, ductal carcinoma *in situ*, lobular carcinoma *in situ,* small-cell lung carcinoma, non-small-cell lung carcinoma, bronchial adenoma, pleuropulmonary blastoma, mesothelioma, brain stem glioma, hypophtalmic glioma, cerebellar astrocytoma, cerebral astrocytoma, neuroectodermal tumours, pineal tumors, sarcoma of the uterus, salivary gland cancers, anal gland adenocarcinomas, mast cell tumors, pelvis tumours, ureter tumours, hereditary papillary renal cancers, sporadic papillary renal cancers, intraocular melanoma, hepatocellular carcinoma (liver cell carcinomas with or without fibrolamellar variant), cholangiocarcinoma (intrahepatic bile duct carcinoma), mixed hepatocellular cholangiocarcinoma, squamous cell carcinoma, malignant melanoma, Merkel cell skin cancer, non-melanoma skin cancer, hypopharyngeal cancer, nasopharyngeal cancer, oropharyngeal cancer, oral cavity cancer, squamous cell cancer, oral melanoma, AIDS-related lymphoma, cutaneous T-cell lymphoma, lymphoma of the central nervous system, malignant fibrous histiocytoma, lymphosarcoma, rhabdomyosarcoma, malignant histiocytosis, fibrosarcoma, hemangiosarcoma, hemangiopericytoma, leiomyosarcoma., canine mammary carcinoma, and feline mammary carcinoma.

8. Pharmaceutical composition comprising at least one compound according to any one of claims 1 to 4 as an active ingredient, together with at least one pharmaceutically acceptable carrier, excipient and/or diluent.

9. Pharmaceutical composition according to claim 8 further comprising one or more other anti-tumor agents.

10. Method for synthesizing the compound of general formula (I) as defined in claim 1 according to the following procedures:
Reacting 2,4-dichloro-1,3,5-triazine with an aniline of the general formula R¹NH₂, wherein R¹ has the meanings as defined in claim 1, in order to give the 2-arylamino-4-chloro-1,3,5-triazine which is then reacted with a boronic acid derivative of the general formula R²-B(OR)₂, wherein R² has the meanings as defined in claim 1 and both R moieties represent independently of each other hydrogen or an alkyl chain with 1 - 10 carbon atoms or a cycloalkyl chain with 3 - 12 carbon atoms or both moieties R represent together a residue derived from pinacol in order to obtain the compound of general formula (I) according to the following reaction scheme: 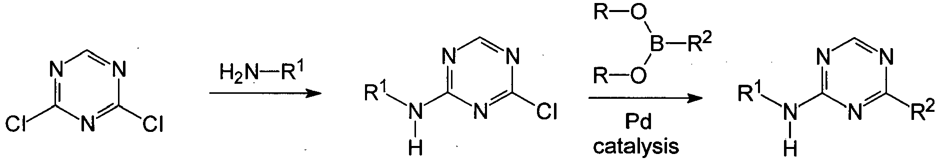 or
reacting 2,4-dichloro-1,3,5-triazine with a boronic acid derivative of the general formula R²-B(OR)₂, wherein R² has the meanings as defined in claim 1 and both R moieties represent independently of each other hydrogen or an alkyl chain with 1 - 10 carbon atoms or a cycloalkyl chain with 3 - 12 carbon atoms or both moieties R represent together a residue derived from pinacol in order to give the R² substituted chloro-1,3,5-triazine which is then reacted with the aniline of the general formula R¹NH₂, wherein R¹ has the meanings as defined in claim 1, in order to obtain the compound of general formula (I) according to the following reaction scheme: 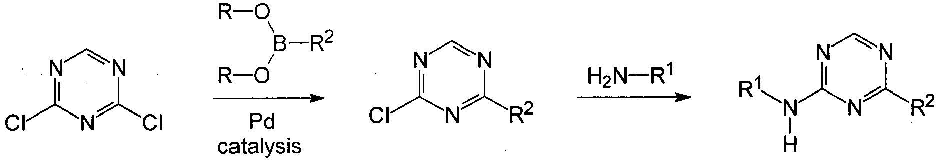 or
reacting an amide of the general formula R²-CONH₂, wherein R² has the meanings as defined in claim 1, with an acetal of N,N-dimethylformamide in order to give the intermediate N-acylformamidine which is subsequently reacted with the guanidine of the general formula R¹-NH-C(NH)NH₂, wherein R¹ has the meanings as defined in claim 1, in order to obtain the compound of general formula (I) according to the following reaction scheme:

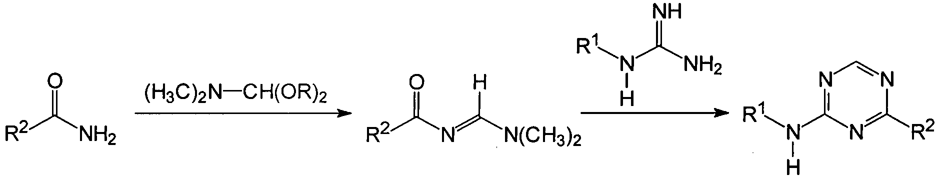

## Patentansprüche

1. Verbindung der allgemeinen Formel (I) 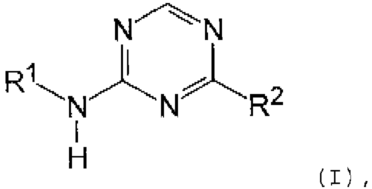 wobei
**R¹** für 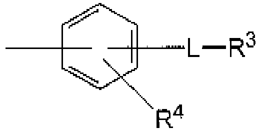 steht,
in welcher
**L** für -CH₂-, -CH₂CH₂- oder -CF₂- steht,
**R³** für -SO₂NH₂, -SO₃NH(CH₃), -SO₂N(CH₃)₂, -SO₂NH(CH₂CH₂OCH₃), -NHSO₂CH₃, -NHSO₂CH₂CH₃, -NHSO₂CH₂CH₂CH₃, -NHSO₂CF₃, -SO₂CH₃, -NHSO₂NH₂, -SO(NH)CH₃ steht,
**R⁴** für -H, -CH₃, -F, -Cl oder -CF₃ steht,
**R²** für 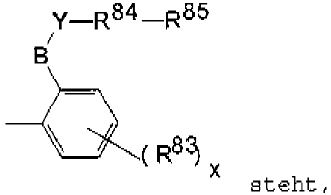 in welcher die Gruppe **-B-Y-R⁸⁴-R⁸⁵** für -OCH₃, -OCH₂CH₃, -OCH₂CH₂CH₃, -OCH₂CH₂CH₂CH₃, -OCH(CH₃)₂, -OPh, -OCH₂Ph, -OCH₂(4-Pyridyl) steht,
R⁸³ für -H, -F oder -Cl steht,
x für 0, 1 order 2 steht,
oder Enantiomere, stereoisomere Formen, Mischungen von Enantiomeren, Diastereomere, Mischungen von Diastereomeren, Hydrate, Solvate, Säuresalzformen, Tautomere und Racemate und pharmazeutisch unbedenkliche Salze davon.

2. Verbindung nach Anspruch 1, wobei
**R¹** für 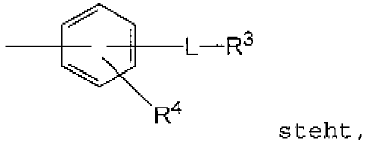 in welcher
der Substituent **-L-R³** für -CH₂SO₂NH₂, -CH₂CH₃SO₃NH₂, -CF₂SO₂NH₂, -CH₂NHSO₂NH₂, -CH₂SO(NH)CH₃ steht,
**R⁴** für -H steht,
R² für 2-Methoxyphenyl, 4-Fluor-2-methoxyphenyl oder 6-Fluor-2-methoxyphenyl steht,

3. Verbindung nach Anspruch 1, wobei
**R¹** für 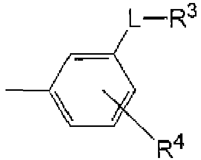 steht,
in welcher
der Substituent **-L-R³** für -CH₂SO₂NH₂ steht,
**R⁴** für -H steht,
**R²** für 2-Methoxyphenyl, 4-Fluor-2-methoxyphenyl oder 2-Benzyloxyphenyl steht,
oder deren Salze, Solvate oder Salze von Solvaten und insbesondere das Hydrochloridsalz oder das Trifluoracetatsalz.

4. Verbindung nach Anspruch 1, wobei die Verbindung ausgewählt ist aus der Gruppe von Verbindungen betstehend aus:
3-[(4-(2-Methoxyphenyl)-1,3,5-triazin-2-yl)amino]benzolmethansulfonamid,
3-[(4-(4-Fluor-2-methoxyphenyl)-1,3,5-triazin-2-yl)amino]benzolmethansulfonamid,
3-[(4-(5-Fluor-2-methoxyphenyl)-1,3,5-triazin-2-yl)amino]benzolmethansulfonamid,
3-[(4-(6-Fluor-2-methoxyphenyl)-1,3,5-triazin-2-yl)amino]benzolmethansulfonamid,
3-[(4-(3,5-Difluor-2-methoxyphenyl)-1,3,5-triazin-2-yl)amino]benzolmethansulfonamid,
3-[(4-(4-Chlor-2-methoxyphenyl)-1,3,5-triazin-2-yl)amino]benzolmethansulfonamid,
3-[(4-(5-Chlor-2-methoxyphenyl)-1,3,5-triazin-2-yl)amino]benzolmethansulfonamid,
3-[(4-(2-Methoxy-4-trifluormethylphenyl)-1,3,5-triazin-2-yl)amino]benzolmethansulfonamid,
3-[(4-(2-Methoxy-5-trifluormethylphenyl)-1,3,5-triazin-3-yl)amino]benzolmethansulfonamid,
3-[(4-(5-Hydroxymethyl-2-methoxyphenyl)-1,3,5-triazin-2-yl)amino]benzolmethansulfonamid,
3-[(4-(5-Formyl-2-methoxyphenyl)-1,3,5-triazin-2-yl)amino]benzolmethansulfonamid,
3-[(4-(3-Ethoxyphenyl)-1,3,5-triazin-2-yl)amino]benzolmethansulfonamid,
3-[(4-(2-Benzyloxyphenyl)-1,3,5-triazin-2-yl)amino]benzolmethansulfonamid,
1-(3-{[4-(2-Phenoxyphenyl)-1,3,5-triazin-2-yl]amino}phenyl)methansulfonamid,
3-[(4-(1,3-Benzodioxol-4-yl)-1,3,5-triazin-2-yl)amino]benzolmethansulfonamid,
3-[(4-(2-((4-Pyridinyl)methoxy)phenyl)-1,3,5-triazin-2-yl)amino]benzolmethansulfonamid,
3-[(4-(2-(4-(tert.-Butoxycarbonylamino)butoxy)phenyl)-1,3,5-triazin-2-yl)amino]benzolmethansulfonamid,
3-[(4-(2-((Morpholin-4-yl)methyl)phenyl)-1,3,5-triazin-2-yl)amino]benzolmethansulfonamid,
3-[(4-(2-((Piperidin-1-yl)methyl)phenyl)-1,3,5-triazin-2-yl)amino]benzolmethansulfonamid,
3- [(4- (2- -(Cyclopropylaminomethyl)phenyl)-1,3,5-triazin-2-yl)amino]benzolmethansulfonamid,
3-[(4-(2-(Methoxymethyl)phenyl)-1,3,5-triazin-2-yl]amino]benzolmethansulfonamid,
2-[3-((4-(2-Methoxyphenyl)-1,3,5-triazin-2-yl)amino)phenyl]ethansulfonamid,
2-[3-((4-[4-Fluor-2-methoxyphenyl)-1,3,5-triazin-2-yl)amino)phenyl]ethansulfonamid,
3-[(4-(2-[4-Aminobutoxy)phenyl)-1,3,5-triazin-2-yl)amino]benzolmethansulfonamid,
3-[(4-(2-Methoxy-5-(methylaminomethyl)phenyl)-1,3,5-triazin-2-yl)amino]benzolmethasulfonamid,
[4-((3-((4-(4-Fluor-2-methoxyphenyl)-1,3,5-triazin-2-yl)amino)phenyl)methylsulfonamido)butyl]carbamidsäure-tert.-butylester,
N-(4-Aminobutyl)-1-[3-((4-(4-fluor-2-methoxyphenyl)-1,3,5-triazin-2-yl)amino)phenyl]methansulfonamid,
4-[(4-(2-Methoxyphenyl)-1,3,5-triazin-2-yl)amino]benzolmethansulfonamid,
1-[3-({4-[4-Fluor-2-(trifluormethyl)phenyl]-1,3,5-triazin-2-yl}amino)phenyl]methansulfonamid,
1-[3-({4-[4-Fluor-2-(propan-2-yloxy)phenyl]-1,3,5-triazin-2-yl}amino)phenyl]methansulfonamid,
1-(3-{[4-(2-Cyano-4-fluorphenyl)-1,3,5-triazin-2-yl]amino}phenyl)methansulfonamid,
1-[3-({4-[2-(Cyclopropylmethoxy)-4-fluorphenyl]-1,3,5-triazin-2-yl}amino)phenyl]methansulfonamid,
1-(3-{[4-(3,4-Difluor-2-methoxyphenyl)-1,3,5-triazin-2-yl]amino}phenyl)methansulfonamid,
1-(3-{[4-{4,5-Difluor-2-methoxyphenyl)-1,3,5-triazin-2-yl]amino}phenyl)methansulfonamid,
3-[(4-(2-Methoxyphenyl)-1,3,5-triazin-2-yl)amino]benzolmethansulfonamid-Trifluoressigsäuresalz,
1-(3-{[4-(4-Fluor-2-methoxyphenyl)-1,3,5-triazin-2-yl]amino}phenyl)methansulfonamidhydrochlorid,
3-[(4-{4-Fluor-2-methoxyphenyl)-1,3,5-triazin-2-yl)amino]benzolmethansulfonamid-Trifluoressigsäuresalz,
3-[(4-(2-Benzyloxyphenyl)-1,3,5-triazin-2-yl)amino]benzolmethansulfonamid-Trifluoressigsäuresalz.

5. Verbindung nach einem der Ansprüche 1 bis 4 zur Verwendung als pharmazeutischer Wirkstoff.

6. Verbindung der allgemeinen Formel (I) nach einem der Ansprüche 1 - 4 zur verwendung bei der Prophylaxe und/oder Behandlung von Infektionskrankheiten einschließlich opportunistischen Krankheiten, immunologischen Krankheiten, Autoimmunkrankheiten, Herz-Kreislauf-Krankheiten, zellproliferativen Krankheiten, Entzündung, erektiler Dysfunktion und Schlaganfall.

7. Verbindung zur Verwendung nach Anspruch 6, wobei die proliferative Krankheit ausgewählt ist aus der Gruppe umfassend oder betstehend aus:
Adenokarzinom, Aderhautmelanom, akuter Leukämie, Akustikusneurinom, Ampullenkarzinom, Analkarzinom, Astrozytom, Basaliom, Bauchspeicheldrüsenkrebs, Bindegewebstumor, Blasenkrebs, Bronchialkarzinom, Brustkrebs, Burkitt-Lymphom, Korpuskarzinom, CUP-Syndrom (Carcinoma of Unknown Primary), Dickdarmkrebs, Dünndarmkrebs, Dünndarmtumore, Eierstockkrebs, Endometriumkarzinom, Ependymom, Epithel-Krebsarten, Ewing-Tumoren, gastrointestinalen Tumoren, Magenkrebs, Gallenblasenkrebs, Gallenblasenkarzinome, Gebärmutterkrebs, Gebärmutterhalskrebs, Glioblastomen, gynäkologischen Tumoren, Hals-Nasen- und Ohrentumoren, hämatologischen Neoplasien, Haarzell-Leukämie, Harnröhrenkrebs, Hautkrebs, Hauthodenkrebs, Hirntumoren (Gliomen), Hirnmetastasen, Hodenkrebs, Hypophysentumor, Karzinoiden, Kaposi-Sarkom, Kehlkopfkrebs, Keimzellentumor, Knochenkrebs, kolorektalem Karzinom, Kopf- und Halstumoren (Tumore des Hals-, Nasen- und Ohrenbereichs), Kolonkarzinom, Kraniopharyngiome, Mundkrebs (Krebs im Wundbereich und auf den Lippen), Krebs des zentralen Nervensystems, Leberkrebs, Leber-metastasen, Leukämie, Lidtumor, Lungenkrebs, Lymphdrüsenkrebs (Hodgkin/Non-Hodgkin-Lymphome), Lymphome, Magenkrebs, malignem Melanom, malignem Neoplasma, malignen Tumoren des Magen-Darm-Trakts, Mammakarzinom, Mastdarmkrebs, Medulloblastome, Melanom, Meningiome, Morbus Hodgkin, Mycosis fungoides, Nasenkrebs, Neurinom, Neuroblastom, Nierenkrebs, Nierenzellkarzinome, Oligodendrogliom, Ösophaguskarzinom, osteolytischen Karzinomen und osteoplastischen Karzinomen, Osteosarkomen, Ovarialkarzinom, Pankreaskarzinom, Peniskrebs, Plasmozytom, Prostatakrebs, Rachenkrebs, Rektumkarzinom, Retinoblastom, Scheidenkrebs, Schilddrüsenkrebs, Schneebergerkrankheit, Speiseröhrenkrebs, Spinaliomen, T-Zell-Lymphom (Mycosis fungoides), Thymom, Tubenkarzinom, Tumoren des Auges, Urethrakrebs, urologischen Tumoren, Urothelkarzinom, Vulvakrebs, dem Auftreten von Warzen, Weichteiltumoren, Weichteilsarkom, Wilm-Tumor, zervixkarzinom, Zungenkrebs, invasivem duktalem Karzinom, invasivem lobulärem Karzinom, duktalem Karzinom in situ, lobulärem Karzinom in situ, kleinzelligem Lungenkarzinom, nicht-kleinzelligem Lungenkarzinom, Bronchialadenom, pleuropulmonalem Blastom, Mesotheliom, Hirnstammgliom, hypophtalmischem Gliom, zerebellarem Astrozytom, zerebralem Astrocytom, neuroektodermalen Tumoren, pinealen Tumoren, Uterussarkom, Speicheldrüsenkrebs, Analdrüsenadenokarzinomen, Mastzellentumoren, Beckentumoren, Harnleitertumoren, angeborenen papillären Nierenkrebsen, sporadischen papillären Nierenkrebsen, intraokularem Melanom, hepatozellulärem Karzinom (Leberzellkarzinomen mit oder ohne fibrolamellarer Variante), Cholangiokarzinom (intrahepatisches Gallengangskarzinom), gemischtem hepatozellulärem Cholangiokarzinom, Plattenepithelkarzinom, malignem Melanom, Merkelzellkarzinom, Nicht-Melanom-Hautkrebs, hypopharyngealem Krebs, nasopharyngealem Krebs, oropharyngealem Krebs, Krebs der Mundhöhle, Plattenepithelkrebs, orales Melanom, mit AIDS in Zusammenhand stehendes Lymphom, kutanem T-Zellen-Lymphom, Lymphom des zentralen Nervensystems, malignem fibrösem Histiozytom, Lymphosarkom, Rhabdomyosarkom, maligner Histiozytose, Fibrosarkom, Hämangiosarkom, Hämangioperizytom, Leiomyosarkom, Mammakarzinom des Hundes und Mammakarzinom der Katze.

8. Pharmazeutische Zusammensetzung, umfassend mindestens eine Verbindung nach einem der Ansprüche 1 bis 4 als Wirkstoff, zusammen mit mindestens einem pharmazeutisch unbedenklichen Träger, Exzipienten und/oder Verdünnungsmittel.

9. Pharmazeutische Zusammensetzung nach Anspruch 8, welche weiterhin ein oder mehrere weitere Antitumormittel umfasst.

10. Vorfahren zur Synthese der wie in Anspruch 1 definierten Verbindung der allgemeinen Formel (I) gemäß den folgenden Vorschriften:
Umsetzung von 2,4-Dichlor-1,3,5-triazin mit einem Anilin der allgemeinen Formel R¹NH₂, in welcher R¹ die in Anspruch 1 definierten Bedeutungen hat, unter Erhalt des 2-Arylamino-4-chlor-1,3,5-triazins, das dann mit einem Boronsäurederivat der allgemeinen Formel R²-B(OR)₂, in welcher R² die in Anspruch 1 definierten Bedeutungen hat und die beiden R-Gruppierungen unabhängig voreinander für Wasserstoff oder eine Alkylkette mit 1-10 Kohlenstoffatomen oder eine Cycloalkylkette mit 3-12 Kohlenstoffatomen stehen oder beide Gruppierungen R zusammen für einen von Pinakol abgeleiteten Rest stehen, umgesetzt wird, unter Erhalt der Verbindung der allgemeinen Formel (I), gemäß dem folgenden Reaktionsschema: 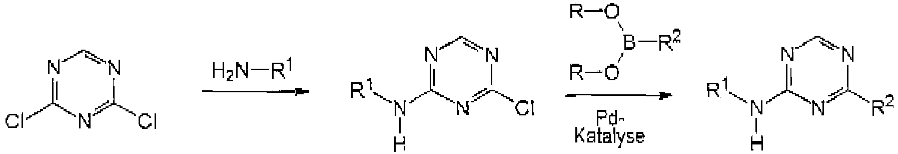 oder
Umsetzung von 2,4-Dichlor-1,3,5-triazin mit einem Boronsäurederivat der allgemeinen Formel R²-B(OR)₂, in welcher R³ die in Anspruch 1 definierten Bedeutungen hat und die beiden R-Gruppierungen unabhängig voreinander für Wasserstoff oder eine Alkylkette mit 1-10 Kohlenstoffatomen oder eine Cycloalkylkette mit 3-12 Kohlenstoffatomen stehen oder beide Gruppierungen R zusammen für eignen von Pinakol abgeleiteten Rest stehen, unter Erhalt des R²-substituierten Chlor-1,3,5-triazins, das dann mit dem Anilin der allgemeinen Formel R¹NH₂, in welcher R¹ die in Anspruch 1 definierten Bedeutungen hat, umgesetzt wird, unter Erhalt der Verbindung der allegemeinen Formel (I) gemäß dem folgenden Reaktionsschema: 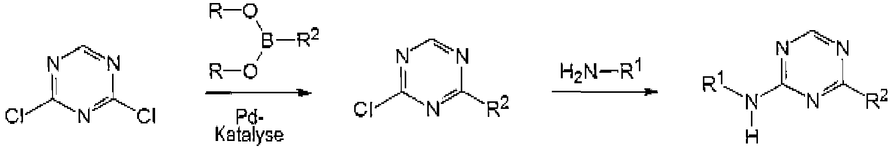 oder
Umsetzung eines Amids der allegemeinen Formel R²-CONH₂, in welcher R² die wie in Anspruch 1 definierten Bedeutungen hat, mit einem Acetal von N,N-Dimethylformamid unter Erhalt des N-Acylformamidin-Zwischenprodukts, das anschließen mit dem Guanidin der allegemeinen Formel R¹-NH-C(NH)NH₂, in welcher R¹ die wie in Aspruch 1 definierten Bedeutungen hat, umgesetzt wird, unter Erhalt der Verbindung der allgemeinen Formel (I) gemäß dem folgenden Reaktionsschema:

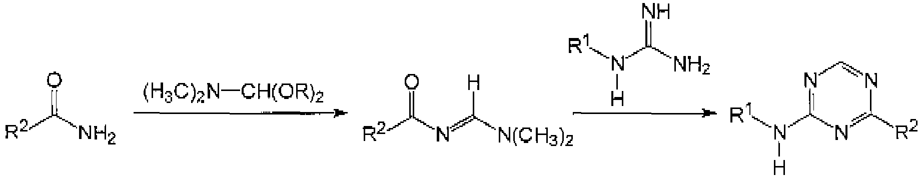

## Revendications

1. Composé de formule générale (I) 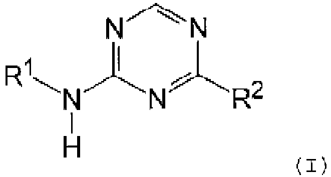 où
R¹ représente 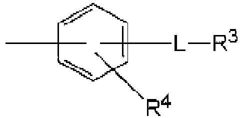 dans laquelle
L représente -CH₂-, -CH₂CH₂- ou -CF₂- ;
R³ représente -SO₂NH₂, -SO₂NH(CH₃), -SO₂N(CH₃)₂, - SO₂NH(CH₂CH₂OCH₃), -NHSO₂CH₃, -NHSO₂CH₂CH₃, -NHSO₂CH₂CH₂CH₃, -NHSO₂CF₃, -SO₂CH₃, -NHSO₂NH₂, -SO(NH)CH₃ ;
R⁴ représente -H, -CH₃, -F, -Cl- ou -CF₃ ;
R² représente 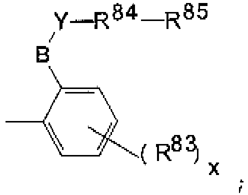 dans laquelle le groupement **-B-Y-R⁸⁴-R⁸⁵** représente -OCH₃, -OCH₂CH₃, -OCH₂CH₂CH₃, -OCH₂CH₂CH₂CH₃, -OCH(CH₃)₂,-OPh, -OCH₂Ph, -OCH₂(4-pyridyle) ;
R⁸³ représente -H, -F ou -Cl ;
x est égal à 0, 1 ou 2 ;
ou énantiomères, formes stéréoisomères, mélanges d'énantiomères, diastéréoisomères, mélanges de diastéréoisomère, hydrates, solvates, formes salines acides, tautomères et racémates, ainsi que leurs sels pharmaceutiquement acceptables.

2. Composé selon la revendication 1, où R¹ représente 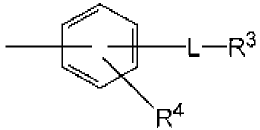 dans laquelle
le substituait -L-R³ représente -CF₂SO₂NH₂,-CH₂CH₂SO₂NH_{2,} -CF₂SO₂NH₂, -CH₂NHSO₂NH₂, -CH₂SO(NH)CH₃ ;
R⁴ représente -H ;
R² représente un groupement 2-méthoxyphényle, 4-fluoro-2-méthoxyphényle ou 6-fluoro-2-méthoxyphényle.

3. Composé selon la revendication 1, où
R¹ représente 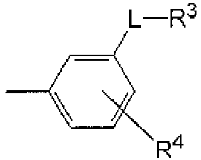 dans laquelle
le substituant -L-R³ représente -CH₂SO₂NH₂,
R⁴ représente -H ;
R² représente un groupement 2-méthoxyphényle, 4-fluoro-2-méthoxyphényle ou 2-benzyloxyphényle,
ou leurs sels, solvates ou sels de solvates, et spécialement le sel de chlorhydrate ou le sel de trifluoroacétate.

4. Composé selon la revendication 1, où le composé est choisi dans le groupe constitué par les composés suivants :
3-[(4-(2-Méthoxyphényl)-1,3,5-triazin-2-yl)amino]benzèneméthanesulfonamide,
3-[(4-(4-Fluoro-2-méthoxyphényl)-1,3,5-triazin-2-yl)amino]benzèneméthane-sulfonamide,
3-[(4-(5-Fluoro-2-méthoxyphényl)-1,3,5-triazin-2-yl)amino]benzèneméthane-sulfonamide,
3-[(4-(6-Fluoro-2-méthoxyphényl)-1,3,5-triazin-2-yl)amino]-benzèneméthane-sulfonamide,
3-[(4-(3,5-Difluoro-2-méthoxyphényl)-1,3,5-triazin-2-yl)amino]benzèneméthane-sulfonamide,
3-[(4-(4-Chloro-2-méthoxyphényl)-1,3,5-triazin-2-yl)amino]benzèneméthane-sulfonamide,
3-[(4-(5-Chloro-2-méthoxyphényl)-1,3,5-triazin-2-yl)amino]benzèneméthane-sulfonamide,
3-[(4-(2-Méthoxy-4-trifluorométhyl-phényl)-1,3,5-triazin-2-yl)amino]benzène-méthanesulfonamide,
3-[(4-(2-Méthoxy-5-trifluorométhyl-phényl)-1,3,5-triazin-2-yl)amino]benzène-méthanesulfonamide,
3-[(4-(5-Hydroxyméthyl-2-méthoxyphényl)-1,3,5-triazin-2-yl)amino]benzène-méthanesulfonamide,
3-[(4-(5-Formyl-2-méthoxyphényl)-1,3,5-triazin-2-yl)amino]benzèneméthane-sulfonamide,
3-[(4-(2-Éthoxyphényl)-1,3,5-triazin-2-yl)amino]benzèneméthanesulfonamide,
3-[(4-(2-Benzyloxyphényl)-1,3,5-triazin-2-yl)amino]benzèneméthanesulfonamide,
1-(3-{[4-(2-phénoxyphényl)-1,3,5-triazin-2-yl]amino}phényl)méthanesulfonamide,
3-[(4-(1,3-Benzodioxol-4-yl)-1,3,5-triazin-2-yl)amino]benzèneméthanesulfonamide,
3-[(4-(2-((4-Pyridinyl)méthoxy)phényl)-1,3,5-triazin-2-yl)amino]benzèneméthane-suifonamide,
3-[(4-(2-(4-(tert-Butoxycarbonylamino)butoxy)phényl)-1,3,5-triazin-2-yl)amino]-benzèneméthanesulfonamide,
3-[(4-(2-((Morpholin-4-yl)méthyl)phényl)-1,3,5-triazin-2-yl)amino]benzène-méthanesulfonamide,
3-[(4-(2-((Pipéridin-1-yl)méthyl)phényl)-1,3,5-triazin-2-yl)amino]benzène-méthanesulfonamide,
3-[(4-(2-(Cyclopropylamino-méthyl)phényl)-1,3,5-triazin-2-yl)amino]benzène-méthanesulfonamide,
3-[(4-(2-(Méthoxyméthyl)phényl)-1,3,5-triazin-2-yl)amino]benzène-méthanesulfonamide,
2-[3-((4-(2-Méthoxyphényl)-1,3,5-triazin-2-yl)amino)phényl]éthanesulfonamide,
2-[3-((4-(4-Fluoro-2-méthoxyphényl)-1,3,5-triazin-2-yl)amino)phényl]éthane-sulfonamide,
3-[(4-(2-(4-Aminobutoxy)phényl)-1,3,5-triazin-2-yl)amino]benzèneméthane-sulfonamide,
3-[(4-(2-Méthoxy-5-(méthylamino-méthyl)phényl)-1,3,5-triazin-2-yl)amino]-benzèneméthanesulfonamide,
Carbamate de tert-butyl-[4-((3-(4-(4-fluoro-2-méthoxyphényl)-1,3,5-triazin-2-yl)amino)phényl)-méthylsulfonamido)butyle],
N-(4-Aminobutyl)-1-[3-((4-(4-fluoro-2-méthoxyphényl)-1,3,5-triazin-2-yl)amino)-phényl]méthanesulfonamide,
4-[(4-(2-Méthoxyphényl)-1,3,5-triazin-2-yl)amino]benzèneméthane-sulfonamide,
1-[3-({4-[4-Fluoro-2-(trifluorométhyl)phényl]-1,3,5-triazin-2-yl}amino)phényl]-méthanesulfonamide,
1-[3-({4-[4-Fluoro-2-(propan-2-yloxy)phényl]-1,3,5-triazin-2-yl}amino)phényl]-méthanesulfonamide,
1-(3-{[4-(2-cyano-4-fluorophényl)-1,3,5-triazin-2-yl]amino}phényl)méthane-sulfonamide,
1-[3-({4-[2-(cyclopropylméthoxy)-4-fluorophényl]-1,3,5-triazin-2-yl}amino)phényl]-méthanesulfonamide,
1-{3-{[4-(3,4-difluoro-2-méthoxyphényl)-1,3,5-triazin-2-yl]amino}phényl)méthane-sulfonamide,
1-(3-{[4-(4,5-difluoro-2-méthoxyphényl)-1,3,5-triazin-2-yl]amino}phényl)méthane-sulfonamide,
3-[(4-(2-Méthoxyphényl)-1,3,5-triazin-2-yl)amino]benzèneméthanesulfonamide, sel d'acide trifluoroacétique,
1-(3-{[4-(4-fluoro-2-méthoxyphényl)-1,3,5-triazin-2-yl]amino}phényl)méthane-sulfonamide, chlorhydrate,
3-[(4-(4-Fluoro-2-methoxyphenyl)-1,3,5-triazin-2-yl)amino]benzène-méthanesulfonamide, sel d' acide trifluoroacétique,
3-[(4-(2-Benzyloxyphényl)-1,3,5-triazin-2-yl)amino]benzèneméthanesulfonamide, sel d'acide trifluoroacétique.

5. Composé selon l'une quelconque des revendications 1. à 4 pour utilisation en tant que principe actif pharmaceutique.

6. Composé de formule générale (I) selon l'une quelconque des revendications 1. à 4 pour utilisation dans le traitement prophylactique et/ou thérapeutique des maladies infectieuses, y comprise les maladies opportunistes, les maladies immunologiques, les maladies auto-immunes, les maladies cardiovasculaires, les maladies cellulaires prolifératives, l'inflammation, les troubles de l'érection et les accidents vasculaires cérébraux.

7. Composé pour utilisation selon la revendication 6, où la maladie proliférative est choisie dans le groupe comprenant les suivantes :
adénocarcinome, mélanome choroïdien, leucémie aiguë, neurinome de l'acoustique, carcinome de l'ampoule, carcinome anal, astrocytome,
carcinome à cellules basales, cancer du pancréas, tumeur desmoïde, cancer de la vessie, carcinome bronchique, cancer du sein, lymphome de Burkitt, cancer du corps de l'utérus, syndrome CUP (carcinome de primitif inconnu), cancer colorectal, cancer de l'intestin grêle, tumeurs de l'intestin grêle, cancer de l'ovaire, carcinome de l'endomètre, épendymome, types de cancer épithéliaux,
sacomes d'Ewing, tumeurs gastro-intestinales, cancer de l'estomac, cancer de la vésicule biliaire, carcinomes de la vésicule biliaire, cancer de l'utérus, cancer du col de l'utérus, glioblastomes, tumeurs gynécologiques, tumeurs de l'oreille, du nez et de la gorge, néoplasies hématologiques, leucémie à tricholeucocytes, cancer de l'urètre, cancer de la peau, cancer cutané du testicule, tumeurs cérébrales (gliomes), métastases cérébrales, cancer du testicule, tumeur hypophysaire, carcinoïdes,
sarcome de Kaposi, cancer du larynx, tumeur des cellules germinales, cancer de l'os, carcinome colorectal, tumeurs de la tête et du cou (tumeurs de la zone de l'oreille, du nez et de la gorge), carcinome du côlon, craniopharyngiomes, cancer oral (cancer de la zone de la bouche et des lèvres), cancer du système nerveux central, cancer du foie, métastases hépatiques, leucémie, tumeur des paupières, cancer du poumon, cancer des ganglions lymphatique (lymphomes hodgkiniens/non hodgkiniens),
lymphomes, cancer de l'estomac, mélanose malin, néoplasme malin, tumeurs malignes du tractus gastro-intestinal, carcinome du sein, cancer du rectum, médulloblastomes, mélanose, méningiomes, lymphoma de Hodgkin, mycosis fongoïde,
cancer du nez, neurinome, neuroblastome, cancer du rein, carcinomes des cellules rénales, oligodendrogliome, cancer de l'oesophage, carcinomes ostéolytiques et carcinomes ostéoplasiques, ostéosarcomes, carcinome ovarien, carcinome pancréatique, cancer du pénis, plasmocytome, cancer de la prostate, cancer du pharynx, carcinome rectal, rétinoblastome,
cancer du vagin, carcinome thyroïdien, maladie de Schneeberg, cancer de l'oesophage, spinaliomes, lymphome à cellules T (mycosis fongoïde), thymome, carcinome tubuleux, tumeurs oculaires, cancer de l'urètre, tumeurs urologiques, carcinome de l'urothélium, cancer de la valve,
éruption de verrues, tumeurs des tissus mous, sarcome des tissus mous, tumeur de Wilms, carcinome du col de l'utérus, cancer de la langue, carcinome canalaire infiltrant, carcinome loculaire invasif, carcinome canalaire *in situ*, carcinome globulaire *in situ*, carcinome pulmonaire à petites cellules, carcinome pulmonaire non à petites cellules,
adénome bronchique, blastome pleuropulmonaire, mésothéliome, gliome du tronc cérébral, gliome hypothalamique, astrocytome du cervelet, astrocytome cérébral, tumeurs neuroectodermiques, tumeurs pinéales, sarcome de l'utérus, cancers des glandes salivaires, adénocarcinomes des glandes anales, tumeurs mastocytaires, tumeurs pelviennes, tumeurs urétrales, cancers héréditaires papillaires rénaux, cancers sporadiques papillaires rénaux, mélanome intraoculaire, carcinome hépatocellulaire (carcinomes hépatocellulaires avec ou sans variante fibrolamellaire), cholangiocarcinome (cancer des canaux biliaires intrahépatiques), cholangiocarcinome hépatocellulaire mixte, carcinome à cellules squameuses, mélanome malin, cancer cutané à cellules de Merkel, cancer cutané non mélanomateux, cancer de l'hypopharynx, cancer du nasopharynx, cancer de l'oropharynx, cancer de la cavité orale, cancer à cellules squameuses, mélanome oral, lymphome lié au SIDA, lymphome cutané à cellules T, lymphome du système nerveux central, histiocytome fibreux malin, lymphosarcome, rhabdomyosarcome, histiocytose maligne, fibrosarcome, hémangiosarcome, hémangiopéricytome, léiomyosarcome, carcinome mammaire canin et carcinome mammaire félin.

8. Composition pharmaceutique comprenant au moins un composé selon l'une quelconque des revendications 1 à 4 en tant que principe actif, avec au moins un vecteur, excipient et/ou diluant pharmaceutiquement acceptable.

9. Composition pharmaceutique selon la revendication 8, comprenant en outre un ou plusieurs autres agents antitumoraux.

10. Procédé de synthèse du composé de formule générale (I) selon l'une quelconque des revendication 1 en fonction des modes opératoires suivants :
réaction de la 2,4-dichloro-1,3,5-triazine avec une aniline de formule générale R¹NH₂, où R¹ prend les valeurs définies dans la revendication 1, pour obtenir la 2-arylamino-4-chloro-1,3,5-triazine qui réagit ensuite avec un dérivé d'acide botanique de formule générale R²-B(OR)₂, où R² prend les valeurs définies dans la revendication 1 et chacune des deux entités R représente indépendamment de l'autre un atome d' hydrogène ou une chaîne alkyl comportant 1 à 10 atomes de carbone ou une chaîne cycloalkyle comportant 3 à 12 atomes de carbone, ou les deux entités R représentent ensemble un résidu dérivé du pinacol pour obtenir le composé de formule générale (I) selon le schéma réactionnel suivant : 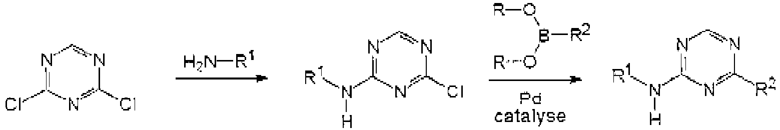 ou
réaction de la 2,4-dichloro-1,3,5-triazine avec un dérivé d'acide boronique de formule générale R²-B(OR)₂, où R² prend les valeurs définies dans la revendication 1 et chacune des deux entités R représente indépendamment de l'autre un atome d'hydrogène ou une chaîne alkyl comportant 1 à 10 atomes de carbone ou une chaîne cycloalkyl comportant 3 à 12 atomes de carbone, ou les deux entités R représentent ensemble un résidu dérivé du pinacol pour obtenir la chloro-1,3,5-triazine substituée par R² qui réagit ensuite avec l'aniline de formule générale R¹NH₂, où R¹ prend les valeurs définies dans la revendication 1, pour obtenir le composé de formule générale (I) selon le schéma réactionnel suivant : 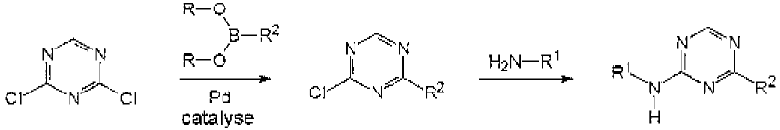 ou
reaction d'un amide de formule générale R²-CONH₂, ou R² prend les valeurs définies dans la revendication 1, avec un acétal de N,N-diméthylformamide pour obtenir l'intermédiaire N-acylformamidine qui réagit ensuite avec la guanidine de formule générale R¹-NH-C(NH)NH₂, où R¹ prend les valeurs définies dans la revendication 1, pour obtenir le composé de formule général (I) selon le schéma réactionnel suivant :
